# EUROPEAN PATENT APPLICATION

(11) **EP 4 847 038 A1**
(43) Date of publication of application: **02.12.2026**
(21) Application number: 25744712.8
(22) Date of filing: 24.01.2025
(51) Int. Cl.: A61K 39/00, A61K 39/39, A61K 48/00, C07K 14/005, C12N 15/10, C12N 15/40, C12N 15/63, C12N 15/86

(54) **SELF-REPLICATING RNA AND USE THEREOF**

(30) Priority: 24.01.2024 CN 202410102577; 06.08.2024 CN 202411074843
(71) Applicant: Nanjing GenScript Biotech Co., Ltd., Nanjing, Jiangsu 211100 (CN)
(72) Inventor: CHEN, Kangming, Nanjing, Jiangsu 211100 (CN); SUN, Zhen, Nanjing, Jiangsu 211100 (CN); ZHANG, Haoyi, Nanjing, Jiangsu 211100 (CN); HU, Fangxiang, Nanjing, Jiangsu 211100 (CN)
(74) Representative: Ström & Gulliksson AB
(86) International application number: PCT/CN2025/074563
(87) International publication number: WO 2025/157249

(57) **Abstract**

The present application relates to a replicable RNA molecule and the use thereof. The replicable RNA molecule comprises, from the 5' end to the 3' end, a 5' cap, a 5' UTR, an open reading frame encoding an RNA replicase, a promoter, a sequence of interest, a 3' UTR and a poly(A) tail, wherein the RNA replicase is capable of amplifying the replicable RNA molecule and is capable of amplifying an RNA molecule containing the sequence of interest and the 3' UTR, wherein the RNA replicase is a nonstructural protein or a functional variant thereof derived from Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV).

## Description

### Cross Reference to Related Application

The present application claims priority to Chinese patent application no. CN202410102577.7 filed on January 24, 2024 and CN202411074843.6 filed on August 6, 2024, the entire contents of which are incorporated herein by reference.

### Technical Field

The present application provides a replicable RNA molecule capable of amplifying and expressing a sequence of interest in a cell. The present application further relates to the use of the replicable RNA molecule in the preparation of a peptide or protein of interest, in the treatment or prevention of certain diseases.

### Background Art

A messenger RNA (mRNA) is a single-stranded RNA that is a template that a cell uses to synthesize a protein. In recent years, scientists have continuously optimized the molecular properties of the mRNA, striving to enable its use as an active pharmaceutical ingredient for various types of therapeutic interventions. When applied to patients, mRNA molecules can express almost any protein of interest. Potential application scenarios include cancer treatment, protein replacement, infectious disease vaccination and the like. In contrast to traditional protein therapy or other types of nucleic acid therapy (such as plasmid DNAs or viral vectors), the advantages of mRNAs lie in their ability to synthesize proteins with natural conformation and the absence of genomic integration risks. Moreover, the production process of mRNAs is relatively simple, requiring only in vitro enzymatic reactions and simple downstream purification, offering significant advantages in process scaling. With the successful application of the COVID-19 mRNA vaccine, mRNA-based pharmaceutical products have attracted great interest from the scientific community and the public.

Despite having many advantages, mRNAs still have many limitations in practical applications, mainly because the mRNAs are extremely unstable and prone to degradation, and have relatively short half-lives, greatly limiting the use of mRNAs as therapeutic drugs. To address this problem, two new types of mRNAs were developed. The first type is a self-replicating RNA (saRNA, also known as a self-amplifying RNA), which, with the aid of the viral replicase system, allows the in vitro synthesized RNAs to be continuously amplified in large quantities within the cell, thereby achieving higher and more persistent target protein expression. The second type is a circular RNA, which achieve resistance to RNase degradation through alteration of the configuration of an RNA, resulting in enhanced stability and prolonged expression of the target protein.

The saRNA sequence is derived from a modification to the bicistronic genome of a positive-strand RNA virus (e.g., an alphavirus, a flavivirus, a lentivirus, a measles virus, a rhabdovirus, and the like). In addition to conventional mRNA elements such as a cap, a 5' UTR, a 3' UTR, and a poly(A) tail, the saRNA comprises a very large open reading frame at the 5' end encoding 4 nonstructural proteins (nsP) of the positive-strand RNA virus, while the genes encoding the structural proteins of the virus, which were originally located behind the subgenomic promoter (SGP), were replaced with a gene encoding a protein of interest. Once the saRNA enters the host cytoplasm, it is first translated into 4 nonstructural proteins (nsP1, nsP2, nsP3, and nsP4), which assemble to form an RNA-dependent RNA polymerase complex, also known as an RNA replicase. The RNA polymerase complex first synthesizes a complementary antisense strand RNA from the sense strand RNA. Then, using this antisense strand as a template, it synthesizes a sense strand RNA copy of the original full-length RNA, as well as multiple subgenomic sense strand RNAs encoding the protein of interest which are located downstream of the SGP; the former further enters the amplification cycle, while the latter can be translated into the protein of interest. This is why the saRNA can achieve efficient and sustained expression of the protein of interest at a low dose.

Compared with a traditional non-replicating linear mRNA, the saRNA achieves higher and more persistent protein expression. However, during the amplification process, the saRNA can form a dsRNA structure, which can potentially induce a strong innate immune response in cells of a host. In the context of using an saRNA to express a protein of interest for a vaccine, this can be beneficial for the recruitment and activation of antigen-presenting cells and adaptive immune system cells. At the same time, the host cell immune response can inhibit translation of the protein of interest encoded in the saRNA subgenome. The key objectives in the development of saRNA molecules are to not only promote the recruitment and activation of downstream immune responses, but also eliminate the adverse effect on the protein of interest encoded in the subgenome.

In recent years, researchers have tried various strategies to design and optimize the sequence of the saRNA vector backbone to reduce the host's natural immune response and increase the intensity and duration of expression of the protein of interest using the saRNA. In 2017, Ugur Sahin, in order to reduce the stimulation of intracellular pattern recognition receptors by an saRNA, and alleviate the inhibitory effect in translating the protein of interest encoded by the saRNA, co-delivered non-replicating mRNAs encoding immune escape proteins E3/K3/B18 with an saRNA encoding luciferase for the first time. This method significantly inhibits the intracellular PKR and IFN pathways, greatly improving the in vivo translation efficiency of saRNA-encoded luciferase in mice. However, the co-delivery of the two types of mRNAs significantly increases the injected dose of mRNAs, losing the advantage of low-dose applications of self-replicating mRNAs. In 2019, Yingzhong Li et al. constructed an in vitro evolution strategy based on the VEEV replicon system and screened for mutant VEEV replicon sequences with high expression and low immunogenicity. In addition to mutant replicons obtained by manual screening, there are a large number of naturally occurring mutant sequences of viral replicons, such as the VEEV-TC83 strain, which exhibits higher expression efficiency than the original VEEV. Thus, screening for native viral replicons is one of effective means for optimizing saRNA sequences. In addition, an mRNA that is replicated intracellularly for target protein expression is essentially identical in structure to a traditional non-replicating mRNA, and thus the sequence optimization manner for a traditional non-replicating mRNA can also be applicable to a subgene sequence of a self-replicating RNA. However, the expression level of target proteins encoded by currently reported self-replicating RNAs, particularly the VEEV replicon system, is far from meeting the requirements of many clinical experiments.

### Summary of the Invention

The inventors of the present application have screened viral replicons with high replication capacity, and when the replicons are used to construct self-replicating RNAs for expressing proteins of interest, the protein expression level is significantly higher than that in the case of self-replicating RNAs constructed from such as VEEV TC83. In addition, through UTR optimization and cis expression of accessory proteins, a self-replicating RNA vector is allowed to exhibit more efficient expression of a protein of interest and less immunogenicity.

Thus, in a first aspect, the present application provides a replicable RNA molecule that can comprise, from the 5' end to the 3' end, a 5' cap, a 5' UTR, an open reading frame encoding an RNA replicase, a promoter, a sequence of interest, a 3' UTR and a poly(A) tail, wherein the RNA replicase is capable of amplifying the replicable RNA molecule and is capable of amplifying an RNA molecule containing the sequence of interest and the 3' UTR.

The replicable RNA molecule can be a single-stranded RNA molecule.

The RNA replicase can be a nonstructural protein or a functional variant thereof derived from Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV). The nonstructural proteins can include nsP1, nsP2, nsP3, and/or nsP4. In some embodiments, the nonstructural proteins can be nsP123 and nsP4. In some embodiments, the nonstructural protein can be nsP1234. The open reading frame encoding the RNA replicase can comprise or be composed of a nucleotide sequence that has at least 85%, 86%, 87%, 88%, 89%, 90%, 91%, 92%, 93%, 94%, 95%, 96%, 97%, 98%, 99% or 100% sequence identity to SEQ ID NO: 12, 36, 52, 56, 60, 16, 20, 24, 28, 32, 40, 44 or 48.

The RNA replicase can have the ability to amplify a replicable RNA molecule, including the ability to transcribe, from the replicable RNA molecule, a RNA strand complementary thereto, and the ability to transcribe the replicable RNA molecule from the transcribed RNA strand. The RNA replicase has the ability to amplify an RNA molecule containing or composed of a sequence of interest and a 3' UTR, including the abilities to transcribe, from a replicable RNA molecule, an RNA strand complementary thereto, transcribe the RNA molecule containing or composed of the sequence of interest and the 3' UTR from the transcribed RNA strand, and optionally add a 5' cap and a poly(A) tail to the RNA molecule. In some embodiments, the amount of an RNA molecule, containing or composed of a sequence of interest and a 3' UTR, amplified by the RNA replicase is greater than the amount of a replicable RNA molecule amplified.

The RNA replicase can have activity of an RNA-dependent RNA polymerase, a protease, a helicase, a terminal adenylyltransferase, methyltransferase and/or guanylyltransferase.

The 5' UTR, the promoter, and/or the 3' UTR in the replicable RNA molecule can cooperate with the RNA replicase to facilitate amplification of the replicable RNA molecule, and/or the RNA molecule containing or composed of the sequence of interest and the 3' UTR. In some embodiment, the 5' UTR, the promoter, and the 3' UTR in the replicable RNA molecule can cooperate with the RNA replicase to facilitate amplification of the replicable RNA molecule, and the RNA molecule containing or composed of the sequence of interest and the 3' UTR. The RNA molecule containing the sequence of interest and the 3' UTR can be a subgenomic RNA molecule of a virus.

In some embodiments, the 5' UTR, the promoter, and the 3' UTR can be derived from a genome of the same virus as the RNA replicase, such as Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV).

In some embodiments, the 5' UTR can be derived from a genome of Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV).

In some embodiments, the promoter can be derived from a genome of Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV). The promoter can be a subgenomic promoter (SGP) of Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV).

In some embodiments, the 3' UTR can be derived from a genome of Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV).

In some embodiments, the 5' UTR, the open reading frame encoding the RNA replicase, the promoter, and the 3' UTR can respectively comprise or be composed of nucleotide sequences that have at least 85%, 86%, 87%, 88%, 89%, 90%, 91%, 92%, 93%, 94%, 95%, 96%, 97%, 98%, 99% or 100% sequence identity to (1) SEQ ID NOs: 11, 12, 13 and 14; (2) SEQ ID NOs: 35, 36, 37 and 38; (3) SEQ ID NOs: 51, 52, 53 and 54; (4) SEQ ID NOs: 55, 56, 57 and 58; (5) SEQ ID NOs: 59, 60, 61 and 62; (6) SEQ ID NOs: 15, 16, 17 and 18; (7) SEQ ID NOs: 19, 20, 21 and 22; (8) SEQ ID NOs: 23, 24, 25 and 26; (9) SEQ ID NOs: 27, 28, 29 and 30; (10) SEQ ID NOs: 31, 32, 33 and 34; (11) SEQ ID NOs: 39, 40, 41 and 42; (12) SEQ ID NOs: 43, 44, 45 and 46; or (13) SEQ ID NO 47, 48, 49 and 50.

The 5' cap can be a natural 5' cap, or a 5' cap analog. The 5' cap analog can be Cap-AU or Cap-AG. The poly(A) tail can contain or consist of consecutive adenylic acids. Alternatively, the poly(A) tail can contain 2-5 fragments of consecutive adenylic acids separated by a spacer sequence, wherein the spacer sequence contains 1-20 nucleotides, and each fragment of consecutive adenylic acids contains 10-100 consecutive adenylic acids.

The sequence of interest can be any sequence. In some embodiments, the sequence of interest can be an open reading frame encoding a peptide or protein of interest. The peptide or protein of interest can be a disease-associated antigen, or a therapeutic agent.

The present application further provides an RNA combination that can comprise a first RNA molecule and a second RNA molecule.

The first RNA molecule can comprise, from the 5' end to the 3' end, a 5' cap, a 5' UTR, an open reading frame encoding an RNA replicase, a 3' UTR, and a poly(A) tail.

The second RNA molecule can comprise a 5' cap, a 5' UTR, a conserved sequence element, a promoter, a sequence of interest, a 3' UTR, and a poly(A) tail. In some embodiments, the second RNA molecule can comprise, from the 5' end to the 3' end, a 5' cap, a 5' UTR, a conserved sequence element, a promoter, a sequence of interest, a 3' UTR and a poly(A) tail. In some embodiments, the second RNA molecule can comprise, from the 5' end to the 3' end, a 5' cap, a 5' UTR, a first conserved sequence element, a second conserved sequence element, a promoter, a sequence of interest, a 3' UTR and a poly(A) tail.

The first RNA molecule can be a single-stranded RNA molecule.

The second RNA molecule can be a single-stranded RNA molecule.

The RNA replicase can be a nonstructural protein or a functional variant thereof derived from Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV). The nonstructural protein derived from Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV) can comprise nsP1, nsP2, nsP3 and/or nsP4. In some embodiments, the nonstructural proteins can be nsP123 and nsP4. In some embodiments, the nonstructural protein can be nsP1234. The open reading frame encoding the RNA replicase can comprise or be composed of a nucleotide sequence that has at least 85%, 86%, 87%, 88%, 89%, 90%, 91%, 92%, 93%, 94%, 95%, 96%, 97%, 98%, 99% or 100% sequence identity to SEQ ID NO: 12, 36, 52, 56, 60, 16, 20, 24, 28, 32, 40, 44 or 48.

The RNA replicase can have activity of an RNA-dependent RNA polymerase, a protease, a helicase, a terminal adenylyltransferase, methyltransferase and/or guanylyltransferase.

The RNA replicase in the first RNA molecule is capable of amplifying the second RNA molecule. The RNA replicase has the ability to amplify the second RNA molecule, including the ability to transcribe, from the second RNA molecule, a RNA strand complementary thereto, and the ability to transcribe the second RNA molecule from the transcribed RNA strand. The 5' UTR, the conserved sequence element, the promoter, and/or the 3' UTR in the second RNA molecule can cooperate with the RNA replicase in the first RNA molecule to facilitate amplification of the second RNA molecule. In particular, the 5' UTR, the conserved sequence element, the promoter, and the 3' UTR in the second RNA molecule can be derived from the same virus as the RNA replicase in the first RNA molecule. In some embodiments, the conserved sequence element can overlap completely or partially with the promoter, and/or the UTR (particularly the 5' UTR).

The promoter in the second RNA molecule can be a subgenomic promoter (SGP) of Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV). The second RNA molecule can be a subgenomic RNA molecule of a virus. In some embodiments, the open reading frame encoding an RNA replicase in the first RNA molecule, and the 5' UTR, the promoter and the 3' UTR in the second RNA molecule can respectively comprise or be composed of nucleotide sequences that have at least 85%, 86%, 87%, 88%, 89%, 90%, 91%, 92%, 93%, 94%, 95%, 96%, 97%, 98%, 99% or 100% sequence identity to (1) SEQ ID NOs: 12, 11, 13 and 14; (2) SEQ ID NOs: 36, 35, 37 and 38; (3) SEQ ID NOs: 52, 51, 53 and 54; (4) SEQ ID NOs: 56, 55, 57 and 58; (5) SEQ ID NOs: 60, 59, 61 and 62; (6) SEQ ID NOs: 16, 15, 17 and 18; (7) SEQ ID NOs: 20, 19, 21 and 22; (8) SEQ ID NOs: 24, 23, 25 and 26; (9) SEQ ID NOs: 28, 27, 29 and 30; (10) SEQ ID NOs: 32, 31, 33 and 34; (11) SEQ ID NOs: 40, 39, 41 and 42; (12) SEQ ID NOs: 44, 43, 45 and 46; or (13) SEQ ID NO 48, 47, 49 and 50.

The RNA replicase in the first RNA molecule can have the ability to amplify the first RNA molecule.

In some embodiments, the RNA replicase in the first RNA molecule is capable of amplifying the first RNA molecule. The RNA replicase can have the ability to amplify the first RNA molecule, including the ability to transcribe, from the first RNA molecule, a RNA strand complementary thereto, and the ability to transcribe the first RNA molecule from the transcribed RNA strand. In some embodiments, the amount of the second RNA molecule amplified by the RNA replicase is greater than the amount of the first RNA molecule amplified. The 5' UTR and/or 3' UTR of the first RNA molecule can cooperate with the RNA replicase to facilitate amplification of the first RNA molecule. In particular, the 5' UTR and/or 3' UTR of the first RNA molecule can be derived from the same virus as the RNA replicase. In some embodiments, the 5' UTR, the open reading frame encoding the RNA replicase, and the 3' UTR in the first RNA molecule can respectively comprise or be composed of nucleotide sequences that have at least 85%, 86%, 87%, 88%, 89%, 90%, 91%, 92%, 93%, 94%, 95%, 96%, 97%, 98%, 99% or 100% sequence identity to (1) SEQ ID NOs: 11, 12 and 14; (2) SEQ ID NOs: 35, 36 and 38; (3) SEQ ID NOs: 51, 52 and 54; (4) SEQ ID NOs: 55, 56 and 58; (5) SEQ ID NOs: 59, 60 and 62; (6) SEQ ID NOs: 15, 16 and 18; (7) SEQ ID NOs: 19, 20 and 22; (8) SEQ ID NOs: 23, 24 and 26; (9) SEQ ID NOs: 27, 28 and 30; (10) SEQ ID NOs: 31, 32 and 34; (11) SEQ ID NOs: 39, 40 and 42; (12) SEQ ID NOs: 43, 44 and 46; or (13) SEQ ID NOs: 47, 48 and 50.

The 5' cap in the first and second RNA molecules can be a natural 5' cap, or a 5' cap analog. The 5' cap analog can be Cap-AU or Cap-AG.

The poly(A) tail in the first and second RNA molecules can contain or consist of consecutive adenylic acids. Alternatively, the poly(A) tail in the first and second RNA molecules can contain 2-5 fragments of consecutive adenylic acids separated by a spacer sequence, wherein the spacer sequence contains 1-20 nucleotides, and each fragment of consecutive adenylic acids contains 10-100 consecutive adenylic acids.

The sequence of interest can be any sequence. In some embodiments, the sequence of interest can be an open reading frame encoding a peptide or protein of interest. The peptide or protein of interest can be a disease-associated antigen, or a therapeutic agent.

In a second aspect, the present application provides a replicable RNA molecule that can comprise, from the 5' end to the 3' end, a 5' cap, a 5' UTR, an open reading frame encoding an RNA replicase, a promoter, a second 5' UTR, a sequence of interest, a 3' UTR and a poly(A) tail, wherein the RNA replicase is capable of amplifying the replicable RNA molecule and is capable of amplifying an RNA molecule containing the second 5' UTR, the sequence of interest and the 3' UTR.

The replicable RNA molecule can be a single-stranded RNA molecule.

The RNA replicase can be a nonstructural protein or a functional variant thereof derived from a self-replicating virus. The nonstructural proteins can include nsP1, nsP2, nsP3, and/or nsP4. In some embodiments, the nonstructural proteins can be nsP123 and nsP4. In some embodiments, the nonstructural protein can be nsP1234. The self-replicating virus can be an alphavirus, a flavivirus, a measles virus or a rhabdovirus. The alphavirus can be any alphavirus, including but not limited to, Aura virus (AURV), Barmah Forest virus (BFV), Bebaru virus (BEBV), Cabassou virus (CABV), Chikungunya virus (CHIKV), Eastern equine encephalitis virus (EEEV), Eilat virus (ELIV), Everglades virus (EVEV), Fort Morgan virus (FMV), Getah virus (GETV), Mayaro virus (MAYV), Madariaga virus (MADV), Mosso das Pedras virus (MDPV), Ndumu virus (NDUV), O'nyong-nyong virus (ONNV), Pixuna virus (PIXV), Ross River virus (RRV), Semliki forest virus (SFV), Sindbis virus (SINV), Tonate virus (TONV or TV), Trocara virus (TROV), Venezuelan equine encephalitis virus (VEEV), Una virus (UNAV), Highlands J virus (HJV), Mucambo virus (MUCV), Ruhugu virus (RHGV), Rio Negro virus (RNV), Rustrela virus (RUSV) and Sagiyama virus (SAGV). In some embodiments, the alphavirus can be Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV). In some embodiments, the RNA replicase can be a nonstructural protein or a functional variant thereof derived from Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV). In some embodiments, the open reading frame encoding the RNA replicase can comprise a nucleotide sequence that has at least 85%, 86%, 87%, 88%, 89%, 90%, 91%, 92%, 93%, 94%, 95%, 96%, 97%, 98%, 99% or 100% sequence identity to SEQ ID NO: 12, 36, 16, 20, 24, 28, 32, 40, 44 or 48. In some embodiments, the sequence of the open reading frame encoding the RNA replicase can be as set forth in SEQ ID NO: 12, 36, 52, 56, 60, 16, 20, 24, 28, 32, 40, 44, or 48.

The RNA replicase can have the ability to amplify a replicable RNA molecule, including the ability to transcribe, from the replicable RNA molecule, a RNA strand complementary thereto, and the ability to transcribe the replicable RNA molecule from the transcribed RNA strand. The RNA replicase has the ability to amplify an RNA molecule containing or composed of a second 5' UTR, a sequence of interest and a 3' UTR, including the abilities to transcribe, from a replicable RNA molecule, an RNA strand complementary thereto, transcribe the RNA molecule containing or composed of the second 5' UTR, the sequence of interest and the 3' UTR from the transcribed RNA strand, and optionally add a 5' cap and a poly(A) tail to the RNA molecule. In some embodiments, the amount of an RNA molecule, containing or composed of a second 5' UTR, a sequence of interest and a 3' UTR, amplified by the RNA replicase is greater than the amount of a replicable RNA molecule amplified.

The RNA replicase can have activity of an RNA-dependent RNA polymerase, a protease, a helicase, a terminal adenylyltransferase, methyltransferase and/or guanylyltransferase.

The 5' UTR, the promoter, and/or the 3' UTR in the replicable RNA molecule can cooperate with the RNA replicase to facilitate amplification of the replicable RNA molecule, and/or the RNA molecule containing or composed of the second 5' UTR, the sequence of interest and the 3' UTR. In some embodiments, the 5' UTR, the promoter, and the 3' UTR in the replicable RNA molecule can cooperate with the RNA replicase to facilitate amplification of the replicable RNA molecule, and the RNA molecule containing or composed of the second 5' UTR, the sequence of interest and the 3' UTR. In some embodiment, the 5' UTR, the promoter, and the 3' UTR in the replicable RNA molecule can cooperate with the RNA replicase to facilitate amplification of the replicable RNA molecule, and/or the RNA molecule containing the second 5' UTR, the sequence of interest and the 3' UTR. In some embodiment, the 5' UTR, the promoter, and the 3' UTR in the replicable RNA molecule can cooperate with the RNA replicase to facilitate amplification of the replicable RNA molecule, and the RNA molecule composed of the second 5' UTR, the sequence of interest and the 3' UTR. The promoter can be a subgenomic promoter (SGP) of a self-replicating virus. The RNA molecule containing the second 5' UTR, the sequence of interest and the 3' UTR can be a subgenomic RNA molecule.

In some embodiments, the 5' UTR, the promoter, and the 3' UTR can be derived from the same self-replicating virus as the RNA replicase, such as an alphavirus, a flavivirus, a measles virus or a rhabdovirus. In some embodiments, the 5' UTR, the promoter, and the 3' UTR can be derived from the same genome of an alphavirus as the RNA replicase. In some embodiments, the 5' UTR, the promoter, and the 3' UTR, along with the RNA replicase, can be derived from Barmah Forest virus (BFV), Bebaru virus (BEBV), Cabassou virus (CABV), Chikungunya virus (CHIKV), Eastern equine encephalitis virus (EEEV), Eilat virus (ELIV), Everglades virus (EVEV), Fort Morgan virus (FMV), Getah virus (GETV), Mayaro virus (MAYV), Mosso das Pedras virus (MDPV), Ndumu virus (NDUV), O'nyong-nyong virus (ONNV), Pixuna virus (PIXV), Ross river virus (RRV), Semliki Forest virus (SFV), Sindbis virus (SINV), Tonate virus (TONV or TV), Trocara virus (TROV), Venezuelan equine encephalitis virus (VEEV), Una virus (UNAV), Aura virus (AURV), Highlands J virus (HJV), Madariaga virus (MADV), Mucambo virus (MUCV), Ruhugu virus (RHGV), Rio Negro virus (RNV), Rustrela virus (RUSV) or Sagiyama virus (SAGV). In some embodiments, the 5' UTR, the promoter, and the 3' UTR, along with the RNA replicase, can be derived from Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV).

In some embodiments, the 5' UTR, the open reading frame encoding the RNA replicase, the promoter, and the 3' UTR can respectively comprise nucleotide sequences that have at least 85%, 86%, 87%, 88%, 89%, 90%, 91%, 92%, 93%, 94%, 95%, 96%, 97%, 98%, 99% or 100% sequence identity to (1) SEQ ID NOs: 11, 12, 13 and 14; (2) SEQ ID NOs: 35, 36, 37 and 38; (3) SEQ ID NOs: 51, 52, 53 and 54; (4) SEQ ID NOs: 55, 56, 57 and 58; (5) SEQ ID NOs: 59, 60, 61 and 62; (6) SEQ ID NOs: 15, 16, 17 and 18; (7) SEQ ID NOs: 19, 20, 21 and 22; (8) SEQ ID NOs: 23, 24, 25 and 26; (9) SEQ ID NOs: 27, 28, 29 and 30; (10) SEQ ID NOs: 31, 32, 33 and 34; (11) SEQ ID NOs: 39, 40, 41 and 42; (12) SEQ ID NOs: 43, 44, 45 and 46; or (13) SEQ ID NOs: 47, 48, 49 and 50. In some embodiments, the sequences of the 5' UTR, the open reading frame encoding the RNA replicase, the promoter, and the 3' UTR can respectively be as set forth in (1) SEQ ID NOs: 11, 12, 13 and 14; (2) SEQ ID NOs: 35, 36, 37 and 38; (3) SEQ ID NOs: 51, 52, 53 and 54; (4) SEQ ID NOs: 55, 56, 57 and 58; (5) SEQ ID NOs: 59, 60, 61 and 62; (6) SEQ ID NOs: 15, 16, 17 and 18; (7) SEQ ID NOs: 19, 20, 21 and 22; (8) SEQ ID NOs: 23, 24, 25 and 26; (9) SEQ ID NOs: 27, 28, 29 and 30; (10) SEQ ID NOs: 31, 32, 33 and 34; (11) SEQ ID NOs: 39, 40, 41 and 42; (12) SEQ ID NOs: 43, 44, 45 and 46; or (13) SEQ ID NOs: 47, 48, 49 and 50.

The second 5' UTR can be any 5' UTR. The second 5' UTR can comprise a nucleotide sequence that has at least 85%, 86%, 87%, 88%, 89%, 90%, 91%, 92%, 93%, 94%, 95%, 96%, 97%, 98%, 99% or 100% sequence identity to SEQ ID NO: 5, 6, 7 or 8. In some embodiments, the second 5' UTR can comprise a nucleotide sequence that has at least 85%, 86%, 87%, 88%, 89%, 90%, 91%, 92%, 93%, 94%, 95%, 96%, 97%, 98%, 99% or 100% sequence identity to SEQ ID NO: 5. In some embodiments, the sequence of the second 5' UTR is as set forth in SEQ ID NO: 5, 6, 7 or 8.

The 5' cap can be a natural 5' cap, or a 5' cap analog. The 5' cap analog can be Cap-AU or Cap-AG.

The poly(A) tail can contain or consist of consecutive adenylic acids. Alternatively, the poly(A) tail can contain 2-5 fragments of consecutive adenylic acids separated by a spacer sequence, wherein the spacer sequence contains 1-20 nucleotides, and each fragment of consecutive adenylic acids contains 10-100 consecutive adenylic acids.

The sequence of interest can be any sequence. In some embodiments, the sequence of interest can be an open reading frame encoding a peptide or protein of interest. The peptide or protein of interest can be a disease-associated antigen, or a therapeutic agent.

In a third aspect, the present application provides a replicable RNA molecule that can comprise, from the 5' end to the 3' end, a 5' cap, a 5' UTR, an open reading frame encoding an RNA replicase, a promoter, a first sequence of interest, an internal ribosome entry site (IRES), a second sequence of interest, a 3' UTR and a poly(A) tail, wherein the RNA replicase is capable of amplifying the replicable RNA molecule and is capable of amplifying an RNA molecule containing the first sequence of interest, the internal ribosome entry site (IRES), the second sequence of interest and the 3' UTR.

The replicable RNA molecule can be a single-stranded RNA molecule.

One of the first and second sequences of interest is an open reading frame encoding an immunosuppressive protein. For example, the first sequence of interest is an open reading frame encoding a peptide or protein of interest, and the second sequence of interest is an open reading frame encoding an immunosuppressive protein; alternatively, the first sequence of interest is an open reading frame encoding an immunosuppressive protein, and the second sequence of interest is an open reading frame encoding a peptide or protein of interest. The peptide or protein of interest can be a disease-associated antigen, or a therapeutic agent.

The immunosuppressive protein can be an interferon inhibitory protein, such as poxvirus E3L protein, poxvirus K3 protein, poxvirus B18/B18R protein, nonstructural protein 1 of influenza virus, PIV5 protein of parainfluenza virus or MERS ORF4a protein. In some embodiments, the immunosuppressive protein can be poxvirus E3L protein. The open reading frame encoding the poxvirus E3L protein can comprise a nucleotide sequence set forth in SEQ ID NO: 10.

The RNA replicase can be a nonstructural protein or a functional variant thereof of a self-replicating virus. The nonstructural proteins can include nsP1, nsP2, nsP3, and/or nsP4. In some embodiments, the nonstructural proteins can be nsP123 and nsP4. In some embodiments, the nonstructural protein can be nsP1234. The self-replicating virus can be an alphavirus, a flavivirus, a measles virus or a rhabdovirus. The alphavirus can be any alphavirus including, but not limited to, Barmah Forest virus (BFV), Bebaru virus (BEBV), Cabassou virus (CABV), Chikungunya virus (CHIKV), Eastern equine encephalitis virus (EEEV), Eilat virus (ELIV), Everglades virus (EVEV), Fort Morgan virus (FMV), Getah virus (GETV), Mayaro virus (MAYV), Mosso das Pedras virus (MDPV), Ndumu virus (NDUV), O'nyong-nyong virus (ONNV), Pixuna virus (PIXV), Ross river virus (RRV), Semliki Forest virus (SFV), Sindbis virus (SINV), Tonate virus (TONV or TV), Trocara virus (TROV), Venezuelan equine encephalitis virus (VEEV), Una virus (UNAV), Aura virus (AURV), Highlands J virus (HJV), Madariaga virus (MADV), Mucambo virus (MUCV), Ruhugu virus (RHGV), Rio Negro virus (RNV), Rustrela virus (RUSV) or Sagiyama virus (SAGV). In some embodiments, the alphavirus can be Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV). In some embodiments, the RNA replicase can be a nonstructural protein or a functional variant thereof derived from Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV). In some embodiments, the open reading frame encoding the RNA replicase can comprise a nucleotide sequence that has at least 85%, 86%, 87%, 88%, 89%, 90%, 91%, 92%, 93%, 94%, 95%, 96%, 97%, 98%, 99% or 100% sequence identity to SEQ ID NO: 12, 36, 52, 56, 60, 16, 20, 24, 28, 32, 40, 44 or 48. In some embodiments, the sequence of the open reading frame encoding the RNA replicase can be as set forth in SEQ ID NO: 12, 36, 52, 56, 60, 16, 20, 24, 28, 32, 40, 44, or 48.

The RNA replicase can have the ability to amplify a replicable RNA molecule, including the ability to transcribe, from the replicable RNA molecule, a RNA strand complementary thereto, and the ability to transcribe the replicable RNA molecule from the transcribed RNA strand. The RNA replicase has the ability to amplify an RNA molecule containing or composed of a first sequence of interest, an internal ribosome entry site (IRES), a second sequence of interest and a 3' UTR, including the abilities to transcribe, from a replicable RNA molecule, an RNA strand complementary thereto, transcribe the RNA molecule containing or composed of the first sequence of interest, the internal ribosome entry site (IRES), the second sequence of interest and the 3' UTR from the transcribed RNA strand, and optionally add a 5' cap and a poly(A) tail to the RNA molecule. In some embodiments, the amount of an RNA molecule, containing or composed of a first sequence of interest, an internal ribosome entry site (IRES) and a second sequence of interest, formed by amplification optionally with the 5' capping and poly(A) tailing of the RNA molecule by the RNA replicase, is greater than the amount of a replicable RNA molecule amplified.

The RNA replicase can have activity of an RNA-dependent RNA polymerase, a protease, a helicase, a terminal adenylyltransferase, methyltransferase and/or guanylyltransferase.

The 5' UTR, the promoter, and/or the 3' UTR in the replicable RNA molecule can cooperate with the RNA replicase to facilitate amplification of the replicable RNA molecule, and/or the RNA molecule containing or composed of the first sequence of interest, the internal ribosome entry site (IRES), the second sequence of interest and the 3' UTR. In some embodiments, the 5' UTR, the promoter, and the 3' UTR in the replicable RNA molecule can cooperate with the RNA replicase to facilitate amplification of the replicable RNA molecule, and the RNA molecule containing or composed of the first sequence of interest, the internal ribosome entry site (IRES), the second sequence of interest and the 3' UTR. In some embodiment, the 5' UTR, the promoter, and the 3' UTR in the replicable RNA molecule can cooperate with the RNA replicase to facilitate amplification of the replicable RNA molecule, and/or the RNA molecule containing the first sequence of interest, the internal ribosome entry site (IRES), the second sequence of interest and the 3' UTR. In some embodiment, the 5' UTR, the promoter, and the 3' UTR in the replicable RNA molecule can cooperate with the RNA replicase to facilitate amplification of the replicable RNA molecule, and the RNA molecule composed of the first sequence of interest, the internal ribosome entry site (IRES), the second sequence of interest and the 3' UTR. The promoter can be a subgenomic promoter (SGP) of a self-replicating virus. The RNA molecule containing the first sequence of interest, the internal ribosome entry site (IRES), the second sequence of interest and the 3' UTR can be a subgenomic RNA molecule of a virus.

In some embodiments, the 5' UTR, the promoter, and the 3' UTR can be derived from a genome of the same self-replicating virus as the RNA replicase, such as a genome of an alphavirus, a flavivirus, a measles virus or a rhabdovirus. In some embodiments, the 5' UTR, the promoter, and the 3' UTR, along with the RNA replicase, can be derived from an alphavirus. In some embodiments, the 5' UTR, the promoter, and the 3' UTR, along with the RNA replicase, can be derived from Barmah Forest virus (BFV), Bebaru virus (BEBV), Cabassou virus (CABV), Chikungunya virus (CHIKV), Eastern equine encephalitis virus (EEEV), Eilat virus (ELIV), Everglades virus (EVEV), Fort Morgan virus (FMV), Getah virus (GETV), Mayaro virus (MAYV), Mosso das Pedras virus (MDPV), Ndumu virus (NDUV), O'nyong-nyong virus (ONNV), Pixuna virus (PIXV), Ross river virus (RRV), Semliki Forest virus (SFV), Sindbis virus (SINV), Tonate virus (TONV or TV), Trocara virus (TROV), Venezuelan equine encephalitis virus (VEEV), Una virus (UNAV), Aura virus (AURV), Highlands J virus (HJV), Madariaga virus (MADV), Mucambo virus (MUCV), Ruhugu virus (RHGV), Rio Negro virus (RNV), Rustrela virus (RUSV) or Sagiyama virus (SAGV). In some embodiments, the 5' UTR, the promoter, and the 3' UTR, along with the RNA replicase, can be derived from Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV). The promoter can be a subgenomic promoter of a virus.

In some embodiments, the 5' UTR, the open reading frame encoding the RNA replicase, the promoter, and the 3' UTR can respectively comprise or be composed of nucleotide sequences that have at least 85%, 86%, 87%, 88%, 89%, 90%, 91%, 92%, 93%, 94%, 95%, 96%, 97%, 98%, 99% or 100% sequence identity to (1) SEQ ID NOs: 11, 12, 13 and 14; (2) SEQ ID NOs: 35, 36, 37 and 38; (3) SEQ ID NOs: 51, 52, 53 and 54; (4) SEQ ID NOs: 55, 56, 57 and 58; (5) SEQ ID NOs: 59, 60, 61 and 62; (6) SEQ ID NOs: 15, 16, 17 and 18; (7) SEQ ID NOs: 19, 20, 21 and 22; (8) SEQ ID NOs: 23, 24, 25 and 26; (9) SEQ ID NOs: 27, 28, 29 and 30; (10) SEQ ID NOs: 31, 32, 33 and 34; (11) SEQ ID NOs: 39, 40, 41 and 42; (12) SEQ ID NOs: 43, 44, 45 and 46; or (13) SEQ ID NO 47, 48, 49 and 50.

A second 5' UTR can be comprised between the promoter and the first sequence of interest. The second 5' UTR can be any 5' UTR. The second 5' UTR can comprise a nucleotide sequence that has at least 85%, 86%, 87%, 88%, 89%, 90%, 91%, 92%, 93%, 94%, 95%, 96%, 97%, 98%, 99% or 100% sequence identity to SEQ ID NO: 5, 6, 7 or 8. In some embodiments, the second 5' UTR can comprise a nucleotide sequence that has at least 85%, 86%, 87%, 88%, 89%, 90%, 91%, 92%, 93%, 94%, 95%, 96%, 97%, 98%, 99% or 100% sequence identity to SEQ ID NO: 5. In some embodiments, the sequence of the second 5' UTR can be as set forth in SEQ ID NO: 5, 6, 7 or 8. The RNA replicase can amplify an RNA molecule containing or composed of a second 5' UTR, a first sequence of interest, an internal ribosome entry site (IRES), a second sequence of interest and a 3' UTR.

IRES can be any suitable IRES, such as IRES from the Coxsackie B3 virus (CVB3). The IRES of the Coxsackie B3 virus (CVB3) can comprise a nucleotide sequence set forth in SEQ ID NO: 9.

The 5' cap can be a natural 5' cap, or a 5' cap analog. The 5' cap analog can be Cap-AU or Cap-AG.

The poly(A) tail can contain or consist of consecutive adenylic acids. Alternatively, the poly(A) tail can contain 2-5 fragments of consecutive adenylic acids separated by a spacer sequence, wherein the spacer sequence contains 1-20 nucleotides, and each fragment of consecutive adenylic acids contains 10-100 consecutive adenylic acids.

In a fourth aspect, the present application provides a DNA molecule encoding the RNA molecule of the first aspect to the third aspect of the present application.

The DNA molecule can comprise a first strand comprising, from the 5' end to the 3' end, a promoter and a sequence encoding the RNA molecule of the first aspect to the third aspect of the present application.

The DNA molecule can comprise a second strand that is complementary to the first strand.

The DNA molecule can be a linear molecule.

The promoter can be an RNA polymerase promoter derived from T7 virus, T6 virus, SP6 virus, T3 virus, or T4 virus. In some embodiments, the promoter can be T7 promoter.

In a fifth aspect, the present application provides a vector comprising the DNA molecule of the fourth aspect. The vector can be a plasmid, a viral vector, etc. The vector can be cyclic, or linear. In some embodiments, the vector can be linear. In some embodiment, the vector can be cyclic and treated to be linear. In some embodiments, the vector can be used to prepare the RNA molecule of the first aspect to the third aspect. About 500 to about 18,000 nt of replicable RNA molecules can be transcribed from the vector of the present application.

In a sixth aspect, the present application provides a cell comprising the DNA molecule of the fourth aspect or the vector of the fifth aspect. The cell can be a host cell, such as a prokaryotic cell or a eukaryotic cell. In some embodiments, the cell can be a mammalian cell.

In a seventh aspect, the present application provides a method for preparing the RNA molecule of the first aspect to the third aspect of the present application, comprising: i) providing the DNA molecule of the fourth aspect of the present application, ii) optionally, making the DNA molecule linear, and iii) performing in vitro transcription under suitable conditions. The suitable conditions in step iii) include providing RNA polymerases, ATPs, UTPs, CTPs, GTPs, cap analogs, etc.

The present application also claims the RNA molecule prepared by the method of the present application.

In an eighth aspect, the present application provides a composition comprising the RNA molecule (including the RNA combination) of the first aspect of the present application, the RNA molecule of the second aspect of the present application, the RNA molecule of the third aspect of the present application, the DNA molecule of the fourth aspect of the present application, the cell of the sixth aspect of the present application or the RNA molecule obtained by the method of the seventh aspect of the present application.

The RNA molecule in the composition can be encapsulated in a liposome, such as a nanoliposome.

The composition can also comprise a suitable carrier.

In some embodiments, the composition can be a pharmaceutical composition comprising an effective amount of the RNA molecule (including the RNA combination) of the first aspect of the present application, the RNA molecule of the second aspect of the present application, the RNA molecule of the third aspect of the present application, the DNA molecule of the fourth aspect of the present application, the cell of the sixth aspect of the present application or the RNA molecule obtained by the method of the seventh aspect of the present application, and a pharmaceutically acceptable carrier.

In a ninth aspect, the present application provides a method for preparing a peptide or protein of interest using the RNA molecule (including the RNA combination) of the first aspect of the present application, the RNA molecule of the second aspect of the present application, the RNA molecule of the third aspect of the present application, or the RNA molecule obtained by the method of the seventh aspect of the present application, comprising:
i) introducing into a host cell the RNA molecule or the RNA combination, wherein the RNA molecule or the RNA molecule in the RNA combination comprises an open reading frame encoding the peptide or protein of interest, and
ii) culturing the host cell under appropriate conditions. The method can also comprise recovering the peptide or protein of interest from the host cell or a host cell culture medium. In some embodiments, the RNA molecule or the RNA molecule in the RNA combination comprises an open reading frame encoding a tagged peptide or protein of interest.

Step i) can comprise transfecting the RNA molecule or the RNA combination directly into the host cell, or into the cell by lipofection, electroporation, or encapsulation with nanocarriers. The nanocarriers can be, for example, lipids, polymers or lipid-polymer hybrids.

In particular, the present application provides a method for preparing a peptide or protein of interest, comprising:
i) providing the RNA molecule (including the RNA combination) of the first aspect of the present application, the RNA molecule of the second aspect of the present application, the RNA molecule of the third aspect of the present application, or the RNA molecule obtained by the method of the seventh aspect of the present application, wherein a sequence of interest in the RNA molecule is an open reading frame encoding the peptide or protein of interest, and
ii) introducing the RNA molecule into a cell.

The cell can be any host cell, such as a prokaryotic cell or a eukaryotic cell. In some embodiments, the host cell can be a mammalian cell.

The method can also comprise culturing the host cell under appropriate conditions. The method can further comprise recovering the peptide or protein of interest from the host cell or a host cell culture medium. In a tenth aspect, the present application provides a method for treating or preventing a disease in a subject in need thereof, comprising administering to the subject a pharmaceutical composition of the present application. The RNA molecule or the RNA molecule in the RNA combination of the present application in the pharmaceutical composition comprises an open reading frame encoding a peptide or protein of interest. The peptide or protein of interest can be a disease-associated antigen, or a therapeutic agent. The disease-associated antigen can be a peptide or protein on the surface of a microorganism, e.g., a virus, a bacterium, a mycoplasma, etc.; or a tumor-associated antigen. The therapeutic agent can be, for example, an antibody.

When the peptide or protein of interest is a peptide or protein on the surface of a microorganism, e.g., a virus, a bacterium, a mycoplasma, etc., the methods of the present application can be used to treat or prevent a disease associated with an infection with the microorganism.

Where the peptide or protein of interest is a tumor-associated antigen, or a protein that targets the tumor-associated antigen such as an antibody, the method of the present application can be used to treat a tumor associated with the tumor-associated antigen.

The peptide or protein of interest can also be a normal protein expressed in mammals, such as humans, which can be used for supplementary therapy in a subject lacking the normal protein.

The subject can be a mammal, such as a human.

The present application also claims the use of the RNA molecule (including the RNA combination) of the first aspect of the present application, the RNA molecule of the second aspect of the present application, the RNA molecule of the third aspect of the present application, or the RNA molecule obtained by the method of the seventh aspect of the present application, in preparing a peptide or protein of interest or in treating or preventing the relevant diseases, and the use of the DNA molecule of the fourth aspect of the present application in preparing the corresponding RNA molecule.

In the present application, the same nucleotide sequence, such as the nucleotide sequence represented by the same SEQ ID NO, can denote both a DNA sequence and an RNA sequence, with the only difference being the substitution of T with U.

### Brief Description of the Drawings

FIGs. 1A-1C show expression levels of EGFP proteins encoded by self-replicating RNA molecules constructed on the basis of different alphaviruses at 24 h and 48 h in HEK293T cells (FIGs. 1A and 1B) and A549 cells (FIG. 1C).
FIG. 2 shows the levels of EGFP proteins expressed from VEEV-TC83 self-replicating RNA molecules with the addition of different subgenomic 5' UTRs at 24 h, 48 h, 72 h, and 144 h after transfection into HEK293T cells.
FIGs. 3A and 3B show the levels of EGFP proteins expressed from VEEV-TC83 or other alphavirus self-replicating RNAs with E3L cis-expressed via IRES (FIG. 3A: unmodified self-replicating RNAs; FIG. 3B: m5C-modified self-replicating RNAs) at 24 h and 48 h after transfection into A549 cells.
FIGs. 4A and 4B show the levels in cells of IL-6 expressed from VEEV-TC83 or other alphavirus self-replicating RNAs with E3L cis-expressed via IRES (FIG. 4A: unmodified self-replicating RNAs; FIG. 4B: m5C-modified self-replicating RNAs) at 48 h after transfection into A549 cells.
FIG. 5 shows cell viability at 24 h and 48 h after transfection of VEEV-TC83 or other alphavirus self-replicating RNAs with E3L cis-expressed via IRES into HEK293T cells.
FIGs. 6A-6C show schematic structures of a self-replicating RNA constructed in the present application for expression of a sequence of interest (GOI) (FIG. 6A), a self-replicating RNA for expression of a sequence of interest (GOI) with addition of a subgenomic 5' UTR (FIG. 6B), and a self-replicating RNA for expression of a sequence of interest (GOI), with addition of a subgenomic 5' UTR, and E3L cis-expressed via IRES (FIG. 6C).
FIG. 7 shows titers of specific antibodies in sera at different times after immunization (10, 20, 30, 40, 50, 60, 70, 80 and 90 days post immunization) of mice with SARS-CoV-2 RBD-saRNA-LNP with E3L cis-expressed via IRES.

### Detailed Description of Embodiments

The terms used herein have the ordinary meaning as used in dictionaries, textbooks, and technical reference books, or as commonly understood by a person skilled in the art, unless otherwise specified. The following description of certain terms is for the purpose of facilitating understanding of the present application only and is not intended to limit the terms specifically unless otherwise specified.

As used herein and in the appended claims, the singular forms "a", "an", and "the" include plural forms of the referent unless the context clearly dictates otherwise.

The term "or" refers to an individual element of the listed optional elements, unless the context explicitly indicates otherwise.

The term "contain" or "comprise" means that the described elements, integers, or steps are included, but the addition of any other elements, integers, or steps is not precluded. When the term "contain" or "comprise" is used herein, unless otherwise indicated, combinations of the described elements, integers, or steps are also included. The term "composed of..." or "consisting of..." generally means that only the described elements, integers, or steps are included, and no other element, integer, or step is added.

The 5' end of a nucleic acid molecule can be a terminal with a free phosphate group, and the 3' end can be a terminal with a free hydroxyl group.

The "replicable RNA" or "self-replicating RNA" herein refers to an RNA molecule that is capable of being amplified by an RNA replicase encoded by the RNA molecule itself. In particular, the "replicable RNA" or "self-replicating RNA" has a modified genome of a self-replicating virus. It can use itself as a template to amplify a complementary strand according to the principle of base "complementarity", and then use the complementary strand as a template to amplify full-length copies and multiple non-full-length copies of the RNA itself. Both the RNA molecule itself and its full-length copies can enter a new amplification cycle for amplifying more full-length and non-full-length copies.

The "complementary" used herein means that two nucleotides or two bases can be paired and bound according to the base complementary principle of A-T, A-U, and C-G. When a nucleotide sequence is "complementary" to another nucleotide sequence, it may mean that the two nucleotide sequences are 100% complementary to each other, or that the two nucleotide sequences are highly complementary, e.g., 90% or more complementary.

The "replication" or "amplification" herein refers to the synthesis of an RNA molecule based on the nucleotide sequence of the specified RNA molecule. The synthesized RNA molecule can be the same as or complementary to the template RNA molecule. RNA replication might synthesize DNA intermediates. The alphaviral RNA replication does not involve DNA intermediates, but is mediated by an RNA-dependent RNA polymerase, which synthesizes a second RNA strand from a first RNA strand or part thereof as a template, and synthesizes a third RNA strand from the second RNA strand or part thereof as a template.

The "RNA replicase" herein refers to an RNA-dependent RNA polymerase, which is an enzyme that catalyzes the synthesis of an RNA using an RNA as a template. Under the catalysis of the alphaviral RNA-dependent RNA polymerase, the (-) complementary strand of the genomic RNA and the (+) genomic RNA strand are sequentially synthesized, leading to RNA replication. In nature, the RNA-dependent RNA polymerases are typically encoded by all RNA viruses except retroviruses, such as alphaviruses. In particular, the "RNA replicase" in the present application can refer to a nonstructural protein of a self-replicating virus, such as an alphavirus.

The "self-replicating type virus" or "self-replicating virus" refers to an RNA virus that is capable of autonomous replication in a host cell. The self-replicating virus can have a single-stranded RNA genome, and includes an alphavirus, a flavivirus, a measles virus, and a rhabdovirus. The alphavirus and flavivirus have a sense-strand genome, while the measles virus and rhabdovirus have antisense-strand ssRNAs. In general, the self-replicating virus is a virus that has a (+) strand RNA genome and is capable of direct translation upon infection of a cell, wherein the translation provides an RNA-dependent RNA polymerase that later generates sense and antisense transcripts. The "transcript" generally refers to a gene transcript, or a transcription unit, that is a nucleotide molecule complementary to the template strand. The (+) strand or sense strand can be a strand that contains or encodes genetic information.

The "alphavirus" shall be understood broadly to include any viral particle with alphaviral characteristics. The alphaviral characteristics include the presence of a (+) strand RNA encoding genetic information (including RNA polymerase activity) suitable for replication in host cells. The term includes alphaviruses found in nature, and any variants or derivatives thereof.

The "nonstructural protein" refers to a protein encoded by a virus that does not form part of the viral particle. The term generally includes enzymes and transcription factors used by a variety of viruses to replicate themselves, such as RNA-dependent RNA polymerases. The "alphaviral nonstructural protein" refers to the individual nonstructural proteins of alphavirus origin, such as nsP1, nsP2, nsP3 and nsP4, or a polyprotein thereof. In some embodiments, the "alphaviral nonstructural protein" refers to nsP123 and/or nsP4. In other embodiments, the "alphaviral nonstructural protein" refers to nsP1234. The "functional variant" of a nonstructural protein is a variant that has a mutation compared to the native nonstructural protein but still has the desired function of the nonstructural protein.

The "promoter" herein refers to a sequence that controls transcript synthesis by providing recognition and binding sites for an RNA polymerase. The promoter region can also comprise recognition or binding sites for other factors involved in transcriptional regulation. The promoter can either be an inducible promoter which initiates transcription in response to an induction signal, or be a constitutive promoter. The inducible promoter, in the absence of an induction signal, cause little or no transcription. The promoter herein can be a subgenomic promoter, such as a subgenomic promoter of an alphavirus. Other specific promoters can be a genomic (+) or (-) strand promoter, such as a genomic (+) or (-) strand promoter of an alphavirus.

The "subgenomic promoter" refers to a nucleic acid sequence upstream of a sequence of interest (e.g., an open reading frame encoding a peptide or protein of interest) in an RNA molecule of the present application, which controls the transcription of the sequence of interest by providing recognition and binding sites for an RNA polymerase (typically an RNA-dependent RNA polymerase, in particular, a functional alphaviral nonstructural protein). The subgenomic promoter can also comprise recognition or binding sites for other factors. The subgenomic promoter is typically a genetic element of a sense strand RNA virus. The subgenomic promoter of an alphavirus is a nucleic acid sequence contained in a viral genomic RNA. The subgenomic promoter is characterized by its ability to initiate transcription, i.e. RNA synthesis, in the presence of an RNA-dependent RNA polymerase (such as a functional nonstructural protein). An RNA (-) strand, i.e. the complementary strand of the alphaviral genomic RNA, is used as a template for the synthesis of the (+) strand subgenomic transcript, and the synthesis of the (+) strand subgenomic transcript usually starts from or in the vicinity of the subgenomic promoter.

The "subgenomic RNA" or "subgenomic transcript" refers to an RNA molecule, which contains a viral structural protein coding sequence or a sequence of interest that replaces the viral structural protein coding sequence, transcribed from a viral RNA genomic molecule as a template. The template RNA comprises a subgenomic promoter that controls the transcription of a subgenomic transcript. The subgenomic transcript can be obtained in the presence of an RNA-dependent RNA polymerase, in particular a functional alphaviral nonstructural protein. For example, the term "subgenomic transcript" can refer to an RNA transcript produced in an alphavirus-infected cell using the complementary strand of the alphaviral genomic RNA as a template, which does not contain a sequence encoding a viral nonstructural protein. The subgenomic transcript can also be obtained by using the (-) complementary strand of the transcript containing the subgenomic promoter, as a template. Thus, the "subgenomic transcript" refers to an RNA molecule transcribed from a fragment of an alphaviral genomic RNA, and an RNA molecule transcribed from a fragment of a replicon.

The "open reading frame" or "ORF" refers to a continuous base sequence beginning at the start codon and ending at the stop codon, which can encode the entire polypeptide chain. In an mRNA sequence, every three consecutive bases (i.e., triplet "codons") encode the corresponding amino acid. There is a start codon AUG and three stop codons UAA, UAG and UGA. The ribosome initiates translation at the start codon and synthesizes the polypeptide chain along the mRNA sequence, with continuous elongation until encountering a stop codon, whereupon the polypeptide chain elongation terminates.

The "UTR" or "untranslated region" refers to a sequence that is located at both ends of a nucleic acid and is not translated. Specifically, the UTR at the 5' end of the nucleic acid is called the 5' UTR, usually starting from the 5' cap to the start codon AUG; while the 3' UTR usually extends from the stop codon at the end of the coding region to the poly(A) tail. The nucleotide sequences of the UTRs at the 5' and 3' ends of the viral genome are highly conserved, often form stem loops or hairpin structures, and contain cis-acting elements which are mainly responsible for regulating the translation of viral proteins and the replication of viral genomes.

The "5' cap", also known as the 7-methylguanosine cap which is abbreviated as m7G, typically plays a role in the recognition of the RNA during its entry and exit from the nucleus, and in the process of translation, it aids ribosomes in recognizing and binding to the mRNA.

The "poly(A) tail" is a sequence consisting of multiple adenylic acids, which provides resistance to enzymatic degradation in the cytoplasm, and also facilitates transcription termination, nuclear export and translation of an mRNA. The poly(A) tail may refer to either a continuous poly(A) tail or a segmented poly(A) tail. The continuous poly(A) tail can contain consecutive adenylic acids. The segmented poly(A) tail can contain 2-5 fragments of consecutive adenylic acids separated by a spacer sequence, wherein the spacer sequence contains 1-20 nucleotides, each fragment of consecutive adenylic acids contains 10-100 consecutive adenylic acids, the two ends of the spacer sequence are non-A bases, and the middle may be either A bases or non-A bases.

The "internal ribosome entry site" or "IRES" refers to an RNA sequence that forms a secondary structure to recruit a translation initiation complex precursor to a translation initiation codon such as AUG. IRES is usually located in the 5' UTR of RNA viruses and may occur elsewhere in the mRNA. However, an mRNA of a dicistroviridae virus has two open reading frames, and the translation of each open reading frame can be guided by two different IRESs. Some mammalian intracellular mRNAs also contain IRESs, which may be located in mRNAs encoding genes involved in stress responses and other functions that are critical to the survival. IRESs are also present in picornavirus and some pathogenic viruses, including human immunodeficiency virus, hepatitis C virus, hand-foot-mouth disease virus, etc. Although these viral IRESs contain different sequences, many of them have similar secondary structures and initiate translation through similar elements. There are 4 types of IRESs. The common feature of types I-III IRESs is that they initiate translation at the AUG start codon, while type IV IRES initiates translation at non-AUG codons (e.g., GCU). Types I-III IRESs require the delivery of the initiator tRNA of methionine with the help of eIF2/GTP (eIF2/GTP/Met-tRNAiMet). Activation of eIF2 under stress leads to phosphorylation of the α subunit of eIF2, which inhibits translation initiation at AUG. Type IV IRES-directed translation is not inhibited by eIF2 phosphorylation.

The "immunosuppressive protein" refers to a protein that is capable of inhibiting or limiting the production of immune response of a cell or body, such as "interferon inhibitory protein" or "IIP". The immunosuppressive protein or interferon inhibitory proteins can reduce the immunogenicity of self-replicating RNAs by reducing the immune response of the cell or body, such as generation of interferon.

Reference herein to "identity" or "sequence identity" refers to the percentage of nucleotides/amino acids in a sequence that are identical to the nucleotides/amino acid residues in a reference sequence after a sequence alignment, and if desired, gaps are introduced in the sequence alignment to achieve the maximum percent sequence identity between the two sequences. A person skilled in the art can perform pairwise sequence alignment or multiple sequence alignment through various methods, such as computer software, to determine the percentage of sequence identity between two or more nucleic acid or amino acid sequences. Examples of such computer software include ClustalOmega, T-coffee, Kalign, MAFFT and the like.

The term "subject" includes any human or nonhuman animal. The term "non-human animal" includes all vertebrates, for example, mammals and non-mammals, such as non-human primates, sheep, dogs, cats, cattle, horses, chickens, amphibians, and reptiles, although mammals are preferred, such as non-human primates, sheep, dogs, cats, cattle, and horses.

The term "effective amount" refers to an amount of the RNA molecule or RNA combination of the present application sufficient to achieve the expected result. The "therapeutically effective amount" is an amount of the RNA molecule or RNA combination of the present application sufficient to prevent or alleviate symptoms associated with a disease or condition. The effective amount or the therapeutically effective amount is associated with a specific context, where a person skilled in the art can easily determine the actual effective amount.

The "conserved sequence elements" or "CSEs" refers to nucleotide sequences in RNAs of self-replicating viruses such as alphaviruses. These sequence elements are "conserved" because orthologs are present in the genomes of different alphaviruses. In particular, orthologous CSEs from different alphaviruses share a high percentage of sequence identity and/or similar secondary or quaternary structures. The term CSE includes CSE1, CSE2, CSE3, and CSE4.

The "CSE1" is required for the synthesis of a (+) strand from a (-) strand template. The "CSE1" refers to the sequence on the (+) strand, and the complementary sequence of CSE1 on the (-) strand serves as a promoter for synthesis of the (+) strand. In particular, CSE1 comprises the most 5'-terminal nucleotides of the alphaviral genome. CSE1 usually forms a conserved stem-loop structure. Without wishing to be bound by theory, it is believed that the secondary structure of CSE1 is more important than the primary structure. In the genomic RNA of Sindbis virus, CSE1 is composed of a conserved sequence of 44 nucleotides, i.e. composed of the most 5'-terminal 44 nucleotides of the genomic RNA (Strauss & Strauss, (1994) Microbiol. Rev. 58:491-562).

The "CSE2" refers to a nucleotide sequence required for the synthesis of a (-) strand from a (+) strand template. The (+) strand template is usually an alphaviral genomic RNA or RNA replicon. The subgenomic RNA replicon does not contain CSE2, and thus is not used as a template for (-) strand synthesis. In the alphaviral genomic RNA, CSE2 is usually located within the coding sequence of nsP1. In the genomic RNA of Sindbis virus, CSE2 is composed of 51 nucleotides, and is located at nucleotide 155-205 of the genomic RNA (Frolov et al., (2001) RNA 7: 1638-1651). CSE2 usually forms two conserved stem-loop structures. Without wishing to be bound by theory, it is believed that the secondary structure of CSE2 is more important than the primary structure.

The "CSE3" refers to a nucleotide sequence derived from an alphaviral genomic RNA and comprising the start site of a subgenomic RNA. CSE3 initiates transcription of the subgenomic RNA on the (-) complementary strand. In an alphaviral genomic RNA, CSE3 usually overlaps the region encoding the C-terminal fragment of nsP4 and extends to a shorter non-coding region upstream of the open reading frame encoding a structural protein.

The "CSE4" is usually the nucleotide sequence on the alphaviral genomic RNA, adjacent to upstream of poly(A) of the alphaviral genome. CSE4 is usually composed of 19 consecutive nucleotides. Without being bound by theory, CSE4 is thought to be the core promoter that initiates (-) strand synthesis (José et al., (2009) Future Microbiol 4:837-856); and/or CSE4 and poly(A) tail sequences in the alphaviral genomic RNA are thought to work together for efficient (-) strand synthesis (Hardy & Rice, (2005), J. Virol. 79: 4630-4639).

The "cooperate with an RNA replicase" refers to the presence, in an RNA molecule, of a sequence, e.g., CSE1, CSE2, CSE3, and/or CSE4, which the RNA replicase can recognize and bind to, so that the RNA replicase can then begin the amplification process via these sequences.

The alphavirus is a packaged sense strand RNA virus whose host includes many organisms, including insects, fish, mammals, such as livestock and humans. The alphavirus can replicate in the cytoplasm of infected cells. Many alphaviruses have a genome length in the range of 11,000 to 12,000 nt, and the genomic RNAs usually have a 5' cap, and a 3' poly(A) tail. The genome of the alphavirus encodes non-structural and structural proteins, in which the nonstructural proteins are involved in the transcription, modification and replication of the viral RNA and protein modification and the like, while the structural proteins are used to form viral particles. There are usually two open reading frames (ORFs) in the genome. The four nonstructural proteins (nsP1-nsP4) are usually encoded by the first ORF near the 5' end of the genome, while the structural proteins are encoded by the second ORF. Typically, the first ORF is larger than the second ORF.

In cells infected with alphaviruses, only nonstructural proteins are translated from the genomic RNAs, while structural proteins are translated from subgenomic transcripts. After infection, and during the initial stage of the viral lifecycle, the first ORF of the (+) strand genomic RNA is directly translated. In some alphaviruses, there is a UGA stop codon between the coding sequences of nsP3 and nsP4. When translation terminates at UGA, polyprotein P123 is produced, and when UGA is translated, polyprotein P1234 is produced. The nsP1234 is hydrolytically cleaved into nsP123 and nsP4. Polypeptides nsP123 and nsP4 form a (-) strand RNA-dependent RNA polymerase complex that transcribes a (-) strand RNA using a (+) strand genomic RNA as a template. Typically, at a later stage, nsP123 is completely cleaved into single proteins nsP1, nsP2 and nsP3. The 4 proteins combine to form a (+) strand RNA-dependent RNA polymerase complex that transcribes new (+) strand genomic and subgenomic RNAs using the (-) strand RNA as a template. The subgenomic RNA, as well as the new genomic RNA, has a 5' cap added by nsP1 and a poly(A) tail added by nsP4. Both the subgenomic RNA and genomic RNA are similar in structure to an mRNA.

The synthesis of an alphaviral RNA is regulated by cis-acting RNA elements, including four conserved sequence elements (CSEs). The alphaviral genome contains the four CSEs, which are important for viral RNA replication in host cells. CSE1, located at or near the 5' end of the viral genome, is considered a promoter in synthesizing the (+) strand from the (-) strand. CSE2, downstream of CSE1, near the 5' end, within the coding sequence of nsP1, is considered as a promoter in synthesizing the (-) strand RNA from the genomic RNA. The subgenomic RNA transcript does not contain CSE2, and thus is not used as a template for (-) strand synthesis. CSE3, located at the junction of the coding sequences for nonstructural proteins and structural proteins, is the core promoter for efficient transcription of subgenomic transcripts. In some embodiments, the subgenomic promoter is the same as CSE3, or overlaps with CSE3, or comprises CSE3. CSE4, as the 3' untranslated region upstream of poly(A), is considered as the core promoter that initiates (-) strand synthesis. CSE4 and the poly(A) tail are thought to act together for efficient (-) strand synthesis.

Using the self-replicating property of alphaviruses, self-replicating RNA molecules have been constructed that can carry exogenous genes of interest. That is, the genome of a positive-sense single-stranded virus is modified to replace a structural protein sequence of the virus with an exogenous gene of interest. Such self-replicating RNA allows the in vitro synthesized RNA to undergo sustained expansion in large quantities within the cell, achieving long-lasting expression of the exogenous protein of interest. Previously, each dose of the RNA vaccine requires 30-100 micrograms of RNAs, with two injections administered several weeks apart. With such self-replicating RNA, the injection amount can be significantly reduced to a few micrograms.

Currently, self-replicating RNAs engineered with Venezuelan equine encephalitis virus (VEEV), Sindbis virus (SINV) and Semliki Forest virus (SFV) are most commonly used in the art. TC83 is an attenuated mutant strain of VEEV and has a long-standing history of use in FDA IND human clinical studies. In recent years, self-replicating mRNAs based on this mutant have also been used for vaccine studies and have shown good results.

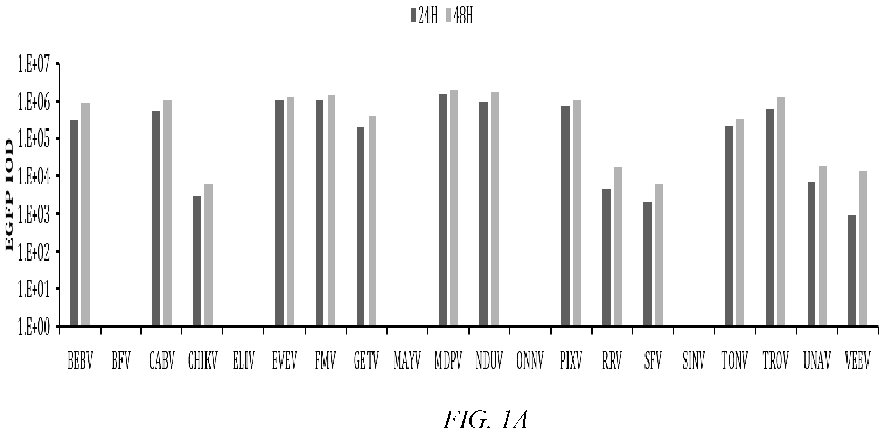

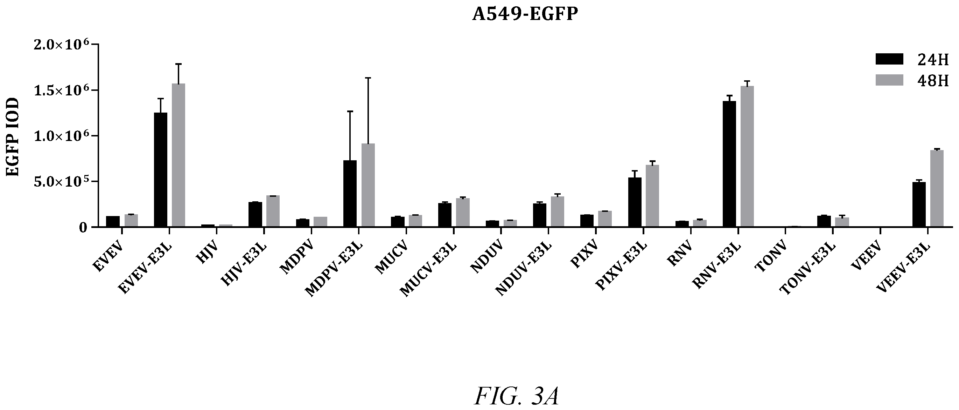

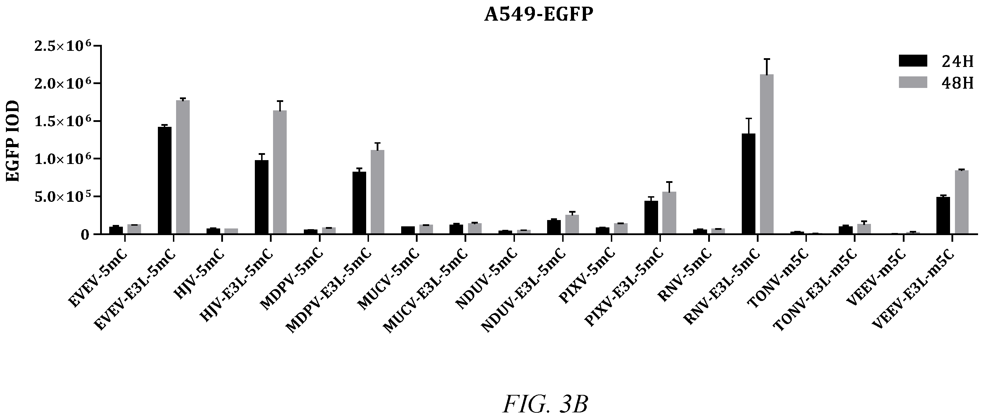

The inventors of the present application screened on numerous members of the Togaviridae family, including Barmah Forest virus (BFV), Bebaru virus (BEBV), Cabassou virus (CABV), Chikungunya virus (CHIKV), Eastern equine encephalitis virus (EEEV), Eilat virus (ELIV), Everglades virus (EVEV), Fort Morgan virus (FMV), Getah virus (GETV), Mayaro virus (MAYV), Mosso das Pedras virus (MDPV), Ndumu virus (NDUV), O'nyong-nyong virus (ONNV), Pixuna virus (PIXV), Ross river virus (RRV), Semliki Forest virus (SFV), Sindbis virus (SINV), Tonate virus (TONV or TV), Trocara virus (TROV), Una virus (UNAV), Aura virus (AURV), Highlands J virus (HJV), Madariaga virus (MADV), Mucambo virus (MUCV), Ruhugu virus (RHGV), Rio Negro virus (RNV), Rustrela virus (RUSV) and Sagiyama virus (SAGV), and the like. Finally, replicons having performance superior to VEEV-TC83 (L01443.1) were screened out. In particular, by replacing a structural protein sequence of each virus with a reporter gene (e.g., an EGFP coding sequence), fluorescence intensity is analyzed by fluorescence microscopy at 24 h and 48 h after transfection of HEK293T or A549 cells, and it is found that self-replicating RNAs based on BEBV, CABV, EVEV, FMV, GETV, MDPV, NDUV, PIXV, TONV, TROV, HJV, MUCV and RNV viruses show significantly higher EGFP expression levels than the self-replicating EGFP RNAs based on VEEV-TC83, as shown in FIGs. 1A-1C. Furthermore, as can also be seen from FIGs. 3A and 3B, VEEV-TC83-based self-replicating RNAs of the same basic structure show much lower EGFP expression level than self-replicating RNAs based on, for example, MDPV, EVEV, HJV, MUCV and RNV. In addition, during the preparation of these self-replicating RNAs through in vitro transcription of relevant synthetic DNAs, it is found that BEBV, CABV, EVEV, FMV, GETV, MDPV, PIXV, TONV, TROV, HJV, MADV, RHGV and SAGV RNA molecules demonstrate high transcription purity.

Thus, in one aspect of the present application, there is provided a replicable RNA molecule, which can comprise, from the 5' end to the 3' end, a 5' cap, a 5' UTR, an open reading frame encoding an RNA replicase, a promoter, a sequence of interest, a 3' UTR and a poly(A) tail, wherein the RNA replicase is capable of amplifying the replicable RNA molecule and an RNA molecule containing the sequence of interest and the 3' UTR, wherein the RNA replicase can be a nonstructural protein or a functional variant thereof derived from Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV).

Nonstructural proteins derived from the above viruses, in addition to the RNA-dependent RNA polymerase activity, have the activity of, for example, a protease, a helicase, a terminal adenylyltransferase, methyltransferase and/or guanylyltransferase. For example, nsP1 can allow the newly generated genomic RNA and subgenomic RNA to have a 5' cap, and nsP4 allows the newly generated genomic RNA and subgenomic RNA to have a poly(A) tail. Thus, the newly generated subgenomic RNA and genomic RNA are similar in structure to an mRNA.

Nonstructural proteins of alphaviruses, may require specific sequences in the genome, such as CSE1, CSE2, CSE3 and/or CSE4 to initiate the amplification program. Thus, the 5' UTR, the promoter, and/or the 3' UTR in a replicable RNA molecule needs to be able to cooperate with the RNA replicase (a nonstructural protein) to facilitate amplification of genomic and subgenomic RNAs. That is, the RNA replicase (a nonstructural protein) can recognize and bind certain sequences in the replicable RNA molecule, thereby initiating amplification. In some embodiments, the 5' UTR, the promoter, and the 3' UTR can be derived from a genome of the same virus as the RNA replicase, such as Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV). The promoter here can be a subgenomic promoter (SGP) of each of the above viruses.

In addition to cis-acting self-replicating RNAs, there are alphavirus-based trans-replication systems that rely on alphaviral nucleotide sequence elements on two separate nucleic acid molecules. In particular, one RNA molecule can encode a viral RNA-dependent RNA polymerase (often called the polyprotein nsP1234), and the other RNA molecule can be trans-replicated (and thus referred to as trans-replication) via the RNA-dependent RNA polymerase. Trans-replication requires the simultaneous presence of the two RNA molecules in the host cell. A nucleic acid molecule capable of being trans-replicated by an RNA-dependent RNA polymerase must contain certain alphaviral sequence elements that allow the RNA-dependent RNA polymerase to recognize the molecule and perform RNA synthesis.

The Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV) found in the present application is also suitable for constructing a trans-replication system. In particular, the present application can provide an RNA combination that comprises a first RNA molecule and a second RNA molecule. The first RNA molecule can comprise, from the 5' end to the 3' end, a 5' cap, a 5' UTR, an open reading frame encoding an RNA replicase, a 3' UTR, and a poly(A) tail. The second RNA molecule can comprise a 5' cap, a 5' UTR, a conserved sequence element, a promoter, a sequence of interest, a 3' UTR, and a poly(A) tail. The RNA replicase can be a nonstructural protein or a functional variant thereof derived from Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV). The RNA replicase in the first RNA molecule is capable of amplifying the second RNA molecule. The 5' UTR, the conserved sequence element, the promoter, and/or the 3' UTR in the second RNA molecule can cooperate with the RNA replicase to facilitate amplification of the second RNA molecule. In particular, the 5' UTR, the conserved sequence element, the promoter, and the 3' UTR in the second RNA molecule can be derived from a genome of the same virus as the RNA replicase, such as Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV). The promoter can be a subgenomic promoter of a virus.

In some embodiments, the second RNA molecule can comprise, from the 5' end to the 3' end, a 5' cap, a 5' UTR, a conserved sequence element, a promoter, a sequence of interest, a 3' UTR and a poly(A) tail. In some embodiments, the second RNA molecule can comprise, from the 5' end to the 3' end, a 5' cap, a 5' UTR, a first conserved sequence element, a second conserved sequence element, a promoter, a sequence of interest, a 3' UTR and a poly(A) tail. The first conserved sequence element and the second conserved sequence element may be conserved sequence element 2 and conserved sequence element 3, respectively, derived from each corresponding virus. In some embodiments, the conserved sequence element can overlap completely or partially with the viral subgenomic promoter, and/or the UTR (particularly the 5' UTR).

The RNA replicase in the first RNA molecule can have the ability to amplify the first RNA molecule. In particular, the 5' UTR and/or 3' UTR of the first RNA molecule can be derived from the same virus as the RNA replicase.

The first RNA molecule can also be a non-replicating RNA. The non-replicating RNA can be made to have longer half-life and higher translation efficiency through a series of modifications and optimizations, such as addition of a β-s-ARCA(D2) cap, human α globin 5' UTR, etc. When using such non-replicating first RNA and the above-mentioned second RNA, the overall protein expression effect is comparable to that of a cis-acting self-replicating RNA (Beissert T et al., (2020) Mol Ther. 28(1):119-128).

During the replication of the self-replicating RNA, the RNA polymerase complex first synthesizes a complementary negative-stranded RNA intermediate from a positive-sense-stranded RNA, and then synthesizes two different positive-stranded RNAs using the intermediate as a template. The first positive-stranded RNA is a copy of the original full-length RNA, and the second positive-stranded RNA is composed of numerous subgenomic RNAs encoding target genes. The latter is capped and poly(A) tailed by the RNA polymerase complex, and finally the target proteins are translated.

The translational regulation of subgenome is similar to that of a conventional mRNA, being controlled by capping, a UTR, and a poly(A) tail. The inventors of the present application try to add a 5' UTR downstream of the subgenomic promoter to see if it could further increase the protein expression level of exogenous genes in a self-replicating RNA.

Thus, in one aspect, the present application provides a replicable RNA molecule that can comprise, from the 5' end to the 3' end, a 5' cap, a 5' UTR, an open reading frame encoding an RNA replicase, a promoter, a second 5' UTR, a sequence of interest, a 3' UTR and a poly(A) tail, wherein the RNA replicase is capable of amplifying the replicable RNA molecule and an RNA molecule containing the second 5' UTR, the sequence of interest and the 3' UTR. The RNA replicase can be a nonstructural protein or a functional variant thereof derived from a self-replicating virus. The promoter can be a subgenomic promoter of a virus.

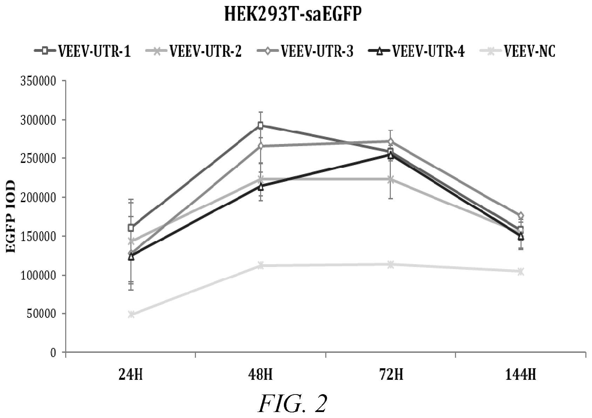

The inventors have selected 4 different UTRs comprising a nucleotide sequence set forth in SEQ ID NO: 5, 6, 7 or 8 respectively. It was found that, regardless of which UTR is added downstream of the subgenomic promoter, the expression level of EGFP after transfection of cells is greatly increased compared to an RNA without a UTR between the subgenomic promoter and the sequence of interest, as shown in FIG. 2.

In addition, similarly to the above, the 5' UTR, the promoter, and/or the 3' UTR in the replicable RNA molecule need to cooperate with the RNA replicase to facilitate amplification of the replicable RNA molecule, and/or the RNA molecule containing or composed of the second 5' UTR, the sequence of interest and the 3' UTR. In particular, the 5' UTR, the promoter, and the 3' UTR can be derived from a genome of the same self-replicating virus, such as a genome of an alphavirus, a flavivirus, a measles virus or a rhabdovirus. In some embodiments, the 5' UTR, the promoter, and the 3' UTR can be derived from the same genome of an alphavirus as the RNA replicase. In some embodiments, the 5' UTR, the promoter, and the 3' UTR, along with the RNA replicase, can be derived from Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV). The promoter can be a subgenomic promoter of a virus. Replication of a self-replicating RNA begins with the synthesis of a complementary negative-stranded RNA intermediate by an RNA polymerase complex using a positive-sense-stranded RNA. Such dsRNA amplification intermediate can be recognized by a natural immune-related signaling pathway of the cell, inducing a strong natural immune response, e.g., inducing type I interferon response through endosomal sensing mediated by TLR3, TLR7 and TLR8, and cytoplasmic sensing mediated by MDA5, RIG-I, PKR, OSA, etc., which is also the main reason for the strong immunogenicity of a self-replicating RNA. While this may be beneficial in terms of recruiting and activating antigen-presenting cells and cells of the adaptive immune system, interferon activation can induce the translational repression and degradation of intracellular mRNAs, including translational repression and degradation of genomic RNAs and subgenomic RNAs of self-replicating RNAs. Therefore, how to control the immunogenicity of self-replicating RNA to promote the recruitment and activation of downstream immune responses while reducing negative effect on the antigen expression remains a problem to be solved in the art. In 2017, Ugur Sahin, in order to relieve the inhibitory effect on the saRNA translation, co-delivered a non-replicating mRNA combination encoding immune escape proteins E3/K3/B18 of vaccinia virus with an saRNA encoding luciferase for the first time. This approach significantly inhibited the PKR and IFN pathways in the cell, and significantly enhanced the in vivo translation efficiency of saRNA-encoded luciferase in mice. In 2021, Robin J. Shattock et al. utilized 2A peptide-mediated cis-expression of innate immune interferon inhibitory proteins (IIPs) to screen for IIPs capable of effectively improving both target protein expression and immunogenicity of saRNAs, and demonstrated that cis-encoded parainfluenza virus PIV5 protein and MERS ORF4a protein could increase protein expression of exogenous genes at cellular level and in mice, and could reduce the immunogenicity of saRNAs encoding rabies virus G glycoprotein in rabbits.

In the present application, the inventors have attempted to reduce the immunogenicity of a self-replicating RNA molecule by adding an exogenous gene and an immunosuppressive protein coding sequence in an open reading frame of the subgenome, and an IRES therebetween. As a result, it was found that after such self-replicating RNA was transfected into cells for 48 h, the expression level of IL-6 in the cells was comparable to that of the blank/negative control, and the cell viability was high at both 24 h and 48 h post-transfection which was comparable to that of the blank/negative control. It can be seen that the cis-expression of the immunosuppressive protein can reduce the immunogenicity induced by the self-replicating RNA, reduce the natural cell immunity caused thereby, and minimize the toxicity to the cell.

In addition, the inventors have found that by adding an IRES between the exogenous gene and the immunosuppressive protein coding sequence, as compared to the 2A peptide used in other studies, no additional amino acid remains on the protein encoded by the exogenous gene, and no uncleaved fusion protein is present, resulting in higher safety.

Thus, in one aspect, the present application provides a replicable RNA molecule that can comprise, from the 5' end to the 3' end, a 5' cap, a 5' UTR, an open reading frame encoding an RNA replicase, a promoter, a first sequence of interest, an internal ribosome entry site (IRES), a second sequence of interest, a 3' UTR and a poly(A) tail, wherein the RNA replicase is capable of amplifying the replicable RNA molecule and an RNA molecule containing the first sequence of interest, the internal ribosome entry site (IRES), the second sequence of interest and the 3' UTR. The RNA replicase can be a nonstructural protein or a functional variant thereof derived from a self-replicating virus.

In particular, the 5' UTR, the promoter, and the 3' UTR can be derived from a genome of the same self-replicating virus, such as a genome of an alphavirus, a flavivirus, a measles virus or a rhabdovirus.

One of the first and second sequences of interest can be an open reading frame encoding an immunosuppressive protein. For example, the first sequence of interest is an open reading frame encoding a peptide or protein of interest, and the second sequence of interest is an open reading frame encoding an immunosuppressive protein; alternatively, the first sequence of interest is an open reading frame encoding an immunosuppressive protein, and the second sequence of interest is an open reading frame encoding a peptide or protein of interest. The peptide or protein of interest can be a disease-associated antigen, or a therapeutic agent.

Compared to a traditional self-replicating RNA, the self-replicating RNA of the present application is not only suitable for applications such as tumor immunity or vaccines, but also suitable for applications such as antibody immunotherapy, protein replacement therapy, and gene editing due to its lower immunogenicity. For example, in gene editing, a self-replicating RNA can be used to express Cas9 protein intracellularly.

Beneficial technical effects of the present invention include: 1) screening out new viral replicons with higher replication and expression capacity and/or in vitro transcription efficiency; 2) further enhancing the expression of an exogenous protein by introducing a 5' UTR sequence of a conventional mRNA in front of a sequence of interest in a self-replicating RNA; and 3) reducing the immunogenicity of a self-replicating RNA and further enhancing the expression of an exogenous protein by cis-expressing an immunosuppressive protein via an IRES element.

The greatest challenge for a self-replicating RNA molecule as a therapeutic or prophylactic agent lies in how to deliver a sufficient amount of RNA molecules to a target cell or tissue. Self-replicating RNA constructs are large anionic molecules of approximately 9,000-15,000 nt in length, which can not be efficiently internalized by cells. Three main delivery platforms are multimeric nanoparticles, lipid nanoparticles and nanoemulsions, although naked saRNAs can also be used. The delivery strategy is essentially to concentrate the anionic saRNAs with cationic carriers into nanoparticles of about 100 nm, which can protect the saRNAs from degradation and facilitate their uptake into target cells (Blakney AK, Ip S, Geall AJ. (2021). Vaccines (Basel). 9(2):97). Animal immunization in vivo with the self-replicating RNAs encapsulated in lipid nanoparticles in the present application can more durably induce antibody production.

The technical solutions of the present invention are further illustrated in detail by the examples and in conjunction with the accompanying drawings. Unless otherwise specified, the methods and materials in the examples described below are conventional products that can be purchased from the market. A person skilled in the art of the present invention would understand that the methods and materials described below are exemplary only and should not be considered as limiting the scope of the present invention.

### Example 1. Construction and characterization of self-replicating RNAs based on viral replicons

The replicons of alphavirus family including Barmah Forest virus (BFV), Bebaru virus (BEBV), Cabassou virus (CABV), Chikungunya virus (CHIKV), Eastern equine encephalitis virus (EEEV), Eilat virus (ELIV), Everglades virus (EVEV), Fort Morgan virus (FMV), Getah virus (GETV), Mayaro virus (MAYV), Mosso das Pedras virus (MDPV), Ndumu virus (NDUV), O'nyong-nyong virus (ONNV), Pixuna virus (PIXV), Ross river virus (RRV), Semliki Forest virus (SFV), Sindbis virus (SINV), Tonate virus (TONV or TV), Trocara virus (TROV), Una virus (UNAV), Aura virus (AURV), Highlands J virus (HJV), Madariaga virus (MADV), Mucambo virus (MUCV), Ruhugu virus (RHGV), Rio Negro virus (RNV), Rustrela virus (RUSV) and Sagiyama virus (SAGV) were tested, to screen for viruses more suitable than the attenuated Venezuelan equine encephalitis virus (VEEV) mutant strain TC83 for constructing self-replicating RNAs expressing exogenous proteins.

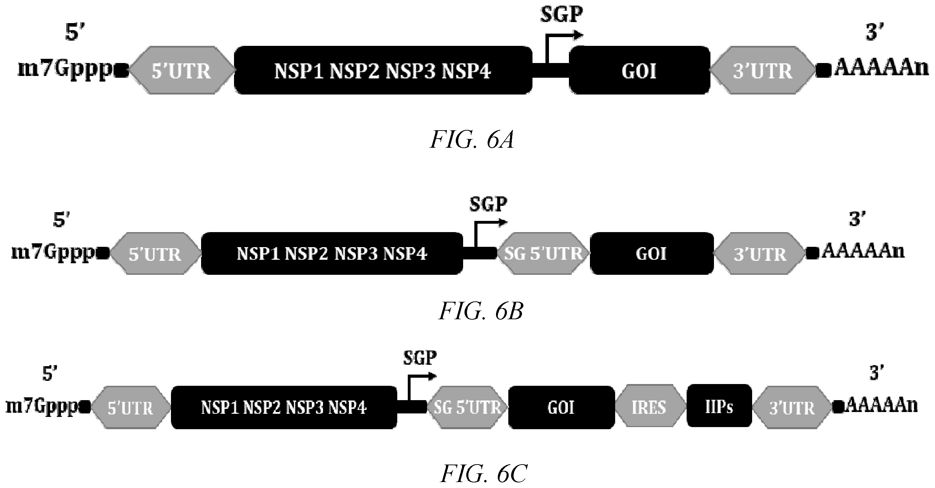

Briefly, the corresponding DNA genomic sequence of each of the above-mentioned viruses was taken, a T7 promoter was added to its 5' end, a sequence encoding a viral structural protein in the genome was replaced with a sequence encoding EGFP, and a polyA sequence composed of 68 As was added to the 3' end of the genomic sequence, as shown in FIG. 6A. That is, the modified coding strand DNA fragment comprises, from the 5' end to the 3' end, the T7 mini promoter sequence (SEQ ID NO: 1), the viral 5' UTR sequence, the sequence encoding viral non-structural proteins nsP1-4, the viral subgenomic promoter, the sequence encoding EGFP (SEQ ID NO: 2), the viral 3' UTR sequence, the polyA sequence, and the restriction enzyme site sequence for plasmid linearization. The corresponding accession number of the genomic DNA sequence of each virus, the position of the nucleotide encoding a structural protein that was replaced with the nucleotide of the EGFP coding sequence in the genomic DNA sequence, and the restriction enzyme site sequence were all listed in Table 1 below.

**Table 1. The corresponding genomic DNA sequences and vector construction information for members of alphavirus family**

| Alphavirus species | Accession numbers of genomic sequences in NCBI or other databases | The position of the nucleotide encoding a structural protein in the genome | Restriction enzyme site sequences |
|---|---|---|---|
| Barmah Forest virus (BFV) | NC_001786 | 7327..11046 | GGAAGAGC |
| | Lee, E et al. Nucleotide sequence of the Barmah Forest virus genome. Virology vol. 227,2 (1997): 509-14. | | |
| Bebaru virus (BEBV) | NC_016962 | 7497..11243 | GGAAGAGC |
| | Forrester, N L et al. Genome-scale phylogeny of the alphavirus genus suggests a marine origin. | | |
| | Journal of virology vol. 86,5 (2012): 2729-38. | | |
| Cabassou virus (CABV) | NC_038670 | 7440..11204 | GGAAGAGC |
| | Kinney, R M et al. Nucleotide sequences of the 26S mRNAs of the viruses defining the Venezuelan equine encephalitis antigenic complex. Am J Trop Med Hyg. 1998;59(6):952-964. | | |
| Chikungunya virus (CHIKV) | NC_004162 | 7567..11313 | GGAAGAGC |
| | Khan, Afjal Hossain et al. Complete nucleotide sequence of chikungunya virus and evidence for an internal polyadenylation site. J Gen Virol. 2002;83(Pt 12):3075-3084. | | |
| Eastern equine encephalitis virus (EEEV) | NC_003899 | 7592..11317 | GGAAGAGC |
| | Volchkov, V E et al. Complete nucleotide sequence of the Eastern equine encephalomyelitis virus genome. Mol Gen Mikrobiol Virusol. 1991;(5):8-15. | | |
| Eilat virus (ELIV) | NC_018615 | 7387..11088 | GGAAGAGC |
| | Nasar F, Palacios G, Gorchakov RV, et al. Eilat virus, a unique alphavirus with host range restricted to insects by RNA replication. Proc Natl Acad Sci U S A. 2012;109(36):14622-14627. | | |
| Everglades virus (EVEV) | NC_038671 | 7516..11280 | GGAAGAGC |
| | Kinney, R M et al. Nucleotide sequences of the 26S mRNAs of the viruses defining the Venezuelan equine encephalitis antigenic complex. Am J Trop Med Hyg. 1998;59(6):952-964. | | |
| Fort Morgan virus (FMV) | NC_013528 | 7470..11186 | GGAAGAGC |
| | Allison, Andrew B et al. Evolutionary genetics and vector adaptation of recombinant viruses of the western equine encephalitis antigenic complex provides new insights into alphavirus diversity and host switching. Virology. 2015;474:154-162. | | |
| Getah virus (GETV) | NC_006558 | 7527..11288 | GGAAGAGC |
| | Submitted (17-DEC-2004) National Center for Biotechnology Information, NIH, Bethesda, MD 20894, USA | | |
| Mayaro virus (MAYV) | NC_003417 | 7417..11145 | ACTAGT |
| | Submitted (04-MAR-2002) National Center for Biotechnology Information, NIH, Bethesda, MD 20894, USA | | |
| Mosso das Pedras virus (MDPV) | NC_038857 | 7607..11386 | GGAAGAGC |
| | Kinney, R M et al. Nucleotide sequences of the 26S mRNAs of the viruses defining the Venezuelan equine encephalitis antigenic complex. Am J Trop Med Hyg. 1998;59(6):952-964. | | |
| Ndumu virus (NDUV) | NC_016959 | 7391..11113 | GGAAGAGC |
| | Forrester, N L et al. Genome-scale phylogeny of the alphavirus genus suggests a marine origin. J Virol. 2012;86(5):2729-2738. | | |
| O'nyong-nyong virus (ONNV) | NC_001512 | 7670..11413 | GGAAGAGC |
| | Levinson, R S et al. Complete sequence of the genomic RNA of O'nyong-nyong virus and its use in the construction of alphavirus phylogenetic trees. Virology. 1990;175(1):110-123. | | |
| Pixuna virus (PIXV) | NC_038673 | 7509..11270 | GGAAGAGC |
| | Kinney, R M et al. Nucleotide sequences of the 26S mRNAs of the viruses defining the Venezuelan equine encephalitis antigenic complex. Am J Trop Med Hyg. 1998;59(6):952-964. | | |
| Ross River virus (RRV) | NC_075016 | 7606..11370 | GGAAGAGC |
| | Jones, Anita et al. Molecular evolutionary dynamics of Ross River virus and implications for vaccine efficacy. J Gen Virol. 2010;91(Pt 1):182-188. | | |
| Semliki forest virus (SFV) | NC_003215 | 7420..11181 | GGAAGAGC |
| | Kääriäinen, L et al. Replication of the genome of alphaviruses. J Cell Sci Suppl. 1987;7:231-250. | | |
| Sindbis virus (SINV) | NC_001547 | 7647..11384 | GGAAGAGC |
| | Strauss, E G et al. Complete nucleotide sequence of the genomic RNA of Sindbis virus. Virology. 1984;133(1):92-110. | | |
| Tonate virus (TONV) | NC_038675 | 7585..11349 | GGAAGAGC |
| | Kinney, R M et al. Nucleotide sequences of the 26S mRNAs of the viruses defining the Venezuelan equine encephalitis antigenic complex. Am J Trop Med Hyg. 1998;59(6):952-964. | | |
| Trocara virus (TROV) | NC_043402 | 7704..11435 | GGAAGAGC |
| | Forrester, N L et al. Genome-scale phylogeny of the alphavirus genus suggests a marine origin. J Virol. 2012;86(5):2729-2738. | | |
| Una virus (UNAV) | NC_043403 | 7640..11389 | ACTAGT |
| | Forrester, N L et al. Genome-scale phylogeny of the alphavirus genus suggests a marine origin. J Virol. 2012;86(5):2729-2738. | | |
| Aura virus (AURV) | NC_003900, Rümenapf T, Strauss EG, Strauss JH. Aura virus is a New World representative of Sindbis-like viruses. Virology. 1995 Apr 20;208(2):621-33. | 7628..11362 | GGAAGAGC |
| Highlands J virus (HJV) | NC_012561, Allison AB, Stallknecht DE. Genomic sequencing of Highlands J virus: a comparison to western and eastern equine encephalitis viruses. Virus Res. 2009 Nov;145(2):334-40. | 7440..11150 | GGAAGAGC |
| Madariaga virus (MADV) | NC_023812, Das,S., Halpin,R.A., Ransier,A. Submitted (14-FEB-2014) J. Craig Venter Institute, 9704 Medical Center Drive, Rockville, MD 20850, USA | 7541..11269 | GGAAGAGC |
| Mucambo virus (MUCV) | NC_038672, Kinney, R M et al. Nucleotide sequences of the 26S mRNAs of the viruses defining the Venezuelan equine encephalitis antigenic complex. Am J Trop Med Hyg. 1998;59(6):952-964. | 7446..11210 | GGAAGAGC |
| Ruhugu virus (RHGV) | NC_076450, Bennett AJ, Paskey AC, Ebinger A, et al. Relatives of rubella virus in diverse mammals. Nature. 2020 Oct;586(7829):424-428. | 6266..9562 | GGAAGAGC |
| Rio Negro virus (RNV) | NC_038674, Kinney, R M et al. Nucleotide sequences of the 26S mRNAs of the viruses defining the Venezuelan equine encephalitis antigenic complex. Am J Trop Med Hyg. 1998;59(6):952-964. | 7526..11302 | GGAAGAGC |
| Rustrela virus (RUSV) | NC_076451, Pfaff F, Breithaupt A, Rubbenstroth D, et al. Revisiting Rustrela Virus: New Cases of Encephalitis and a Solution to the Capsid Enigma. Microbiol Spectr. 2022;10(2):e0010322. | 6124..9555 | GGAAGAGC |
| Sagiyama virus (SAGV) | MW410934, Kim,D., Zhang,Y., Tan,L. and Wang,X. Submitted (24-DEC-2020) Department of Infectious Diseases and Public Health | 7527..11288 | GGAAGAGC |
| Venezuelan equine encephalitis virus-TC83 attenuated strain (VEEV-TC83) | L01443 | 7562..11329 | GGAAGAGC |
| | Kinney, R M et al. The full-length nucleotide sequences of the virulent Trinidad donkey strain of Venezuelan equine encephalitis virus and its attenuated vaccine derivative, strain TC-83. Virology. 1989;170(1):19-30. | | |

The above DNA fragments and their complementary strands were synthesized and cloned into the pUC57-mini-Kana-BsmBI terminator-free T7-deleted vector (GenScript), respectively. The resulting pUC57-saRNA plasmids were transformed into competent cells and inoculated on Kana-resistant plates for screening. Single clones were picked, and clones with correct sequences were selected by Sanger sequencing. All of the above experiments were performed by Nanjing GenScript Biotech Co., Ltd.

The pUC57-saRNA plasmid was taken and digested with the corresponding restriction endonucleases specific for SEQ ID NO: 3 or SEQ ID NO: 4 to linearize the plasmid. The linearized plasmid was recovered by alcohol precipitation twice, was measured for the concentration by Nanodrop, and subjected to 1% agarose gel electrophoresis. The electrophoresis results showed that the linearized product had a single band with no obvious nonspecific band.

The transcription system was configured according to Table 2 (adding CTP or modified 5-Me-CTP) to perform in vitro transcription (ITV) of the obtained linearized plasmid. Specifically, the transcription system in Table 2 was incubated at 37°C for 3 h. Subsequently, 2 µl of DNase I was added, mixed well, and incubated at 37°C for 30 min to obtain the transcription stock solution.

**Table 2. In vitro transcription systems**

| Reagents | Volume or weight |
|---|---|
| 10×T7 transcription buffer (GenScript) | 2 µl |
| ATP (100 mM) | 2 µl |
| UTP (100 mM) | 2 µl |
| CTP (100 mM) | 2 µl |
| CTP or 5-Me-CTP (100 mM) | 2 µl |
| RNase inhibitor (40 U/µl) | 0.5 µl |
| T7 RNA polymerase (200 U/µl) | 0.5 µl |
| Cap analog Cap-AU or Cap-AG (Synthgene, CAP30112, CAP3011) | 2 µl |
| Linearized plasmid | 1 µg |
| Enzyme-free water | Adding to 20 µl |

To the IVT stock solution, 22 µl of enzyme-free water and 20 µl of 8 M LiCl solution were supplemented to make the LiCl concentration be 2.5 M, mixed well and incubated in a refrigerator at -20°C for not less than 30 min. The mixture was centrifuged at 12,000 g for 15 min at 4°C, and the supernatant was discarded. 1 ml of 75% ethanol was added, inverted for mixing uniformly, centrifuged at 12,000 g for 5 min at 4°C, the supernatant was discarded. The process was repeated once more. Centrifugation was performed at 12,000 g for 2 minutes at 4°C, all the supernatant was pipetted, 100 µl of enzyme-free water was added to dissolve the RNAs, and the RNA concentration was detected on a Nanodrop. The results showed that RNA products, including unmodified RNA products and RNA products containing m5C modification, could be transcribed from all viruses except EEEV.

Subsequently, capillary electrophoresis of each RNA product was performed using the Agilent Fragment Analyzer 5200 system. Specifically, the RNA length and integrity were detected using the RNA Analysis Kit (Agilent, DNF-472-1000) according to the instructions of the Agilent Fragment Analyzer 5200 and the RNA kit. The results showed that except for EEEV, which failed to be transcribed successfully, and RRV, which exhibited abnormal peak shapes due to abnormal transcription yields, the integrity of the transcription products in all other groups was not less than 80%. In particular, the purities of unmodified complete products of Bebaru virus (BEBV), Barmah Forest virus (BFV), Cabassou virus (CABV), Chikungunya virus (CHIKV), Eilat virus (ELIV), Everglades virus (EVEV), Fort Morgan virus (FMV), Getah virus (GETV), Mayaro virus (MAYV), Mosso das Pedras virus (MDPV), Ndumu virus (NDUV), O'nyong-nyong virus (ONNV), Pixuna virus (PIXV), Ross river virus (RRV), Semliki Forest virus (SFV), Sindbis virus (SINV), Tonate virus (TONV or TV), Trocara virus (TROV), Una virus (UNAV), Aura virus (AURV), Highlands J virus (HJV), Madariaga virus (MADV), Mucambo virus (MUCV), Ruhugu virus (RHGV), Rio Negro virus (RNV), Rustrela virus (RUSV), Sagiyama virus (SAGV) and Venezuelan equine encephalitis virus (VEEV) were 95.7%, 95.6%, 94.0%, 88.0%, 99.5%, 92.3%, 99.9%, 95.8%, 93.3%, 99.5%, 89.5%, 91.0%, 96.6%, 78.3%, 96.8%, 96.6%, 91.2%, 93.9%, 97.1%, 90.5%, 94.1%, 96.4%, 89.2%, 98.0%, 84.7%, 92.2%, 97.3% and 85.4% respectively; and the purities of m5C-modified complete products of Aura virus (AURV), Bebaru virus (BEBV), Cabassou virus (CABV), Eilat virus (ELIV), Everglades virus (EVEV), Fort Morgan virus (FMV), Getah virus (GETV), Highlands J virus (HJV), Madariaga virus (MADV), Mosso das Pedras virus (MDPV), Mucambo virus (MUCV), Ndumu virus (NDUV), Pixuna virus (PIXV), Ruhugu virus (RHGV), Rio Negro virus (RNV), Rustrela virus (RUSV), Sagiyama virus (SAGV), Tonate virus (TONV), Trocara virus (TROV) and Venezuelan equine encephalitis virus (VEEV) were 85.0%, 95.7%, 94.0%, 92.0%, 95.5%, 94.0%, 95.0%, 93.5%, 84.0%, 95.0%, 90.7%, 96.7%, 92.5%, 96.7%, 80.9%, 97.4%, 98.2%, 88.1%, 97.7% and 85.7% respectively.

Further, the LiCl-purified RNA of each group was transfected into HEK293T cells and A549 cells for expression testing. Specifically, HEK293T cells in the logarithmic growth phase were seeded into 96-well plates at 2 × 10⁴ cells per well and cultured in a 37°C, 5% CO₂ incubator for about 30 h. Transfection was performed using Lipofectamine^{™} MessengerMAX^{™} Transfection Reagent (ThermoFisher, LMRNA015) when the cell confluency reached about 70-90%. The transfection complex was prepared. The amounts of transfection reagents and mRNAs to be added are shown in Table 3. After mixing solution A and solution B, the mixture was incubated at room temperature for 10 min, then 10 µL of the mixture was added to each well. The plate was incubated in a 37°C, 5% CO₂ incubator.

**Table 3. Transfection reagent formulation table**

| | | |
|---|---|---|
| Solution A (OPTI + Lip2000) | OPTI | Lipofectamine^{™} MessengerMAX^{™} |
| | 5 µL | 0.3 µL |
| Solution B (OPTI + mRNA) | OPTI | mRNA |
| | 5 µL | 0.1 µg |

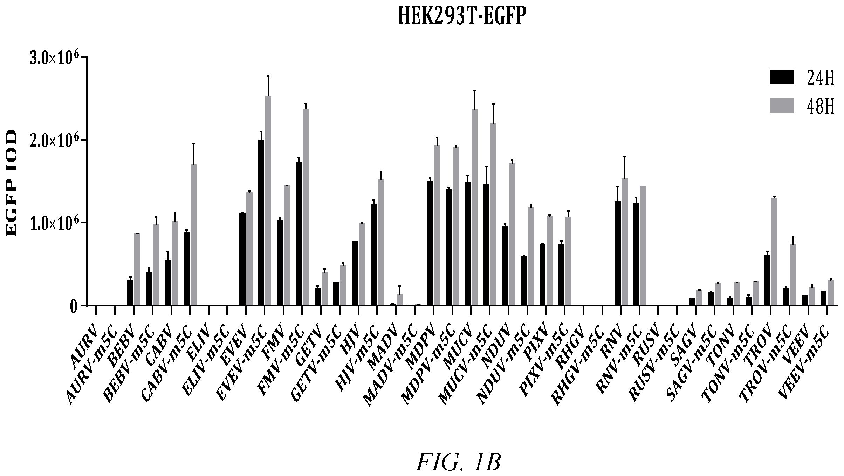

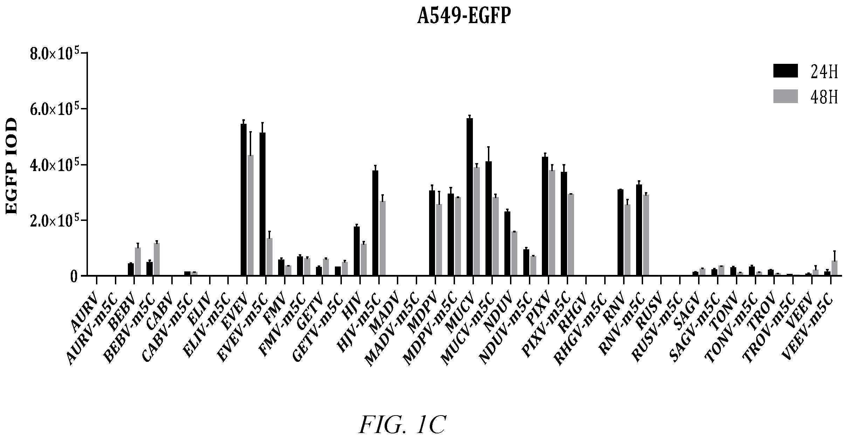

After 24 h and 48 h of culture following transfection, images were captured using a fluorescence microscope (Mshot, MF53-N), and the fluorescence intensity was analyzed. The expression results in HEK293T cells are shown in FIGs. 1A and 1B. For the unmodified and m5C-modified self-replicating RNAs constructed based on viruses BEBV, CABV, EVEV, FMV, GETV, MDPV, NDUV, PIXV, TONV, TROV, HJV, MUCV and RNV, their EGFP expression levels were significantly higher than those of self-replicating RNAs constructed based on other viruses, including the self-replicating RNA based on VEEV-TC83. The expression results in A549 cells are shown in FIG. 1C. The unmodified and m5C-modified self-replicating RNAs constructed from EVEV, HJV, MDPV, MUCV, NDUV, PIXV and RNV exhibit superior expression in A549 cells, which are sensitive to immunogenicity. This indicates that the self-replicating RNAs constructed from these viruses may have lower immunogenicity. As shown in FIG. 1B, the m5C-modified self-replicating RNAs constructed based on viruses BEBV, CABV, EVEV, FMV, GETV, HJV, PIXV, SAGV and TONV exhibit higher expression levels in HEK293T cells than the unmodified self-replicating RNAs.

### Example 2. Addition of a 5' UTR in front of a subgenomic sequence enhances the expression of an exogenous protein encoded by a self-replicating RNA

The DNA fragment constructed based on VEEV-TC83 in Example 1 was taken, and a sequence encoding a 5' UTR was inserted between the viral subgenomic promoter and the sequence encoding EGFP, as shown in FIG. 6B, to test whether the addition of this 5' UTR can enhance the generation of the self-replicating RNA.

The modified DNA fragment comprises, from the 5' end to the 3' end, the T7 mini-promoter sequence (SEQ ID NO: 1), the sequence of the 5' UTR of VEEV-TC83 virus, the sequence encoding non-structural proteins nsP1-4 of the VEEV-TC83 virus, the subgenomic promoter of the VEEV-TC83 virus, the additionally inserted 5' UTR sequence (SEQ ID NO: 5, 6, 7, or 8, as specified in Table 4), the sequence encoding EGFP (SEQ ID NO: 2), the sequence of the 3' UTR of VEEV-TC83 virus, a polyA sequence (68 As), and the BspQI restriction enzyme site sequence for plasmid linearization. The DNA fragment without the newly added 5' UTR was used as a control, designated as VEEV-NC.

As described in Example 1, the above DNA fragment was synthesized, cloned into the pUC57-mini-Kana-BsmBI terminator-free T7-deleted vector, transfected into competent cells, and the vector with a correct sequence was picked. The vector was linearized by digestion with a single enzyme BspQI, and the linearized plasmid was recovered by alcohol precipitation twice. According to the transcription system in Table 2, the obtained linearized plasmid was subjected to in vitro transcription (IVT), and the IVT transcription stock solution was purified by LiCl.

**Table 4. The 5' UTR inserted between the viral subgenomic promoter and the sequence encoding EGFP**

| 5' UTR ID | Sequence of the 5' UTR (the same SEQ ID NO can correspond to both a DNA and an RNA, with the only difference being U and T) |
|---|---|
| UTR-1 | CTTGTCTCGCTCCGGGGAACGCTCGGAAACTCCCGGCCGC CGCCACCCGCGTCTGTTCTGTTACACAAGGGAAGAAAAGC CGCTGCCGCACTCCGAGTGTGCCACC (SEQ ID NO: 5) |
| UTR-2 | CACTCGCGCTGCCATCACTCTTCCGCCGTCTTCGCCGCCAT CCTCGGCGCGACTCGCTTCTTTCGGTTCTACCAGGTAGAGT CCGCCGCCATCCTCCACCGCCACC (SEQ ID NO: 6) |
| UTR-3 | TAGCATTCTTCTGGTCCCCACAGACTCAGAGAGAACCCGC CACC (SEQ ID NO: 7) |
| UTR-4 | GACATTTGCTTCTGACACAACTGTGTTCACTAGCAACCTCA AACAGACACC (SEQ ID NO: 8) |

In addition, as described in Example 1, capillary electrophoresis of each RNA product was performed using the Agilent Fragment Analyzer 5200 system to detect RNA length and integrity. The results showed that the integrity of RNAs containing different newly added 5' UTRs prepared by in vitro transcription was all not less than 70%. Specifically, the purities of the complete products of self-replicating RNAs containing UTR-1, UTR-2, UTR-3, and UTR-4 were 73.1%, 75.1%, 72.9%, and 72.8%, respectively.

Further, as described in Example 1, each LiCl-purified RNA was transfected into HEK293T cells for expression testing. Specifically, EGFP expression in each group was observed at 24 h, 48 h, 72 h, and 144 h post-transfection. The results were as shown in FIG. 2, indicating that the expression levels of self-replicating RNAs with 5' UTRs added in front of the subgenomes were significantly higher than that of the self-replicating RNA without addition of the subgenomic 5' UTR. Additionally, there were no significant difference in protein expression among the groups with different newly added 5' UTRs. The results indicate that adding a subgenomic 5' UTR can improve the translation ability of an exogenous protein encoded by a self-replicating RNA.

### Example 3. Construction and characterization of a self-replicating RNA containing a sequence encoding an immunosuppressive protein

The effect of a signal immunosuppressive protein on the expression of an exogenous protein encoded by a self-replicating RNA was tested by cis-expression of the signal immunosuppressive protein via IRES.

Specifically, a DNA fragment used for transcription of a self-replicating RNA was constructed based on VEEV-TC83, EVEV, HJV, MDPV, MUCV, NDUV, PIXV, RNV or TONV. Sequences encoding IRES and E3L were placed after the stop codon of the reporter gene (EGFP), as shown in FIG. 6C.

The DNA fragment based on VEEV-TC83 comprises, from the 5' end to the 3' end, the T7 mini promoter sequence (SEQ ID NO: 1), the sequence of the 5' UTR of VEEV-TC83 virus, the sequence encoding non-structural proteins nsP1-4 of VEEV-TC83 virus, VEEV-TC83 viral subgenomic promoter, the UTR-1 sequence (SEQ ID NO: 5), the sequence encoding EGFP (SEQ ID NO: 2), the CVB3-IRES sequence (SEQ ID NO: 9), the sequence encoding the vaccinia virus E3L protein (SEQ ID NO: 10), the sequence of the 3' UTR of VEEV-TC83 virus, a polyA sequence (68 As), and the BspQI restriction enzyme site sequence for plasmid linearization.

Each of the DNA fragments based on EVEV, HJV, MDPV, MUCV, NDUV, PIXV, RNV and TONV comprises, from the 5' end to the 3' end, the T7 mini promoter sequence (SEQ ID NO: 1), the sequence of the 5' UTR of each virus, the sequence encoding non-structural proteins nsP1-4 of each virus, the subgenomic promoter of each virus, the UTR-1 sequence (SEQ ID NO: 5), the sequence encoding EGFP (SEQ ID NO: 2), the CVB3-IRES sequence (SEQ ID NO: 9), the sequence encoding the vaccinia virus E3L protein (SEQ ID NO: 10), the sequence of the 3' UTR of each virus, a polyA sequence (68 As), and the BspQI restriction enzyme site sequence for plasmid linearization. Refer to Table 5 for details.

**Table 5. DNA sequences for constructing self-replicating RNAs based on various viruses**

| Alphavirus species | Sequence of viral 5' UTR | Sequence encoding viral nonstructural protein nsP1-4 | Viral subgenomic promoter | Sequence of viral 3' UTR |
|---|---|---|---|---|
| MDPV | SEQ ID NO: 11 | SEQ ID NO: 12 | SEQ ID NO: 13 | SEQ ID NO: 14 |
| PIXV | SEQ ID NO: 15 | SEQ ID NO: 16 | SEQ ID NO: 17 | SEQ ID NO: 18 |
| TROV | SEQ ID NO: 19 | SEQ ID NO: 20 | SEQ ID NO: 21 | SEQ ID NO: 22 |
| CABV | SEQ ID NO: 23 | SEQ ID NO: 24 | SEQ ID NO: 25 | SEQ ID NO: 26 |
| TONV | SEQ ID NO: 27 | SEQ ID NO: 28 | SEQ ID NO: 29 | SEQ ID NO: 30 |
| BEBV | SEQ ID NO: 31 | SEQ ID NO: 32 | SEQ ID NO: 33 | SEQ ID NO: 34 |
| EVEV | SEQ ID NO: 35 | SEQ ID NO: 36 | SEQ ID NO: 37 | SEQ ID NO: 38 |
| FMV | SEQ ID NO: 39 | SEQ ID NO: 40 | SEQ ID NO: 41 | SEQ ID NO: 42 |
| GETV | SEQ ID NO: 43 | SEQ ID NO: 44 | SEQ ID NO: 45 | SEQ ID NO: 46 |
| NDUV | SEQ ID NO: 47 | SEQ ID NO: 48 | SEQ ID NO: 49 | SEQ ID NO: 50 |
| RNV | SEQ ID NO: 51 | SEQ ID NO: 52 | SEQ ID NO: 53 | SEQ ID NO: 54 |
| MUCV | SEQ ID NO: 55 | SEQ ID NO: 56 | SEQ ID NO: 57 | SEQ ID NO: 58 |
| HJV | SEQ ID NO: 59 | SEQ ID NO: 60 | SEQ ID NO: 61 | SEQ ID NO: 62 |
| VEEV | SEQ ID NO: 63 | SEQ ID NO: 64 | SEQ ID NO: 65 | SEQ ID NO: 66 |

As described in Example 1, the above DNA fragment was synthesized, cloned into the pUC57-mini-Kana-BsmBI terminator-free T7-deleted vector, transfected into competent cells, and the vector with a correct sequence was picked. The vector was linearized by digestion with a single enzyme BspQI, and the linearized plasmid was recovered by alcohol precipitation twice. According to the transcription system in Table 2 (with addition of CTP or modified 5-Me-CTP), the obtained linearized plasmid was subjected to in vitro transcription (IVT), and the IVT transcription stock solution was purified by LiCl. Each of the LiCl-purified RNA products (including unmodified RNA products and RNA products containing m5C modification) was transfected into A549 cells for expression testing. As shown in FIGs. 3A and 3B, compared with the absence of cis-expression of E3L, the expression levels of the proteins of interest encoded by unmodified self-replicating RNAs (EVEV-E3L, HJV-E3L, MDPV-E3L, MUCV-E3L, NDUV-E3L, PIXV-E3L, RNV-E3L and TONV-E3L) or m5C-modified self-replicating RNAs (EVEV-E3L-5mC, HJV-E3L-5mC, MDPV-E3L-5mC, MUCV-E3L-5mC, NDUV-E3L-5mC, PIXV-E3L-5mC, RNV-E3L-5mC and TONV-E3L-5mC) with E3L protein cis-expressed via IRES were significantly improved. Moreover, all self-replicating RNAs showed higher EGFP expression at 48 h after transfection into A549 cells.

Additionally, the A549 cells transfected with the above-mentioned RNAs were subjected to detection of IL-6 levels in the culture supernatant at 48 h post-transfection using a human IL-6 ELISA kit, to assess the immunogenicity of each self-replicating RNA.

Specifically, the IL-6 levels in the supernatants of A549 cells transfected for 48 h were detected using a human IL-6 ELISA kit (ThermoFisher, EH2IL6) according to the instruction. A549 cells without transfection treatment were used as the negative control, designated as NC. A549 cells transfected with ordinary linear mRNAs encoding EGFP were also used as a control, designated as EGFP. The ordinary linear mRNA encoding EGFP comprises, from the 5' end to the 3' end, a 5' cap, a 5' UTR (SEQ ID NO: 3), a sequence encoding EGFP (SEQ ID NO: 2, with all T replaced by U), a 3' UTR (SEQ ID NO: 4), and a polyA sequence (100 As).

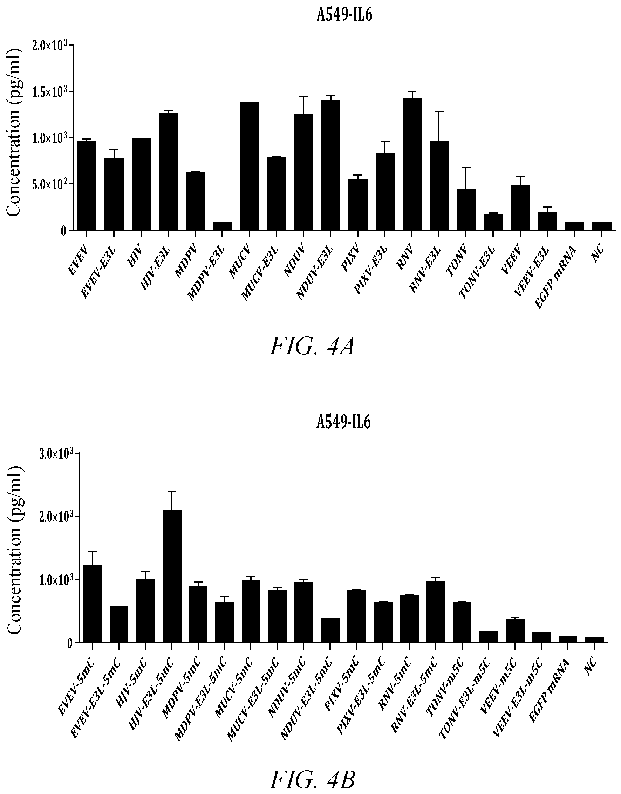

The results of IL-6 expression detection are shown in FIGs. 4A and 4B. Transfection with self-replicating RNAs can result in significantly upregulated expression level of the inflammation-related cytokine IL-6 in cells, while cis-expression of E3L via CVB3 IRES can significantly reduce IL-6 expression in cells in most of unmodified and m5C-modified saRNA transfection groups. However, in some saRNA transfection groups, no reduction in IL-6 levels was observed, and there was even a slight increase. On the one hand, this may be related to differences in the signaling pathways of immune responses induced by saRNAs derived from different viruses. A comprehensive assessment of other inflammatory cytokines such as IFN-α and IFN-β can better reflect the immune response. On the other hand, one of the causes of the high immunogenicity of self-replicating RNAs is the dsRNA structure formed during replication. The addition of immunosuppressive proteins such as E3L to inhibit cell immunity can increase the expression of self-replicating RNAs, and accordingly lead to the production of more dsRNAs, further inducing cellular immune response and affecting the expression of inflammation-related cytokines. In summary, the above results indicate that cis-expression of the immunosuppressive protein E3L can reduce the immunogenicity induced by a self-replicating RNA and suppress the natural cell immunity caused thereby, which significantly improves the expression of the protein of interest encoded by the self-replicating RNA.

Additionally, the LiCl-purified self-replicating RNAs described above were transfected into HEK293T cells, and cell viability was measured by CCK-8 assay at 24 h and 48 h post-transfection.

Specifically, HEK293T cells in the logarithmic growth phase were seeded into 96-well plates at 1 × 10⁴ cells per well. After seeding, the plates were placed in a 37°C, 5% CO₂ incubator and cultured for about 30 h. Transfection was performed using Lipofectamine^{™} MessengerMAX^{™} Transfection Reagent (ThermoFisher, LMRNA015) when the confluency of HEK293T cells reached about 70-90%. The transfection complex was prepared. After mixing solution A and solution B in Table 3, the mixture was incubated at room temperature for 10 min, and 10 µL of the transfection reagent was added to each well. Then the plate was incubated in a 37°C, 5% CO₂ incubator. At 24 h and 48 h post-transfection, 10 µL of CCK-8 (Beyotime, C0038) solution was added to each well, followed by further incubation in the cell incubator for 1 h. The absorbance was measured at OD450 nm. HEK293T cells treated with the transfection reagent only without mRNA addition were used as the negative control, designated as LIP. HEK293T cells transfected with the above ordinary linear mRNA encoding EGFP were also used as a control, designated as EGFP.

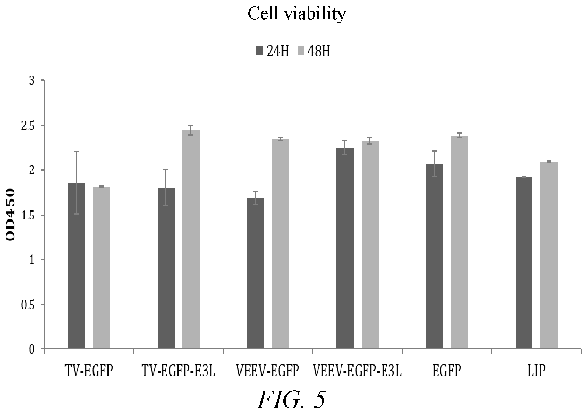

The results are as shown in FIG. 5. At 24 h and 48 h post-transfection, there was no significant reduction in cell viability in each transfection group compared to the negative control, indicating that self-replicating RNAs, with or without E3L expression, do not result in obvious cytotoxicity within at least 48 h post-transfection.

### Example 4. In vivo activity testing of self-replicating RNAs

Immunological effect of a self-replicating RNA in animals was tested, using the SARS-COV2 RBD (delta) antigen designed based on the TONV self-replicating RNA comprising immunosuppressive protein E3L.

To express the SARS-COV2 RBD antigen, we constructed a TONV-based DNA fragment comprising, from the 5' to 3' end, the T7 mini promoter sequence (SEQ ID NO: 1), the viral 5' UTR sequence (SEQ ID NO: 27), the sequence for non-structural proteins nsP1-4 (SEQ ID NO: 28), the subgenomic promoter (SEQ ID NO: 29), the UTR-1 sequence (SEQ ID NO: 5), the sequence encoding RBD protein (SEQ ID NO: 67), the CVB3-IRES sequence (SEQ ID NO: 9), the sequence encoding vaccinia virus E3L protein (SEQ ID NO: 10), the viral 3' UTR sequence (SEQ ID NO: 30), a polyA sequence (68 As), and the BspQI restriction enzyme site sequence for plasmid linearization.

As described in Example 1, the above DNA fragment was synthesized, cloned into the pUC57-mini-Kana-BsmBI terminator-free T7-deleted vector, transfected into competent cells, and the vector with a correct sequence was picked. The vector was linearized by digestion with a single enzyme BspQI, and the linearized plasmid was recovered by alcohol precipitation twice. According to the transcription system in Table 2 (with addition of modified 5-Me-CTP), the obtained linearized plasmid was subjected to in vitro transcription (IVT), and the IVT transcription stock solution was purified by LiCl. The results of 5200 CE showed that the purity of RBD saRNAs was 88.8%.

Through microfluidic technology, lipid components (SM102, cholesterol, DSPC, and DMG-PEG-2000, at a molar ratio of 50 : 38.5 : 10 : 1.5) and RBD saRNA self-assembled to form RNA-LNP complexes. After buffer exchange and concentration of the complexes, the final LNP product was obtained. After preparation, the particle size distribution of the RNA-LNP complexes was characterized by a particle size analyzer using the dynamic light scattering principle. The results showed that the polydispersity index (PDI) of RBD saRNA-LNPs was less than 0.112, demonstrating good dispersibility and uniformity of the LNP particles. The particle size was 82.06 nm. The encapsulation efficiency of LNPs was determined using the Ribogreen method. Ribogreen is an ultrasensitive fluorescent nucleic acid dye used for quantitative detection of the RNA content in a solution, which can not penetrate LNPs. Therefore, the content of RNAs free outside LNP particles in the RNA-LNP complex solution was first detected; then, Triton X-100 was used to disrupt the structure of LNPs, releasing the RNAs encapsulated inside the RNA-LNP complexes into the external solution, so as to detect the total RNA content; and the encapsulation efficiency was calculated based on the difference between the two values. The results showed an encapsulation efficiency of 85.68%, indicating the good saRNA loading capacity of LNPs.

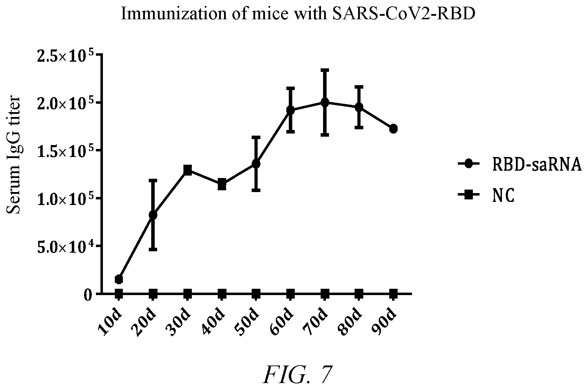

The SARS-CoV-2 RBD-saRNA-LNP prepared above was further subjected to an in vivo immunization experiment in mice, and the specific antibody titers in serum were detected. First, C57/B6 mice were immunized with the SARS-CoV-2 RBD-saRNA-LNP complex via a single tail vein injection at a dose of 0.25 mg/kg, approximately 100 µl. Serum was collected by blood sampling at 10, 20, 30, 40, 50, 60, 70, 80, and 90 days post-immunization, and the titers of SARS-COV-2 S protein-specific antibodies in serum were detected using the Mouse Anti-SARS-CoV-2 Antibody IgG Titer Serologic Assay Kit (Acrobiosystems, RAS-T091). The results showed (FIG. 7) that immunization with a single dose of SARS-CoV-2-RBD-saRNA could induce a high level of specific antibody expression. The specific antibody titers in serum increased persistently for 80 days post-immunization and started to decrease on day 90. Compared with the reported linear mRNA vaccines (where antibody titer peaks about on day 30), the self-replicating RNA vaccine of the present invention can induce more persistent antibody production.

The same SEQ ID NO can correspond to both a DNA and an RNA, with the only difference being U and T.
SEQ ID NO: 1 - T7 promoter
   TAATACGACTCACTATA
SEQ ID NO: 2 - sequence encoding EGFP 
GCCACCATGGTGAGCAAGGGCGAGGAGCTGTTCACCGGGGTGGTGCCCATCCTGGTCG
AGCTGGACGGCGACGTAAACGGCCACAAGTTCAGCGTGTCCGGCGAGGGCGAGGGCG
ATGCCACCTACGGCAAGCTGACCCTGAAGTTCATCTGCACCACCGGCAAGCTGCCCGT
GCCCTGGCCCACCCTCGTGACCACCCTGACCTACGGCGTGCAGTGCTTCAGCCGCTACC
CCGACCACATGAAGCAGCACGACTTCTTCAAGTCCGCCATGCCCGAAGGCTACGTCCA
GGAGCGCACCATCTTCTTCAAGGACGACGGCAACTACAAGACCCGCGCCGAGGTGAA
GTTCGAGGGCGACACCCTGGTGAACCGCATCGAGCTGAAGGGCATCGACTTCAAGGAG
GACGGCAACATCCTGGGGCACAAGCTGGAGTACAACTACAACAGCCACAACGTCTATA
TCATGGCCGACAAGCAGAAGAACGGCATCAAGGTGAACTTCAAGATCCGCCACAACA
TCGAGGACGGCAGCGTGCAGCTCGCCGACCACTACCAGCAGAACACCCCCATCGGCGA
CGGCCCCGTGCTGCTGCCCGACAACCACTACCTGAGCACCCAGTCCGCCCTGAGCAAA
GACCCCAACGAGAAGCGCGATCACATGGTCCTGCTGGAGTTCGTGACCGCCGCCGGGA
TCACTCTCGGCATGGACGAGCTGTACAAGTAA
SEQ ID NO: 3 - 5' UTR in a linear RNA
   AGGGCTAGCATTCTTCTGGTCCCCACAGACTCAGAGAGAACCC
SEQ ID NO: 4 - 3' UTR in a linear RNA 
GCTGGAGCCTCGGTGGCCatGCTTCTTGCCCCTTGGGCCTCCCCCCAGCCCCTCCTCCCC
TTCCTGCACCCGTACCCCCGTGGTCTTTGAATAAAGTCTGA
SEQ ID NO: 9 - sequence of CVB3-IRES 
TTAAAACAGCCTGTGGGTTGATCCCACCCACAGGCCCATTGGGCGCTAGCACTCTGGT
ATCACGGTACCTTTGTGCGCCTGTTTTATACCCCCTCCCCCAACTGTAACTTAGAAGTA
ACACACACCGATCAACAGTCAGCGTGGCACACCAGCCACGTTTTGATCAAGCACTTCT
GTTACCCCGGACTGAGTATCAATAGACTGCTCACGCGGTTGAAGGAGAAAGCGTTCGT
TATCCGGCCAACTACTTCGAAAAACCTAGTAACACCGTGGAAGTTGCAGAGTGTTTCG
CTCAGCACTACCCCAGTGTAGATCAGGTCGATGAGTCACCGCATTCCCCACGGGCGAC
CGTGGCGGTGGCTGCGTTGGCGGCCTGCCCATGGGGAAACCCATGGGACGCTCTAATA
CAGACATGGTGCGAAGAGTCTATTGAGCTAGTTGGTAGTCCTCCGGCCCCTGAATGCG
 
GCTAATCCTAACTGCGGAGCACACACCCTCAAGCCAGAGGGCAGTGTGTCGTAACGGG
CAACTCTGCAGCGGAACCGACTACTTTGGGTGTCCGTGTTTCATTTTATTCCTATACTG
GCTGCTTATGGTGACAATTGAGAGATCGTTACCATATAGCTATTGGATTGGCCATCCGG
TGACTAATAGAGCTATTATATATCCCTTTGTTGGGTTTATACCACTTAGCTTGAAAGAG
GTTAAAACATTACAATTCATTGTTAAGTTGAATACAGCAAA
SEQ ID NO: 10 - sequence encoding E3L 
ATGAGCAAGATCTACATCGACGAGCGGAGCAACGCCGAGATTGTGCGAGGCCATC
AAGACCATCGGAATCGAAGGCGCCACAGCCGCTCAGCTGACCAGACAGCTGAACATG
GAAAAGCGGGAAGTGAACAAGGCCCTGTACGACCTGCAGAGAAGCGCCATGGTGTAC
AGCAGCGACGACATCCCTCCTCGGTGGTTTATGACCACAGAGGCCGACAAGCCTGACG
CCGATGCTATGGCCGACGTGATCATCGACGACGTGTCCCGCGAGAAGTCCATGAGAGA
GGACCACAAGAGCTTCGACGATGTGATCCCCGCCAAGAAGATCATCGATTGGAAGGGC
GCCAATCCTGTGACCGTGATCAACGAGTACTGCCAGATCACCAGAAGAGACTGGTCCT
TCCGGATCGAGAGCGTGGGCCCTAGCAATAGCCCTACCTTCTACGCCTGCGTGGACAT
CGACGGCAGAGTGTTCGATAAGGCCGACGGCAAGAGCAAGCGGGACGCCAAAAACAA
TGCCGCCAAGCTGGCCGTGGATAAGCTGCTGGGCTATGTGATCATCCGGTTCTAA
SEQ ID NO: 11 - sequence of MDPV 5' UTR
   ATGGGCGGCGCATGAGAGATTAGCCCAATACCAAATCATACTACCCAAAT
SEQ ID NO: 12 - sequence of MDPV nonstructural protein nsP1-4 
ATGGAGAAAGTTCACGTTGACATCGAGGAGGATAGCCCCTTCCTCAGAGCTTTACAAC
GGAGCTTCCCGCAGTTTGAGGTAGAAGCCAAGCAGGTCACCGATAACGACCATGCTAA
TGCTAGAGCCTTTTCGCATCTAGCTTCTAAGTTGATCGAAACGGAGGTAGACCCATCCG
ATACGATCCTAGACATAGGCAGTGCACCTGCCCGTAGAATGTATTCTAAACACAAATA
TCATTGTATCTGTCCAATGAAGTGCGCCGAAGACCCGGACAGATTGTTCAAGTACGCG
TCTAAGTTGAAGAGGAATTGTAAGGAGATTACAGATAAGAACTTAGAGAAGAAGATG
GAAGACCTGGCAGCAGTCATGTCAGACCCGAATTTGGAACGAGATTCGATGTGTCTTC
ACGACGACGAATCCTGCCGATTCGAGGGACAAGTGGCCATCTATCAAGATGTGTATGC
AGTGGACGGCCCTACTAGCCTATATCATCAGGCAAACAAAGGAGTCAGAGTCGCATAC
TGGGTAGGCTTTGACACAACCCCGTTTATGTTCAAGAACCTTGCGGGAGCCTATCCCTC
ATACTCCACCAACTGGTCAGACGAAGTGGTGCTGACGGCTCGTAATATCGGGCTGTGC
AGTTCAGACGTCATGGAGCGGTCGAGGAGGGGTCTGTCCATTATGAGGAAAAAGTATT
TGAAACCATCCAATAGTATCATCTTCTCGGTTGGTTCTACTATCTACCACGAGAAGCGT
GATTTATTAAAGAGCTGGCACCTACCGTCCGTGTTCCACCTGCGTGGAAAAAACAACT
ACACATGTCGGTGTGAGACTATAGTTAGTTGCGACGGGTACGTCGTAAAGAGAATAGC
TATTAGTCCAGGCCTGTATGGGAAACCTTCAGGCTACGCATCCACCATGCACCGCGAG
GGTTTCTTGTGTTGCAAGGTGACGGACACGCTTAACGGTGAGAGGGTCTCTTTCCCCGT
GTGCACTTACGTGCCAGCAACTTTGTGCGACCAGATGACAGGTATCCTAGCGACTGAC
GTTAGCGCAGATGACGCTCAGAAGCTGTTGGTTGGGCTCAACCAGAGGATAGTCGTGA
ACGGCCGTACGCAAAGGAATACTAATACTATGAAAAATTATTTGCTCCCAGTTGTAGC
GCAGGCGTTTGCGCGATGGGCTAAGGAGTATAAGGAAGACCAGGAAGATGAAAGACC
CCTAGGGCTGAGAGATCGCCAATTGGTGATGGGTTGCTGCTGGGCTTTCAAAAAACAC
 
AAAGTCACATCTGTGTACAAGAAGCCCGACACTCAGACCATTATTAAGGTGCAAAGCG
ACTTCCATTCGTTCGTGTTACCACGCATTGGAAGCACGTCGCTGGACATAGGTCTGAGA
GACAGGATTCGTAAACTGATGGAGGACAAAAAGAACCCGACACCTATCATCACAGCC
GAAGATGTTCACGAAGCCAAAGCAGCCGCCGACGAAGCACGCGAGGTGAGAGAGGCT
GAAGAACTGAGGGCTGCGCTCCCTCCTCTAGCACCCGATGTAGAGGAGCCGGCCCTGG
AAGCCGAAGTCGACCTGATGCTACAGGAAGCCGGAGCCGGTTCGGTGGAAACACCTC
GGGGTCTTATTAAGGTGACTAGTTACAAAGGTGAGGAGAAGATCGGATCTTACGCTGT
ACTGTCACCTCAAGCAGTGTTAAAGAGCGAAAAATTAAGCTGCATTCATCCATTGGCC
GAACAAGTGGTTGTAGTGACGCACTCTGGCCGGAAAGGGCGATACGCCGTAGAACCTT
ACCATGGTAAAGTAGTGGTTCCTGAAGGGCACGCCATACCCGTGACGGATTTTCAGGC
CATGAGTGAAAGTGCCACCATTGTCTACAACGAAAGAGAGTTTGTGAACAGGTACTTG
CACCACATCGCTGTGCACGGAGGCGCTCTGAATACGGACGAGGAGTACTACAAGGTAG
TGAAACCGTCAGAATACGACGGTGAATATCTGTATGACATAGACAAAAAACAATGTGT
CAAGAAGGAGCAAGTCTCCGGGTTGGGCCTAACGGGTGAGCTGATTGAACCGCCATTC
CACGAGTTCGCCTACGAGAGCCTAAAGACCAGACCAGCAGCACCGTACCAGGTACCCA
CTATTGGAGTGTACGGGGTCCCAGGATCCGGAAAATCAGGCATTATCAAAAGCGCTGT
GACAAAAAGGACCTGGTGGTCAGCGCAAAGAAGGAAAATTGCGCAGAAATCATCCG
GGATGTTAAACGTATGAGGGGGCTGGACGTTACAGCCCGAACTGTGGATTCCGTGTTG
CTAAACGGTTGTAAATATCTGGTGGACACGCTCTATGTGGACGAAGCGTTCGCTTGTCA
CACAGGGACACTGCGTGCACTAATTGCCATTGTCAAACCTAAGAAGACTGTGCTTTGT
GGTGACCCTAAACAGTGCGGTTTCTTCAACATGATGTGTCTTAAGGTGCATTTCAATCA
CGAACTGTGCACCCAAGTGTTCCATAAGAGTATTTCTAGACGGTGTACTCCGGCCGTCA
CCTCCATTGTCTCCACCCTGTTTTATGACAAAAGAATGAGAACGACCAACACACGGAA
TACGAAGATAGAGATAGACACCACCGGTAGTACTAAGCCAAAAAAGGATGATTTGATT
TTAACCTGCTTCAGGGGGTGGGTGAAACAGCTGCAAATTGATTACAAAGGCAATGAAG
TGATGACTGCAGCCGCATCTCAGGGGTTAACCCGTAAAGGTGTCTATGCAGTGCGATA
TAAAGTCAATGAAAATCCACTGTACGCTTCCAACTCCGAGCACGTGAATGTATTGCTG
ACAAGGACGGAAGACAGGATAGTGTGGAAAACCTTGGCCGGTGATCCCTGGGTGAAA
ACATTAACAGCAAGGTACCCAGGAAACTTTTCAGCCACCCTGGAAGAATGGCAACAAG
AACACGACGCCATTATGAAGCACATAATGGAGAAGCCGGATCCTATTAACGTGTTCCA
AAACAAGGCCAACGTGTGCTGGGCCAAAGCACTGGTCCCGATATTACGCACGGCCGGA
ATTAACATGACTGCAGAGCAGTGGAACACGGTACCGTATTTCAAAGAGGACAAGGCA
CACTCAGCCGAAATTGTATTGAACCAGTTGTGCACAAGGTTTTTCGGACTTGATTTAGA
TTCAGGGTTGTTTTCTGCCCCGACGGTACCCCTAGCCATCCGTAACAATCACTGGGACA
ATTCACCGTCTCCCAACATGTACGGACTAAACAGTGAAGTGGTGAGGCAGCTAGTGAA
GCGTTACCCTCAGTTGCCAAAGGCGACGGCGGCTGGAAGGATGTTGGACATGAACACA
GGAAATCTGCGGGATTATGACCCGGTCGTGAACACTGTGCCAGTGAACCGTAGATTGC
CTCACGCTCTGGTAACCGTTCACACACAACAGCCTGCCAGCGACTATTCAGCCTTCGTG
AGCAAACTGAAGGGCAGGACTGTACTTGTGGTGGGGGATAAGATGAACATTCCCGGA
AAAACTGTAGACTGGCTTTCTGAACATCCAGATGCTACCTTCAGATCACGCCTGGATCT
TGGCATACCGGCTGAAGTACCTAAGTATGATCTTGTGTTTGTTAATGTCAGGACACCTT
 
ACATCCACCACCACTACCAACAGTGTGAAGATCACGCTATCAAGCTGAGTATGCTTAC
TAAGAAAGCTTGCTTGCATCTCCAACCGGGCGGCACCTGTGTCAGCATTGGCTACGGG
TATGCTGACAGAGCGAGCGAGAATATAATTGGGGCGATTGCCAGACAGTTCAAATTCA
CCCGAGTTTGTAAGCCGAAGGTATCTCTAGAAGAGACAGAGGTACTTTTGTGTTTATC
GGGTACGACCGGAGGAACAGGACCCACAACCCCTACAAGTTGTCCACCACATTGACTA
ACATATACACAGGATCGGGACTGCATGAAGCCGGCAGTGCACCTTCCTACCACGTGGT
GCGAGGTGATATCGCTACCGCGTCAGAAACTGTTATAGTTAATGCTGCTAATAGTAGG
GGCAACCAGGAGGAGGGGTTTGCGGGGCCCTCTATAAGAAATTCCCGGAGAGCTTCG
ATCTGCAAGCCATAGAGGTCGGCAAGGCACGACTTGTTAAAAACGGGGCGAAGCACA
TCATCCACGCCGTCGGGCCCAATTTCAATAAGGTTGCAGAGGTCGAGGGAGACAGACA
GCTGGCCGAAGCGTATGAATCAATTGCTAAACTGGTTAATGACAATAACTACCGGTCT
GTAGCCATACCTTTGCTTTCGACAGGCATATTTGCAGGAGGCAAGGACCGGCTCATGC
AGTCGGTGAATCACCTTTTGACAGCAATGGACACTACAGATGCCGATGTGGCCATCTA
TTGTAGGGATAAGAAATGGGAGATGACATTAAAGGAAGTAATCGCAAGGAGGGAAGC
CGCAGAAGAGATCTGTATTTCTGATGATGCTAGCGAAGAACCGGATGCGGAACTGGTT
AGGGTTCATCCAAAAAGCTCCTTGGCAGGTAGGAAAGGATACAGCACAGTCGATGGTA
AGACTTTCTCCTACCTGGAGGGCACAAAGTTCCACCAAGCAGCGAAGGACATAGCAGA
AATCAATGCTATGTGGCCGAAGGAAACGGAAGCTAATGAACAGGTCTGCTTGTACATT
CTGGGCGAAAGCATGAGTAGTATACGCTCCAAGTGCCCTGTTGAGGAGTCGGAAGCCT
CTACGCCGCCGACGACGCTCCCATGCCTATGTATCCATGCTATGACCCCCGAGCGGGTC
CAAAGGCTGAAGGCATCTCGCCCTGAACAAATTACCGTCTGTTCATCCTTCGCGTTGCC
GAAGTACCGTATTGCTGGTGTGCAGAAGATCCAGTGCTCTCACCCCATCCTTTTCTCGC
CGAAGGTGCCGGATTACATTCCACCCCGACGTTACTTGCCTGCAACCCTGCCCACTCCT
ACACTTGAGGCACCTGAATTGCCCGCTCCAGCCCTGGAGGTGCATGGGCAGGAGGGGC
AGCCGGAGCGGCCACCCTTAGCTGAATCAGAGCCCGAGGAACAGCCTCTCGAGCACG
AGGAACGGCCTCTCGAGATAACACCGAATGTGATTATCGTGCAGGCGGATGTTCATAT
GGCACCATCTATGTCAGAATGGTCAGTACCATCGGCCTCAGACTTTGATTTGGACAGC
ATCTCCGTCTTGGACGGATCAAGTGAGACCTCTAGTACATCAGACGCTGGCACATTTTC
TATCCGTTCAGCACCTAGTTTGACCGGTAGTGACATTTCAACGAGGAGCAGACACTTTG
CGCACACTACTTCTAATGTCCCTGCGGCATCACAACAGTCTGCAGAACTAACTTTCACT
AATTCGAATATAGCAGCTCGAGCTACGAGTCCTACAAGTACGGCGGTTGTCTCAGCTG
CCGCTTCCAGAGCAAACTCACGAGCTTCCCGCGCTGGCTCAGGAACTGGTTCTGTACG
CTCAGTACATACTACGTCCAGCAACGATACTCGTGCAGAGTTTAGCGCAAACAGGACA
CCGGTAGCTAGCTCCAGATCCAGTTTGACCTCTAATCCACCCGGAGTCAATAGGGTCAT
CACCAGAGAGGAGTTTGAAGCGTTTGTTGCGCAACAGCAATGACGCTATGACGCGGGT
GCATACATCTTCTCTTCGGATACCGGACAAGGACATTTACAACAGAAATCGGTGAGGC
AAACCGTCTTGTCCGAGGTGATTCTAGAGCGCACAGAACTGGAAATTGCGTATGCCCC
GCGCCTCGACTTAGCTAAAGAAGAACTGCTGAGAAAGAAGTTGCAAGTCAATCCAACC
CCTGCTAATCGTAGTCGCTATCAATCGCGCAAAGTAGAGAATATGAAAGCACTCATTA
CGCAGAGGATGCTAAAAGGCTTGGGTCACTACTTGCGTGCAGAAGGGAAAGTCGAAT
GTTATCGTACTTTGTATCCTGTCCCTTTGTACTCAGCTTGTGTAGACCGAGCATTTTCTA
 
ATCCTAAGTTCGCAGTAGAAGCATGCAATGTGATGCTAAAGGAGAATTTTCCTACAGT
TACTTCTTATGGCATAGTACCGGAGTATGATGCCTATTTAGATATGGTAGATGGTGCGT
CGTGTTGCTTAGACACAGCTAGCTTCTGCCCTGCTAAGCTTAGGAGCTTCCCGAAGAAG
CATTCCTATCTGGATCCCACTATACGATCAGCAGTACCATCGGCAATCCAAAATACTTT
GCAGAATGTATTGGCAGCGGCCACTAAGAGAAACTGCAATGTTACACAAATGAGAGA
ACTACCAGTCCTTGACTCTGCAGCCTTTAATGTTGAGTGCTTCAAGAAATACGCCTGTA
ACCAGGAATATTGGGACACATTTAAAGATAATCCTATTAGATTGACAACTGAAAACGT
AACAAATTATATTACAAAGTTAAAAGGACCTAAGGCAGCTGCACTGTATGCAAAGACA
CACAATCTAGCGATGCTGCAGGACATTCCCATGGACCGTTTCGTTATGGATTTGAAGCG
CGACGTTAAGGTTACACCGGGAACAAAGCACACAGAGGAGCGCCCTAAAGTGCAAGT
GATACAGGCTGCCGATCCGTTGGCTACCGCATACCTGTGTGGGATCCATCGTGAGCTG
GTCCGGCGCTTGAACGCAGTGTTACTTCCTAATGTCCACACGCTATTTGATATGTCAGC
AGAAGACTTTGACGCCATCATCGCCGAACACTTTGTGCAAGGCGACAGTGTACTAGAA
ACTGACATTGCGTCGTTCGATAAGAGCGAAGACGACGCAATGGCACTGACCGCACTAA
TGATCCTGGAGGATCTTGGTGTGGATCAGGAGTTGCTGACATTGATAGAAGCAGCATT
TGGAGAGATTTCCTCTGTGCATCTACCCACTAAGACGAAGTTCAAATTTGGAGCCATG
ATGAAATCCGGTATGTTTTTGACATTATTTGTGAATACAATTATCAATATAGTTATTGC
AAGTCGCGTGTTACGCGAAAGATTGACACATTCTCCTTGCTCTGCATTTATTGGTGATG
ATAATATTATAAAAGGAGTCAAATCAGATAAGTTGATGGCTGACAGATGTGCAACCTG
GTTGAACATGGAAGTGAAGATCATTGATGCTGTGGTTGGTGAAAAAGCACCTTATTTTT
GTGGCGGATTCATCCTGTGCGACACGGTTACAGGTACAGCGTGCAGGGTAGCGGATCC
GTTGAAACGGCTGTTCAAGTTGGGTAAGCCGTTGGCCGCTGACGATGAGCATGATGAT
GACCGCCGTAGAGCACTGCAGGAGGAAACTGCTAGATGGAATAGGGTCGGCATCTTTG
AGGAGCTATGTAAGGCAGTTGAATCAAGGTACGAGGTAGTCGGTACATCGGTCATTAT
AATGGCCATGGCTGCACTGGCCAGGAGTACCTCGTCTTTTAGGTGCCTGAGAGGGAAC
CCCATAACCCTCTACGGCTGA
SEQ ID NO: 13 - MDPV subgenomic promoter
   CCTGAATGGATTGCGACGTAGTCCGATCCGCCAAC
SEQ ID NO: 14 - sequence of MDPV 3' UTR 
AGTATGTAGCAATTGGCAAGCTACTTTGTAAAATTTTTATTTGATTTTCCGATTAATTG
GATTTTGTTTTTAATATTTC
SEQ ID NO: 15 - sequence of PIXV 5' UTR
   ATGGGCGGCGCATGAGAGAAGCCCATTTAATATCTACCCAAT
SEQ ID NO: 16 - sequence encoding PIXV nonstructural protein nsP1-4 
ATGGAGAAAGTTCACGTTGATATCGAGGAGGATAGCCCATTCCTCAGAGCTTTGCAAC
GGAGCTTCCCACAGTTTGAGGTAGAAGCAAAGCAGGTCACTGATAACGACCATGCTAA
CGCCAGAGCGTTTTCGCATCTGGCATCAAAGTTGATCGAGACGGAGGTAGATCCATCC
GAGACGATCCTTGATATTGGAAGTGCGCCCGCCCGCAGAATGTACTCTAAGCATAAGT
ACCATTGTATCTGTCCTATGAAATGCGCAGAGGACCCAGACAGACTCTATAAGTACGC
CTCCAAGCTGAAGAAGAACAGTAAGGCGATAACTGATAAAGAGCTGGACAAGAAGAT
GACCGAACTGGCAGCTGTCATGAGTGATCCTGACCTTGAGCAGGACACCATTTGCTTG
 
CATGACGACGAGTCATGCCGTTTTAGCGGCCATATAGCGGTGTATCAGGACGTCTACG
CGGTAGACGGACCAACCAGTCTTTACCACCAAGCTGAAAAAGGGGTGAGAGTGGCCT
ACTGGATTGGGTTTGACACCACCCCTTTCATGTTCAAAAATCTTGCTGGGGCGTACCCG
TCCTACTCCACAAACTGGGCCGACGAGTCAGTGCTAACTGCGCGCAATATAGGACTTT
GCAGCTCCGATGTTATGGAGCGTTCTAGGAGAGGGTTATCTATCCTTAGAAAGAAGTA
TCTGAAACCGACAGACAACATCATCTTTTCAGTCGGGTCTACTATCTACCATGAGAAAC
GAGAGTTGCTGAGAAGCTGGCACTTACCTTCAGTGTTCCACCTACGCGGAAAAAACAG
CTACACATGTCGGTGTGAGACTATAGTTAGTTGCGACGGGTACGTTGTCAAAAGGATA
GCTATAAGTCCAGGCCTGTACGGGAAACCTTCGGGCTACGCCGCAACGATGCATCGCG
AGGGATTCTTGTGTTGCAAGGTGACGGACACACTCAACGGGGAGAGGGTTTCTTTTCC
CGTGTGTACGTACGTGCCAGCTACGCTCTGTGACCAGATGACCGGAATACTGGCTACA
GACGTCAGTGCAGACGACGCTCAAAACTGCTTGTTGGGCTCAACCAGCGGATTGTCG
TCAACGGTCGCACGCAGAGAAATACTAACACTATGAAAATTACCTGCTGCCTATCGT
CGCACAAGCATTTGCGCGGTGGGCCAAAGAGTACAAGGAAGATCAAGAGGATGAAAG
GCCGATGGGACTGCGGGACCGACAATTAGTCATGGGTTGCTGCTGGGCGTTTAAGAAG
CACAAAATAACGTCAGTGTATAAGCGCCCGGATACCCAGACTATTGTCAAAGTGCCCA
GTGATTTCCATTCTTTCGTGCTACCACGTGTTGGGTCATGCACACTGGATATCGGGCTG
AGAGATCGCATTAAGAAGATGCTCGCGGACCGAAAGGAGACCGCGCCGCTGATTACA
GCTGATGACATTGAGGAGGCCAGGAGTGCAGCGGAAGAGGCGAAGGAAGTGCAGGCT
GCTGAGGCTCTGAGAGAGGCCCTTCCTCCTCTCGCCCCGGATGTTGAAGAACCCACCCT
AGAGGCAGACGTAGACCTCATGTTGCAGGAGGCAGGGGCGGGATCTGTCGAGACACC
CCGCGGATTGATTAAAGTCACCAGCTACCCAGGAGAGGAGAAGATTGGATCTTACGCG
GTGTTGTCACCTCAGGCAGTGCTTAGGAGCGAGAAACTGTCCTGCATACACCCCTTGG
CCGAGCAAGTGATAGTGATAACGCACTCGGGACGACGCGGAAGGTACGCTGTCGAGC
CGTATCACGGCAAGGTGGTGGTCCCAGAAGGACATGCCATTCCAGTCCCTGACTTCCA
GGCCTTGAGCGAGAGCGCCACCATCGTGTATAACGAACGGGAGTTCGTAAACAGATAC
CTACATCACATAGCCGTCAATGGCGGAGCTCTGAACACCGACGAAGAGTACTATAAGG
TCGTGAAACCAGAAGACCATGACGGTGAGTATCTGTATGACATAGACAGGAAGCAGT
GTGTCAAGAAAGACCAAGTGCACGGGCTGGGGCTGACCGGTGAGCTGGTAGAGCCGC
CGTTCCATGAGTTTGCATATGAGAGTCTAAGGACCAGGCCTGCAGCCCCCTACCAGGT
GCCAACAATCGGCGTGTATGGTGTGCCCGGGTCGGGCAAATCGGGCATCATCAAGAGC
GCAGTTACCAAGCGAGACCTGGTGGTAAGCGCTAAGAAAGAGAACTGCGCTGAGATC
ATGAGAGATGTCAAGAGGATGCGCAACCTGGAGGTCTCGGCGCGCACAGTGGACTCTG
TGCTGCTAAACGGGTGCAAGCACTCAGTGGAGACCCTGTACGTGGATGAAGCCTTCGC
GTGTCACGCGGGGACTTTACGCGCACTGATTGCAATCGTTAAACCCAGGAAAGCAGTG
CTATGCGGAGACCCCAAGCAGTGCGGCTTCTTTAACATGATGTGCCTAAAGGTGCACT
ACAACCATGAGATATGCACGCAAGTATTCCACAAGAGCATATCACGTAGGTGCACCAA
ATCAGTAACATCTGTCGTGTCGACGTTGTTCTACGACAAGAAGATGAGGACCACGAAC
CCCAGAGAGACACCGATTGAGATAGACACAACGGGCAGCACCAAGCCGAAGAAGGAG
GATCTGATCCTCACATGCTTTAGGGGGTGGGTGAAACAATTGCAGATAGACTACAAAG
GCCACGAGGTGATGACTGCGGCGGCCTCCCAGGGGCTAACGCGAAAGGGCGTTTACGC
 
GGTCAGATACAAGGTAAATGAGAACCCTCTCTACTCACCGACGTCGGAGCATGTCAAT
GTCCTGCTCACGCGTACTGAGGATCGGGTGGTATGGAAGACCCTGGCCGGGGACCCGT
GGATCAAGACACTTACAGCCAAGTACGCGGGCAACTTTAGTGCCACGCTCGAGGAGTG
GCAGGCGGAGCACGACGCCATCATGCAGCACATTATGGAGAAACCTGCACCGGCCGA
CGTCTTTCAGAACAAAGCGAACGTCTGCTGGGCTAAGGCGCTGGTGCCCATTCTCAGG
ACGGCGGGGGTGACGATGACCGCAGAACAGTGGAATACGGTGGGATACTTTAAGGAG
GATAAAGCACATTCGGCCGAAATAGTGCTAAACCAATTGTGTGTGAGGTTCTTTGGAC
TGGATCTTGACTCGGGTCTATTCTCAGCCCCGACAGTCCCGTTGTCAATTAGGAATAAC
CACTGGGACAACTCGCCCTCACCAAACCGATATGGACTGAACAAAGATGTGGTTAGGC
AGCTATCCCACCGGTATCCACAGTTACCCCGCGCGGTTGCGACGGGTCAAGTTTTAGA
CATGAGTTCCGGGACACTGCGGAACTTTGATCCGTGCGTTAACCTGGTCCCGGTGAAC
AGGAGACTGCCACATGCCCTGGTGACTCACCATACCGAGCAGCCGAGAAGCGATTTCT
CTGGTTTTATCGGTAAGTTGAGGGGCCGGACTGTCCTAGTGGTCGGGGACCAGGCCAA
CATACCCGGGAAAGTGGTTGATTGGCTGTCGGACAACCCGGAGGCTAACTACAGGGCC
CGCCTAGATTTAGGCATCCCAAGTGAGATCCCGAAGTTTGACATTGTGTTTGTGAACGT
GAGGACCCCGTATAGATACCACCATTATCAACAGTGTGAGGACCATGCTATCAAACTT
AGCATGCTGACAAAGAAGGCATGCCTGCACCTAAATCCTGGAGGGACTTGTGTCAGCA
TCGGGTACGGGTACGCGGACCGTGCCAGCGAGAGCATTATTGGCGCCGTCGCTAGGCA
GTTTCGGTTTTCGCGTGTCTGCAAGCCGAAGACTTCACTGGAGGAGACAGAGGTGCTA
TTCGTTTTCATAGGGTACGATAGAAGGGCGCGTACCCACAACCCCTACAAACTTTCATC
TACACTTACCAACATATACACTGGAGCAGGTTTGCATGAGGCGGGGTGCGCACCATCG
TACCATGTGGTCCGGGGGGATATTGCCTTGGCGGAAGAGGGAGTGATAGTAAACGCGG
CTAATAGCCGCGGACAGCCAGGCAGCGGAGTGTGCGGAGCGCTGTACAGGAAGTACC
CAGAGAACTTCGACCTGCAACCCATCGAGGTGGGAAAAGCAAGGCTTGTCCAGGGTCC
GTTGAAGCACGTGATACACGCGGTGGGACCGAACTTCAATAAAGTGTCTGAAGTGGAG
GGCGACAGACAGCTGGCTGAGGCATATGAGTCTGTGGCAAAAATTATAAATGATAACC
ACTACCGCTCCGTGGCGGTGCCATTGTTGTCCACCGGAGTATTTGCTGGAAACAAAGA
TAGGCTGATGCAGTCATTTAACCACCTGTTGACTGCGTTGGATTCCACGGATGCGGACG
TGGCAATTTACTGCAGAGACAAAAAATGGGAGACTACACTGAAAGAAGTAATAGCCC
GTAGGGAAGCGACAGAGGAGATTTGCCTCTCCGAGGATGAGTCGCTATGCGAACCTGA
CGCTGAGTTGGTCAGGGTGCACCCGAAGAGTTCACTGGCGGGCAGGAAGGATACAG
TACTACTGATGGAAAGACCTTTTCATATTTGGAGGGGACTAAATTCCACCAGGCGGCA
AAGGATATCGCGGAGATCAACGCGATGTGGCCAGTGGCAGCAGATGCTAACGAGCAG
ATTTGTATGTATATCTTGGGGGAGAGTATGAGTAGCATCAGGTCTAAATGTCCAGTGG
AGGAGTCAGAGGCGTCTATGCCGCCAACGACGCTTCCTTGTTTGTGCATACATGCTATG
ACACCTGAGAGAGTGCAACGCCTGAAAGCCTCACGCCCTGAGCAGATTACAGTGTGCT
CCTCTTTTCCTTTGCCGAAGTATCGCATTGCGGGTGTGCAGAAGATTCAATGTGCTTGC
CCGATACTTTTTTCACCGAAGGTACCTGAACACATACACCCTCGCAGATATATTCAAAC
ACCCACCGTCCAAGAGAGCGGACTACAACAGTGCGACTCAACAGTCGCCCAGGCGGT
GGACGCTGATGGGTCAGCACCTAGTATAACAGTGGATGCGGGCGTGGACCGACTGGAC
ACCGATTCTGTGTGGAGTGACAATCCCATCTTGCCTGGTCCGACCGGGGGCGCGGAGG
 
TACGTCTGGAGGGCATGGATTGGTCAACTGACAGCACCAGGCTCTCTGATGCGTCTGG
CCCGATCGTGATCGGGGCCGAAATCCATGTGGAGCAGAGTTCGCTGTCAGACGTCTCC
ATTAGCGCTTCCACGGAAGATGTGTCCGGTCCAGTTGTGATCAAGGCCGAAATCCACG
CGGAGCAGGGTTCGCTGTCAGACATCTCCGTCGAGTCATGGGAGAGTTATGATGGCAG
CACATCCACGTACGCTGCCATGCGCCCTGTGCCGGCGCCGCGCACTGTGTTTAGGCGCC
CACCAACCCCGATGCCGCGCTCCCTGCCTTTGAAACCGGCCCCGTCCCGGTGCGGGTC
GCAGCTGAGTTTGTCCTCGCGCCCTCCGGGCGTCAACCGTTCTATAACGGTTGAGGAAT
TCGAGGCGTTCATCGCTCAACAGCAATGACGGTTTGACGCGGGTGCATACATCTTTTCC
TCCGACACCGGTCAGGGGCACCTGCAACAGAAATCAGTCCGGCAAACGGTTTTGTCGG
AGGTGGTGCTGGAGAGGACCGAGTTGGAGATAGCGTATGCCCCGCGCCTCGACCTGGA
GAAGGAGGAAGCACTACGGAAGAAGCTGCAGTTGAATGCATCACAGGCAAATAGGAG
CAGGTACTTGTCGCGTAAGGTGGAGAATATGAAGGCAGTAACGACTAATAGGATTTTG
AAAGGCTTGGGTCATTACCTGAGATCAGAGGGACGGGTCGAGTGCTATCGGACCTTGC
ATCCGGTGCCGTTGTACTCCGCGTGTGTTGACCGCGCATTTGCTAGCCCGCGCGTGGCC
ATTGAGGCGTGTAACGTTGTGCTCAAGGATAACTTTCCAACTGTCGCCTCATACTGCAT
CATGCCGGAGTACGATGCATATCTGGATATGGTGGACGGAGCATCTTGCTGCCTTGAT
ACGGCCAGTTTCTGTCCAGCGAAGTTGCGAAGCTTCCCGAAAAACACACTTATTTGG
AACCCACAATAAGATCCGCAGTGCCGTCAGCTATTCAGAACACACTTCAGAACGTCCT
GGCGGCTGCAACGAAGAGGAATTGTAATGTTACCCAAATGAGAGAGCTGCCTGTGTTG
GATTCCGCCGCTTTTAATGTGGAGTGCTTTAAAAAATATGCATGCAATAATGAGTACTG
GGAGACATATCGAGAAAACCCCATTAGATTGACCACAGAAAATGTCACTAACTACATC
ACCAAGCTAAAAGGACCGAAGGCTGCCGCATTGTTCGCTAAGACACACGACCTGACCA
TGCTTCAGGACATCCCGATGGACAGGTTCGTGATGGATTTGAAGCGGGATGTCAAGGT
GACCCCTGGAACTAAACATACTGAGGAGAGACCGAAGGTACAGGTAATACAAGCTGC
CGACCCCTTGGCCACGGCGTACCTGTGTGGGATACACAGGGAGCTTGTCCGCAGGTTG
AACGCAGTGTTATTGCCTAACATCCACACACTGTTTGACATGTCTGCTGAGGACTTCGA
CGCCATCATCGCCGAACACTTCCTGAATGGGGATAGCGTGCTGGAGACTGACATCGCG
TCATTTGATAAGAGCGAGGATGACGCTATGGCTCTTACGGCGCTGATGATACTGGAGG
ACTTGGGTGTGGACCAAGAGTTGTTGACACTGATAGAGGCAGCATTCGGGGAGATTAC
GTCCATTCACCTGCCGACCAAAACTAAATTCAAGTTTGGAGCCATGATGAAATCGGGC
ATGTTCCTGACCCTGTTTGTTAACACAGTCATTAACATCGTTATTGCGAGCAGGGTATT
GAGGGAGCGCTTGACAAATTCACCTTGTGCAGCATTCATCGGAGATGACAACATCGTT
AAAGGGGTAAAGTCTGACAAGCTGATGGCGGACAGGTGCGCCACATGGTTAAATATG
GAGGTCAAGATCATTGATGCGGTGGTCGGCGAGAAAGCACCATATTTTTGCGGCGGAT
TCATCTTGTGCGACACGGTGACTGGAACCGCCTGTAGGGTGGCTGACCCCCTCAAGAG
ATTATTTAAACTGGGCAAACCACTCGCCGTGGACGATGAGCACGATGACGATCGCCGC
CGTGCCCTACACGAAGAGTCACAACGCTGGTGCCGCGTGGGCATATTCGAGGAGCTGT
GTAAAGCTGTGGAGTCCCGGTATGAGACAGTAGGTACCGCAGTGATCGTCATGGCTAT
GGCTACGCTGGCAGGTAGTGTGCAGTCATTCCGCCATCTGCGAGGGGACCCTATATGC
CTCTACGGCTGA
SEQ ID NO: 17 - PIXV subgenomic promoter
   CCTGAATGGACTGCAACATAGTTCAGTCCGCA
SEQ ID NO: 18 - sequence of PIXV 3' UTR 
TCGCAGCATTGCTGCATCTAAATTTTTATAATTTTCTCTGCCAATATTAGATTGGATTTT
GTTTTTAATATTTC
SEQ ID NO: 19 - sequence of TROV 5' UTR
   ATCGAGGCCGTTTTACACACTTTAGCTCCGGCCTCGAAAACGATAGTCACCATC
SEQ ID NO: 20 - sequence encoding TROV nonstructural protein nsP1-4 
ATGTCTGCTGAGAAGCCACAAGTTAGCGTTGACATTGACAGTCAAAGTCAATTTGTTA
ATAAGCTACAATTGTGCTTCCCGCAATTTGAGGTGACTGCACAGCAGGTCACTGCCAA
TGACCATGCTAATGCCAGAGCGTTCTCGCATCTGGCCAGTAAATTAATAGAGCTAGAA
CTGCAAGAAGGTGTAACTGTCCTCGATATCGGTAGTGCACCTGCACGTCGAATGTATTC
GAAGGTGAAATACCATTGCATCTGTCCGATGAGAACACCAGAAGACCCGGACCGCCTC
TATAATTATGCTGATAAACTAATGGCTAAGTATGAAGACATTAAGGACAAGAACTTAG
CAGGCAAACTTAAAGATCTAAGTACCATCCTGGATCGCCCAGATGAGGAAACAGAGA
CTATCTGTTTCCACACTGCCGCTACCTGCAGGATGAGGGCACCAGTAGCTATTATGCAA
GATGTGTACATAGATGCCCCTAGTGCCATATACTATCAGGCACTCAAGGGAGTACGCA
GAATATACTGGATAGGGTTCGACACTACGCAATTTATGTTCGAATCTATGGCAGGAGC
GTACCCGTCCTACGGGACTAACTGGGCTGACGAGAAAGTTCTCCAGGCCCGTAACATC
GGCCTATGCAGTGCACAGTTGCACGAAGAATCAACATCTGGACTATCTATACTGCGGA
AGAAGATAGTCAAGCCGGGCAATAGAGTATTCTTCTCTGTGGGAGGTACTCTGTACCC
CGAGAGTCGTGCCGTCCTGCAAAGTTGGCACTTACCTTCAGTTTTCCATCTAAAGGGTA
AACAAAACTACACATGCAGGTGTGACGTGATGGTCAATTGCGACGGGTACGTCGTCAA
GAAGATAACCATCAGCCCGGGCCTTGTCGGGGATCCGACTGGCTACGCCGTCACTAAT
CACAGCGAGGGATTCTTGCTGTGTAAAACTACGGACACTATTAAAGGGGAGAGGGTAT
CTTTCCCTGTTAGTATGTATGTCCCAGCGGTCATCTGTGATCAGATGACCGGCATTTTG
GCTACCGACATCCAGCCAGAAGATGCACAAAAATTATTAGTAGGATTGAACCAGCGCA
TAGTAGTTAATGGAAAGACTACCAGAAACCAGAACACCATGGCGAACTACTTGTTGCC
AGCTGTCGCTGTTGGATTCAGTAAATGGGCAAAAGAACGGAAGAAGGACTTGGATAA
CGAGAAGCCCTTGTTCACCAGGGAGAGGTCGTTGACCATGTGCTGTATGTGGACATTC
CGCAGAGATAAAATCCATGCATTCTACAGACCACCAGGCACGCAAACTATTGTCAAGA
CGCCATCCAGCTACAGTGCACTACCACTTGCACAGCTATGGACCTCAAGCGTGCCTATT
CCGTTCAGGCAAAAAATGAGCCTATTACTGAGGAAGAAAGTAAAAGAACCGCTTGTA
ACAATCCCCGAGTCAGCTATTGTTTCCGCGGAATTCGCCGAAAAAGAGTATAAGGAAG
AACAGCGGGCGGAAGAACTCAAGGTCGCCCTTCCTCCATTAGCACCTGAGCCTAAGCA
AGAGGAAACACCCAAAGACGAAGCAGCCCTCGTGGATGATATCGGTGCGGCCCTCGT
GGACACTCCACGTGGAGGGGTTCGTATCACCCCTTCCCCTGACAGTCTCATGATAGGG
GACTACTTGGTCATCACACCTCAGGCTGTACTCCAAAATGAGAAACTCTCGAGACTCC
ATCCTTTAGCGGAACAAGTGAAGCTCATAACACACAATGGGAGAAGCGGCAGATATG
CCGTCGAGCGCTACGACGGTAGAGTTCTAGTACCTACAGGTGGGTGCGTGCCATGGGC
TCAGTTCTTGGCCCTTAGCGAGAGTGCAACGCTGGTATACAATGAGAGGGAGTTTGTC
AACAGAAAACTGTACCACATTGCGACACATGGAGGGGCGAAGAACACAGATGAAGAA
 
CACTATCGTGTGTGTAAACCTACAGAAACAGATGACGAGTACGTGTACGACGTGGATG
CCCGCACGTGCGTTAAGAGAGAAGAAGCAGGTACGTTGGCCCTAACTGGAGATCTGAC
GAATCCCCCGTACCACGAACTTGCGTATGAAGGGCTAAAAATTAGACCGACCATACCG
TATAACATTGAAACCATAGGGATCATTGGAGTACCTGGATCGGGAAATCAGCTATCA
TCAAAGCCGCTGTGACATCTCATGATCTGGTGGCCAGCGGTAAGAAAGAAAACTGCAC
CGAGATAGAAAAAGATGTGCTGGCGTCTAGAGGTATCACTATCCGGGCGAGAACGGTT
GATTCCATTCTGCTAAACGGTAGCCCGCGCAGGACCAAGATATTGTATGTCGACGAGG
CATTCGCATGCCATGCCGGTACACTGCTAGCTCTGATAGCTATCGTGAGGCCAACAGA
TAAAGTAGTCCTGTGCGGTGACCCTAAGCAGTGCGGCTTCTTTAACCTAATGCAGTTGC
AGGTGAATTTCAACGACCCTACCAATACCGTCTGTACGGCCACGCACTACAAATACAC
CTCTCGTAGGTGCATCCCAGCAGTTACGGCGGTTGTGTCGACACTACACTATGATGGTA
AGATGAAAACAACTAATCAGAGTAAGAGCAGTATCATCATCGACATTAATGGTACCAC
TAAACCGAAGAAAGGGGATATCATCTTGACGTGCTTCCGAGGTTGGGTAAAACAGCTA
CAACAAGAGTACCCAGGCTTCGAAGTAATGACAGCAGCGGCGTCCCAAGGGCTGACA
AGAAAGGGGGTATATGCTGTTCGTCAGAAAGTGAATGAAAACCCACTGTACTCCACTA
CTTCTGAGCACGTCAACGTATTGCTTACTAGGACCGAAGATCGAATAGTCTGGAAAAC
ACTCCAGGGAGACCCATGGATTAAAACCCTCACAAATATACCCAAAGGACCTTTTACG
GTATCAATAGACCACTGGCACGAAGAACATAGAGCTATACTGGCTTCCATCTCAACAC
CGTCAGTCGAGCGATCACCCTTTAACACCAGAGTACATGTATGTTGGGCAAAAGCGCT
GGAACCTATACTCCAGACTGCTGGGATCAAGCTGACTGAAGACCAATGGGCAGATCTC
TTCCCAGAATTCTTGAGAGACCAAGGCCATTCCGCCCTGTACGCGCTGGACACACTGG
CCATAAAATTCTTCGGGGTCGATTTGACCAGTGGTATATTTCCGCCAAGACGGTCCCT
CTAACATTCCACCCTCGTGCGTCAGGTCGCTCATTGCCGCATTGGGATAATGCGGAGA
GCAATCAGAGGTACGGGTTCAGTGCTATTAGATTGGAATCATTACAGAAGCAGTATCC
TGCCCTTCGAAAGGTGAAACCAGGAATGCAGGTAGACCTGTCTACTGGTATGGTGCAC
GAGGTGACAAGTAAGTGCAATTTAGTTCCTTGGAATAGATCGCTACCGCACACGCTGG
TCCCCACCTACGTGCACAGCGGAACTGGAAACATCAATGAAATACTGAAGAAACTTAG
AGCCAATCGTGTACTAGTGGTTGCCGACAGGAAGCCGGATGCCCCTCAGAAGCAAATC
ACTTGGATTGCCCCAGTGGGAACAAAGGAGGCACCGAAGACAGCTGATCTGACTTATG
GAATACCGGACCAGGGCTCTTTTGCATGTGTGGTTATTGATATAGCCACCCCATATAGA
AATCACCATTTCCAGCAGTGTGAAGATCATGTGTTGAAGATGCGCACGTTGTCCAATTC
CGCGCTCACCATGATCAAACCAGGTGGCACACTAGTGCTGAAATGTTATGGATACGCA
GATAGGAACTCAGAAGATGTCATCACCGCACTCCGTAGAAAGTTTACACGAGTCACTG
CGACTCGCAGCTCATCCCCAGAGAGCAACACGGAGGTAATACTTGCTTTCCATTGCTTC
GATAACAGAAGATGGAGGGACGGAAACTTAATGAATTTGAACAGTAGCGTAACTAGG
CTTTACGAAGGCACATCTACAGGCATCGGCTGCGCTCCGTCTTACCGTGTTAAGCGCAT
GGACATATCCCAAGCAGACGAGCAAGCTGTAGTTAACGCTGCTAACCCGCAAGGTAAA
CCGGGAGACGGAGTCTGCAAAGCAGTGTACAAGAAGTGGCCAGACAGCTTTAAAGAT
ACTAAGACTGAAGTTGGAACTGCAGTTGCTCGTGAAGCCGGGGGTAAACATATCATCC
ATGCCGTCGGTCCGGACTATAGAAGAGTAGCAGACTCGGAAGCAAACACACTACTGCA
GCGCGCGTATTATTCTGCTGCTAAATTAGTGGTGGAAAAGAACATCAAATCTGTAGCT
 
GTCCCACTGCTGTCGACAGGCATCTACTCTGGAGGCAAAGATAGGATGCGTGACTCTT
TAGGCTACTTGTTTACGGCTTTTGATCAGACTGACGCAGATGTCAGTATCTATTGCTTA
GATAAGAAATGGGAAGCCGACATCCAAAGAGCTATAGACCTAAAGGAGTCCGTCACT
GTCTTGGAGGACGTAAACGTCGAATTGGACGCCGAACTTGTACGAGTACACCCGGACA
GCTGCTTAGCGGAACATAAGGGCTACAGTGTGACGGCTGGAAAAACTCCTCTTACTT
GGAAGGGACCAAGTTCCATCAGACATCCCTGGACATTGCTGAGATAGTGGCCATGTTT
CCAAATCGTCAACACGCCAACGAGCAGATTTGTGCGTATGTTTTGGGAGATACAATGG
AGTGCATAAGAGACAAGTGTCCGGTGGCGGACTCACCAGCTTCCGTGCCGCCATGCAC
TCTTCCGTGCATGTGCATGTACGCGATGACTGCCGAACGGATACAGAGATTAAGGGCC
GCTGGGACTAAGCAGTTCACAGTGTGTTCGTCATTTCCGCTGCCTAAGTACAGAATTGT
AGGAGTGCAGAAGATCGTGTGCTCCTCCCCGATCATGTTCAATGACAATGTCCCCGCG
TACATCCCGGCGCGAAAATACGTTCAGGAGCAGCCAGACGGGCAGACAACACTACCA
AGTGTACAGCATAAGGAATCTGCAAGCCCTGCCCAAAGCATCGTCAATTCCACGAAGA
AATTGCTGAATACCGTCCTCAAGATTAACAAAGAAACGAAAGACGACTTATCTGCTCC
CAGCGCCGCGCCTTCACTTAGATCCCAGGAGAGCGAGCCTGAAGATCAGGATGCTGCT
GTAGTGGAACTAGTCCTCGGCGATTCCCCGGCACCTAGAAGGAGGAGGTACGAATCAA
CCGGATCAGATGCAGTTCTACCATCCCTAGAGCTACCAGAAGACTCTGCTAGTTCAGT
GTCTGAGGAGCTCCATATTGTGGAAGTGCATAGATCACTAGGATCCTCCGTCACCACG
CTCTATTCTGAAGAATCCGTCAACTGCATGTCAGAAACAAGCACGGCTCCATCTACGC
CACCACCGACTCCGCCACCCAGACCGAAACGCAAAGCGAAGCTGCTGGACATCGCTAG
GGCACTGATCCCTATACCTGTGGCTCCTATACCGAAACCTAGGACAATGCTTAATGTCC
GACCAATTCCGCTTCCAAGGACAAAACTCCCTAGAACATCATCGACAGACTCCATGAC
ACCTCTGACTTTCGGAGACTGGAACCGTGAGGAGATTGATGGAGTTATTAACCCTGTCT
GCTTCGGTAACTTTCTCCCTGGGGAAGTGGATACTATCTGCAATCGCAACGAATTCTGA
CTGAACGGCGTGGGTGGCTATATTTTTCAACCGATGTAGGGCAAGGCCACCTCCAATT
GCAGTCCGTGCTGTTGAACCGGACAACGGAGTCTGTATTGGAGAAGAATGATCTTAGC
CACCCTTACGCCCCAGTTTTGAATCAGGAGAAAGAGGAGACGTTAAAATTTAAATACC
AGATGAACCCCACGGATACCAACAAAAACAGGTATCAATCTAGGAAAGTAGAAAATC
AAAAAGCTATTACCATCGAGCGACTTCTTAAAGGTCTTCAACTGTATCTGACGCCTGCT
CTACAGCCGGAATGCTACAAGGTCACGTACCCTAGACCGCAGTATTCTCATTCGTACTC
GCTAGGATTCAGTTCCCCGAAGGTGGCCGTGGCTGTGTGTAACAACTTTCTCCACGAG
AACTACCCGACAGTAAGAAACTACCAGATCACAGACGAGTACGATGCATACTTAGATA
TGCTAGACGGTTCGGTCTCTTGCTTGGATACAGCTACGTTTTGTCCTGCGAAGTTGCGC
AGCTTTCCTAAAAAACATAGTTATCTAGAGCCCGAAATCAAGAGCGGGGTTTCTTCAC
CGCTGCAGAACACTCTCCAGAATGTCTTGGCTGCCGCAACTAAACGGAACTGTAATGT
CACGCAGATGCGAGAACTACCCACGTTAGATTCTGCGGTCTTTAACGTTGAGTGCTTCA
AGAAATATGCTTGCAATGATGAATACTGGGATACCTATGCGAAGAACCCTATTAGGAT
CACCACCGAGTACGTCACAGCGTACGTTACGAGACTGAAAGGACCAAAAGCTGCTGCC
CTGTTCGCAAAAACGCATAAGTTAACACCTTTGCAAGATGTACCTATGGACCGGTTCAT
CATGGACCTGAAGAGAGACGTCAAGGTTACTCCAGGCTCGAAGCATACCGAGGAGAG
ACCCAAGGTCCAGGTCATCCAGGCAGCCGAGCCAATGGCAACGGCTTACCTATGCGGC
 
ATACACCGTGAGCTGGTGCGCAGGCTAACAGCCGTTTTGAAACCTAACATCCATACTC
TGTTTGATATGTCTGCGGAAGACTTTGACGCTATAGTCTCCGAACATTTTCACTTTGGA
GACAGCGTGTTGGAAACAGATATTGCGTCCTTTGATAAGAGCCAAGACGACGCTATGG
CTCTCACAGCCTTAATGATCCTGGAAGACCTCGGAGTTGACGACAGACTGCTGGACCT
GATCGAGTGCGCCTTCGGAGAGATTACTAGCACGCACCTCCCAACAAGCACCCGGTTT
AAGTTTGGCGCGATGATGAAGTCGGGAATGTTTCTGACTTTGTTCGTCAACACAGTGCT
GAATGTCGTCATAGCAAGCAGAGTACTAGAGCACCGTCTTACCGATTCCGCGTGCGCT
GCCTTCATAGGCGACGACAACATCATACACGGAGTCGTGTCAGACAAAATAATGTCCG
ACCGCTGTGCCGGGTGGCTGAATATGGAGGTTAAGATCATAGATGCTGTAGTCGGTAT
CTATCCACCATATTTCTGCGGCGGATTTATTTTGCAGGATACAGTCACTGGTACAAGTT
GCAGGGTATCTGACCCTTTGAAGCGCCTGTTTAAATTAGGTAAACCACTTCCGTGCGAT
GACGAGCAAGACGATGATAGACGGCGTGCCTTGTCAGATGAGAGCAAAGCCTGGTGC
AGGATTGGCATCATTCATGAGCTGACGGCCGCAGTAGAGGCCCGCTACGAAGTACATA
ACGTGTACCCAGTCATAGAGGCTCTCTGCACCTTTGCCAAGAGCAAAGCGGCATTTAG
AGTCATTAGGGGGAGACAGGTGGACCTCTACAAGTAA
SEQ ID NO: 21 - TROV subgenomic promoter 
CCTTAATCGTCTGCGTAGTACACAATTGACCACGCAGACACGTTAATTGAGTGACAGC
A
SEQ ID NO: 22 - sequence of TROV 3' UTR 
TAGCCGTGTGCACCTAGTTTAATAATAAGATATATAGTTCAAAGGGAAGAACAACCCC
TGAATAGTAACAAAACACAAAAATAATAAATATAGTTTTAAGGCTTTAGTATTATAAT
AATGATAGTATTAGGTATATTAGTAGTTTAGTAGTAAAATATGTAGTTCAAAGGGAAG
AACAACCCCTGAATAGTAACAAAATACAAAAACAATAAACATAGTTTTAAGGCTTTAG
TATCAAAATAATGATAGTGTTTAATGAATAGTAACAAAATGCATAATTGATATATACG
AATGTAATTAGTGTTAGATATTTAGTTCAAAGGGAAGAACAACCCCTGAATAGTAACA
AAATACAAAAACACCAAATATAGAGTTAAGTTTTTAGTATCAATTAAATTATGAAATT
GATTTGTCAATCACCACCGGACGCCGGGATCGGCGTCCTACCATGTGTAGTTGAAAAC
CGTATAATTTTCTTAAAATTTTCTTATACCGAATTTGATACACAAATTAGACAATCTTTA
ATTTTTCTTTTTATTTTCTTTTTAATTTTCTTTATAAAATTTTAATTTTGTTTTTAATATTC
C
SEQ ID NO: 23 - sequence of CABV 5' UTR
   ATGGGCAATACGTAAGAGTAGCCCAAAAACTGAATACACCTACCCATC
SEQ ID NO: 24 - sequence encoding CABV nonstructural protein nsP1-4 
ATGGAGAAAGTTCACGTTGACATCGAGGAAGACAGCCCGTTCCTTCGAGCTTTACAGC
GGAGCTTCCCACAGTTTGAGGTAGAAGCCAAGCAGGTCACCGATAATGACCATGCTAA
TGCCAGAGCGTTTTCGCATCTGGCCTCCAAATTGATCGAGACGGAGGTAGAACCATCC
GATACGATCCTAGACATAGGCAGTGCACCGGCACGCCGAATGTATTCAAAGAATAAGT
ATCATTGCATTTGCCCAATGAAATGTGCAGAAGATCCGGACAGATTATACAAATATGC
GGCGAAACTAAAGAAAAACTGCAAAGACATCACGGACAAGGACTAGATCAGAAGCT
AGCAGATCTTGCAGCAGTCATGCAGGAACCTTCCTTAGAGCGAGAATCGATTTGTCTTC
ATGACGACGAGTCATGCCGCTATGAAGGGCAGATTGCTGTCTACCAAGATGTCTATGC
 
AGTAGACGGACCTACTAGTCTTTATCATCAAGCCAATAAGGGTGTAAGGGTCGCCTAT
TGGATTGGCTTTGACACTACGCCTTTTATGTTTAAGAATTTAGCCGGCGCATACCCCTC
CTATTCGACCAACTGGGCCGACGAGACAGTGTTAACAGCTCGTAATATAGGCCTATGT
AGTTCCGATGTAATGGAACGGTCCAGGAGAGGACTATCCATCCTTAGGAAGAAATACT
TAAAACCGTCGAACAATATCATATTTCGGTGGGCTCTACTATCTATCACGAAAAGAG
GGACCTACTGCGGAGCTGGCACTTACCATCAGTGTTCCATTTACGCGGTACGCATAATT
ACACGTGTCGGTGTGAGACGATAGTAAGCTGCGACGGGTACGTCGTTAAGAGAATTGC
TATCAGTCCAGGCCTGTACGGGACACCGTCGGGCTATGCGGCCACGATGCATCGCGAG
GGATTCTTGTGTTGCAAAGTCACGGATACACTTAACGGGGAGAGGGTTTCTTTTCCCGT
ATGTACGTACGTGCCTGCTACCTTGTGCGACCAGATGACAGGTATACTGGCGACAGAC
GTAAGCGCAGATGACGCTCAGAAGTTGCTGGTAGGACTTAACCAGCGGATTGTAGTGA
ACGGACGCACTCAAAGAAATACTAATACTATGAAGAATTACTTACTACCAGTAGTGGC
TCAGGCATTCGCCAGGTGGGCAAGAGAGTACAAGGAGGACCAGGACGATGAACGTCC
GCTGGGATTGAGAGATCGCCAGCTGGTGATGGGATGTTGCTGGGCCTTTAAGAAGCAT
AAGATCACTTCTGTGTACAAACGCCCGGATACCCAAACGGTAGTAAAAGTGCCCAGTG
ATTTCCATTCTTTCGTTCTGCCAAGAGTAGGGAGCAGTACTTTGGAAATCGGCCTCAGA
GACCGAATTAAGAAATTGCTGGAGAAACCAAAGGAACCTGTACCGCTGGTTACAGAC
GAAGACATCGGCGAGGCTAAGAACGCAGCTGCTGAGGCGAAAGAAATCAAAGAGGCA
GAAGAATTGCGGGCAGCCTTGCCGCCCCTGACTGCTGACGTTGAAGAACCGACGCTCG
AAGCAGATGTTGACCTCATGTTGCAAGAAGCAGGGGCAGGATCCGTAGAGACGCCGC
GGGGGTTGATCAAAGTCACCAGCTATAGCGGTGAGGAGAAAATCGGTTCCTACGCTAT
ACTCTCACCGCAAGCCGTGTTACGCAGTGAAAAACTTGCGTGTATTCATGCACTCGCTG
AGCAGGTGATCGTGATTACCCATTCCGGCAGGAAAGGTAGGTATGCAGTGGAGCCGTA
TCATGGAAAAGTAATTGTACCTGAGGGCACCGCCATTCCTGTGCCTGATTTTCAGGCAC
TCAGTGAGAGTGCCACGATAGTGTATAACGAACGGGAGTTCGTGAACAGGTACTTGCA
CCATATCGCGGTCCACGGAGGGGCGCTTAATACCGACGAAGAGTACTATAAGACAGTG
AAACCTGGCGACTATGACGGAGAATATCTATATGATATAGACAAAAAACAGTGCGTGA
AGAAAGAACTGGTTAGCGGTCTAGGGATGACGGGTGAACTCATTGAACCCCCATTTCA
TGAGTTCGCGTACGAAAGCCTGAAGACTAGACCTGCCGCACCCCACCAAGTACCTACT
ATTGGGGTGTATGGAGTACCAGGTTCTGGTAAATCTGGAATCATAAAGAGCGCAGTCA
CGAAGAAAGATTTAGTAGTTAGCGCTAAAAAAGAGAATTGTGCGGAAATTATCAGAG
ACGTCAAACGCGCTAGAGGGCTGGACGTCAATGCGCGGACTGTGGACTCCGTACTGCT
GAACGGTTGTAAGTACCCAGTCGAAACCTTATACATTGACGAAGCTTTTGCGTGCCAC
GCAGGAACCCTTAGGGCCTTGATTGCGATTGTCAAGCCCAAGAAAGCCGTGTTGTGCG
GGGACCCTAAGCAATGTGGGTTCTTCAACATGATGTGCCTGAAGGTACACTTCAATCA
TGAAATTTGCACACAAGTGTTCCATAAGAGCATATCGCGTAGATGCACCAAGTCTGTG
ACTTCTGTCGTGTCCACCCTGTTTTACGACAAGAAAATGAGGACAACCAACCAGAAAG
AGACGCAAATCGAAATCGATACCACAGGGAGCACGAAGCCTAAGAAGGAGGATTTGA
TCCTCACTTGCTTCCGAGGGTGGGTAAAGCAACTGCAGATTGACTACAAAGGCAATGA
AATCATGACAGCGGCAGCATCGCAAGGACTAACCCGGAAGGGCGTCTATGCCGTCAG
GTACAAGGTCAATGAGAACCCGCTGTATGCCCCCAATTCCGAACACGTGAATGTTCTG
 
CTGACTCGAACGGAGGACCGAATCGTTTGGAAGACCCTGGCGGGTGACCCCTGGATTA
AGACCCTGACCGCCAAGTATCCAGGTGAATTCTCAGCGACGCTAGAAGAATGGCAAGC
TGAACATGACGCCATTATGAAGCACATCATGGAAGAACCTAACCCTGCCGACGTCTTC
CAGAACAAAGCGAACGTTTGCTGGGCTAAAGCTCTTGTCCCTGTGCTGAAAACAGCCG
GAATTTCCTTGACGGCAGACCAATGGAATACCGTCGAAAATTTTAAAACTGACAAGGC
GCACTCTGCAGAGATTGTGTTAAATCAACTGTGCGTACGGTTCTTCGGGCTAGACTTGG
ATTCAGGTATATTCTCAGCGCCTACAGTCCCGCTGTCTATAAAGAATAATCACTGGGAC
AACTCCCCATCTCCGAATATGTACGGAATCAACATGGACGTGGTGAGGCAGCTTTCTA
GGCGCTACCCGCAGCTGCCACGAGCGGTAACCACAGGGCGAGCGCTGGATATGAACTC
GGGTGCGTTGCGCGCCTACGATCCCCGGATAAACTTAGTGCCTATGAACAGGAGGTTA
CCCCATGCTCTTGTCACCAACCACTCAGAACACCCTCCTAGCGACTTTTCGACATTTGT
TAGTAAACTTAAAGGTCGGACCGTACTAGTGGTCGGAGACAAAATGACCGTGACCGGA
AAGACCGTCGACTGGCTGTCCGAATCCCCCGAAGCTACTTTTCGCACGCGCCTAGACTT
AGGGATTCCAGCAGAGCTTCCTAAGTACGACATTGTCTTCGTCAATGTAAGAACGCCG
TATAGATACCACCACTATCAGCAGTGTGAAGATCACGCTATTAAGCTGAGCATGCTGA
CTAAGAAAGCATGTTTGCACCTCAATCCTGGGGGCACATGCGTCAGCGTGGGATACGG
ATATGCAGACCGAGCCAGTGAAAGCATAATAGGTGCAGTTGCCAGACAGTTCAAATTC
GCCAGAGTGTGCAAACCTAAAATATCCTTAGAAGAGACGGAAGTGCTGTTTGTATTCA
TTGGATATGATCGTAGGACACGCACCCACAACCCGTATAAACTCTCCTCTACGCTAAC
AAACATCTATTCGGGCTCCAACTTTCATGAAGCGGGGTGTGCTCCCTCATACCATGTTG
TCCGAGGGGACATTGCCACCGCAGAAGATGGGGTGATCATTAACGCAGCTAATAGTAG
GGGCCAACCAGGAGGAGGCGTGTGTGGGGCTCTATACAAGAAGTTTCCGGAAAGCTTT
GACCTACAGCCTATAGAAGTGGGTAAGGCGCGCTTGGTAAAGGGTGCGAAAAAACAC
ATCATACACGCGGTCGGACCTAACTTTAACAAGGTGTCAGAAATAGAAGGCGACAAAC
AACTCGCCGAGGCGTATGAATCCGTCGCCAAACTAGTGAATGACAATAATTACCGATC
AGTCGCCATCCCGCTCCTGTCCACAGGCATATTTGCCGGTAATAAAGACCGACTTATGC
AGTCATTAAACCACCTCCTCACTGCCATGGATACGACCGACGCGGATGTCGCTATCTAC
TGTCGCGATAAAAAATGGGAAACTACGCTCAAGGAAGTGGTGGCCCGGAGGGAGGCG
GTAGAGGAGATATGCATATCGGAAGACGCTTCCCTGACCGAGCCTGATGTGGAGTTAG
TTCGGGTTCACCCTAAGAGCTCGCTAGCCGGCAGAAAAGGTTATAGCATCACGGATGG
AAAAACCTTTTCTTACCTAGAAGGAACCAAGTTCCACCAGGCGGCGAAAGATGTGGCC
GAAATAAACGCCATGTGGCCGAATGCAACAGAAGCTAACGAGCAAATATGCCTCTATA
TCCTGGGGGAAAGCATGAGTAGCATAAGATCTAAATGCCCCGTAGAAGAGTCTGAAGC
ATCAACACCACCTAATACACTCCCTTGTTTATGTATCCATGCTATGACACCAGAGCGTG
TTCAGCGGTTGAAGGCATCAAGGCCGGAACAGATCACGGTGTGCTCATCGTTCCCGTT
GCCGAAGTACAGAATAACCGGCGTACAGAAGATCCAATGTTCTTGCCCTATATTGTTCT
CTCCGAAGGTGCCAGAGTACATCCACCCTAGAAGATACCTTCCTCGCCCACTTGAAATT
GAGGTGGAACGTGCAGGAACCCCGGCGCCGGCTTCGAACGTAGAAGCTGCAACGGAA
GAGAACTTTGAAACGATCTCCGTAGTTGACAGCGTAGTTAGCTCGGAGAGTACAGCCG
AAACAAATCTTGACGGACCAAACGCAATCGTAGTAGAGGCAGATGTACACGCAGACTT
GATGCGTTTCTCATCCACCTGGTCTATCCCACACGCTTCCGATTTCGATGCGGACAGCA
 
TCTCCTTGGCTTTGTCAGAGTTTTCCTCGACAGACGCACGAAGCGTAGTTTCCAGTTCT
AGTTCTGATGCGGGCATAAGACCTATCCCTGCTCCTAGGACTATCTTCAGACAGGCGCC
TGTTCCTCGACCTCGCAAAACTAGGAGTTCTAGTGCGTCACTGTCGCCACCGATTCACG
CTCGTAGGCAAGTATCAGGCTCCAGATCCAGTCTGGCATCCAATCCGCCAGGAGTTAA
CAGAGTGATCACCAGGGAGGAGTTCGAAGCCTTCGTCGCACAACAGCAATGACGGTAT
GAAGCGGGTGCGTACATCTTTTCCTCCGATACAGGCCAAGGACATTTGCAACAAAAAT
CCGTACGGCAGACCGTTCTATCTGAGGTCGTATTGGAACGCACGCTATTGGATGACGT
GTACGCCCCGCGTCTCGATCTTAACAAGGAGGAGTTACTGAGGAAGAAGCTCCAACTT
AACCCTACTCAAGCTAATAGGAGCAGATATCAGTCACGGAAAGTAGAGAATATGAAG
GCGATAACCACCATGAGAATGTTGCGCGGTCTAGGTCATTACTTGAAAACCGACAACA
AAGTAGAGTGCTACCGCACCTTATACCCCGTCCCGCTGTATTCCAGCAGTGTGAACCG
ATCGTTTTCATGCCCTAAAGTGGCCGTAGAAGCCTGCAACGTGGTGCTTAAAGAGAAT
TTTCCGACCGTGGCCTCGTATTGCATTACCCCCGAATACGACGCATACCTAGATATGGT
GGACGGCGCTACTTGCTGCCTAGATACCGCCAGTTTTTGCCCCGCTAAGCTGCGCAGTT
TTCCTAAGAAACACTCATACCTGGAACCGACCATTAGATCAGCAGTTCCGTCAGCGAT
TCAGAATACACTGCAGAACGTACTGGCCGCTGCTACAAAAAGAAACTGCAATGTTACA
CAAATGCGTGAACTGCCAGTGCTGGACTCCGCGGCCTTTAATGTAGAGTGTTTCAAGA
AGTATGCCTGCAACAACGAGTATTGGGACACATACAGAGATAATCCTATCAGATTAAC
TGAGGAAAATGTAACTAACTACATCACCAAGCTAAAAGGGCCGAAGGCAGCAGCACT
GTATGCCAAGACCCACAATCTCTGCATGCTGCAAGATATACCTATGGACAGATTCGTG
ATGGATTTGAAACGTGACGTCAAAGTGACGCCGGGTACTAAACATACTGAAGAACGGC
CCAAGGTTCAGGTGATCCAAGCCGCTGATCCACTAGCTACTGCGTACCTATGCGGAAT
ACATCGTGAGCTGGTTCGGCGGCTGAATGCAGTACTACTACCTAACGTCCATACACTTT
TTGATATGTCGGCAGAAGACTTCGATGCGATTATTGCCGAGCATTTCCAACACGGAGA
TCAAGTTCTAGAAACGGACATAGCGTCCTTCGATAAAAGCGAAGACGATGCGATGGCG
CTAACTGCACTTATGATACTTGAAGATCTAGGAGTCGACCAAGAGCTATTGACTCTAAT
TGAAGCTGCCTTTGGTGAGATATCTTCTATCCACCTCCCCACTAAAACGAAATTCAAAT
TTGGGGCGATGATGAAGTCAGGCATGTTTCTTACTCTATTCGTAAATACAATTATAAAC
ATAGTTATTGCGAGCAGAGTATTGCGAGAAAGGCTTACTAACTCTCCGTGCGCAGCTTT
CATCGGAGACGATAATATAGTGAAAGGAGTAAAATCTGATAAGCTAATGGCCGATAG
GTGCGCTACGTGGTTAAACATGGAAGTGAAGATCATAGATGCAGTTGTAGGGGAGAA
AGCACCGTATTTCTGCGGTGGTTTCATCCTGTGCGATACCGTGACCGGAACTGCATGTC
GCGTCGCGGATCCCCTAAAAAGGCTATTTAAGTTGGGGAAACCATTGGCAGCCGACGA
CGAGCATGACGACGACCGCAGACGAGCGCTGTTTGAGGAGGCAGAGAGGTGGAACCG
GGCTGGCATTTCCGTAGAACTTTGCAAGGCAGTAGAATCCAGGTACGAGATCGTGGGC
ACTTCTATTATAATAGCAGCTATGGCCACCTTAGCTCGTAACGTAGCGACATTCAAACA
CCTGCGAGGAAACCCCATACACCTCTACGGCTAA
SEQ ID NO: 25 - CABV subgenomic promoter
   CCTGAATGGACTGTGATATAGTACAGTCCGCAACC
SEQ ID NO: 26 - sequence of CABV 3' UTR 
ACTAACACAGCAATTGGCAGGCTGTAAATTGAGTACCATTAATCAGATAATAGCAGCA
ATTGGCGAGCTGCATAAAATTTTTAATAACAACTATTATAACTATCGTAGCAGCAATTG
GCTAGCTGCTTTTACCATTATTTTATTTTCTTTACCAACAATTGGATTTTGTTTTTAATAT
TTC
SEQ ID NO: 27- sequence of TONV 5' UTR
   ATGGGCGGCGTATGAGAGAAGCCCAAAACCTAGACTACCCATA
SEQ ID NO: 28 - sequence encoding TONV nonstructural protein nsP1-4 
ATGGAGAAGGTTCACGTTGACATCGAGGAGGACAGCCCCTTCCTCAGAGCACTACAAC
GGAGCTTTCCGCAGTTTGAGGTAGAAGCCAAGCAGGTCACGGATAATGACCATGCTAA
TGCTAGAGCGTTTTCGCATCTAGCTTCCAAACTGATCGAGACGGAGGTGGAACCATCC
GATACGATCCTAGACATTGGAAGTGCGCCCGCCCGCAGAATGTATTCAAATCACAAGT
ACCATTGTATCTGTCCGATGAAGTGTGCAGAAGACCCGGACAGATTATACAAATATGC
ATCTAAATTGAAGAAGAACTGTAAAGATATTACAGATAAAGAATTGGACAAGAAAAT
GGCAGAGCTTGCAGCGGTCATGGAGGACCCGGATCTAGAGCAGGAAACCATTTGTTTG
CATGATGATGAATCATGCCGCTATGAAGGCAACGTGGCGGTATATCAGGACGTCTACG
CTGTAGATGGTCCCACTAGTATATACCATCAGGCAAACAAAGGCGTTAGAGTCGCCTA
CTGGATAGGCTTTGATACTACGCCATTTATGTTCAAAAATTTAGCTGGAGCCTACCCTT
CCTACTCTACTAACTGGGCCGACGAGACCGTGCTGACAGCCCGTAACATCGGCTTATG
CAGCTCAGATGTCATGGAACGGTCTCGTCGGGGACTGTCCATTTTAAGGAAGAAGTAT
TTGAAACCATCAAACAATGTCATATTTTCCGTGGGTTCTACCATCTACCACGAGAAAAG
AGACTTATTAAAAAGCTGGCACCTACCTTCTGTATTTCACCTACGTGGCAAGCAAAATT
ACACATGTCGGTGTGAGACGATAGTGAGCTGCGACGGGTACGTCGTTAAGCGTATCGC
TATCAGTCCTGGCCTGTACGGGAAGCCGTCAGGCTATGCTGCCACTATGCACCGCGAG
GGATTCTTGTGCTGCAAGGTAACAGACACGTTGAACGGGGAGAGGGTCTCTTTCCCAG
TTTGTACTTATGTGCCAGCTACCCTATGTGACCAGATGACTGGCATTCTGGCAACAGAT
GTCAGCGCGGAAGACGCGCAAAAGCTGCTGGTTGGGCTCAACCAGCGCATTGTCGTGA
ATGGCCGCACTCAGAGGAATACTAATACCATGAAGAATTACTTACTACCAGTAGTTGC
ACAGGCATTTGCCCGGTGGGCTAAGGAGTACAAAGAGGACCAAGATGATGAAAGACC
GCTGGGATTGAGGGACAGACAACTTGTGATGGGTTGCTGCTGGGCGTTTAAGAAGCAC
AAGATTACATCCGTGTACAAACGCCCTGACACCCAGACAGTCATAAAGGTACCCAGTG
ACTTCCACTCCTTTGTGCTACCCAGATTGGGGAGCAGTTCTCTGGAGATGGGGCTAAGA
ACTAGAATAAAGAAGTTGCTGGAAGATAAGAAGACAACCCCACCTATAATTACTCCTG
AAGACATCTCATCGGCAAAAGATGCTGCCACGGAAGCTAAGGAAATCAAGGAGGCAG
AAGAGTTACGTATTGCACTGCCTCCTATGGTTCCAGACGTGGAGGAACCGACTCTGGA
AGCCGATGTAGATTTGATGCTGCAAGAAGCAGGGGCAGGGTCAGTAGAGACGCCCCG
AGGACTAATTAAAGTTACCAGCTATTCCGGTGAAGACAAGATCGGCTCCTATGCGGTA
CTTTCACCCCAGGCTGTCCTTCGGAGTGAGAAACTCTCCTGCATACACCCGCTGGCGGA
AGAGGTTATTGTTATAACCCACGCGGGAAGGAAAGGAAGGTATGCAGTTGAACCTTAT
CACGGCAAAGTGGTGGTCCCTGAAGGGCACGCTATTCCCGTAGCAGACTTCCAGGCAC
TCAGTGAGAGCGCCACCATTGTGTATAACGAGCGGGAGTTTGTGAACAGGTACCTACA
CCACATCGCCATCAACGGAGGAGCCTTGAATACCGATGAAGAGTACTACAAAGTGGTC
 
AAACCCAATGAACATGATGGAGAATACCTATATGACATTGATAAAAAACAGTGTGTTA
AGAAGGAACAGGTGTCCGGCCTCGGGCTGGCAGGTGAACTGGTGGAGCCTCCGTTCCA
CGAATTCGCGTATGAGAGCCTAAGGACTAGACCGGCAGCTCCTTATCAAGTACCAACT
ATAGGAGTGTACGGAGTACCTGGCTCGGGTAAGTCCGGGATTATAAAAAGTGCCGTGA
CAAAAAGAGATCTGGTGGTCAGTGCAAAGAAGGAAAATTGTACCGAGATCATACGCG
ACGTGAAGAAGATGCGCGATCTAGACATAGTGGCGCGTACTGTCGATTCGGTACTGCT
GAATGGCTGTAAACACCCAGTTGACACGCTGTACATTGATGAGGCCTTCGCATGTCAC
GCAGGAACGCTACGTGCGTTGATTGCCATCATTAAACCTAAAAAGGCAGTTCTCTGCG
GAGATCCGAAACAGTGTGGCTTTTTAACATGATGTGCCTTAAAGTGCATTTTAATCAT
GAAATTTGCACGCAGGTGTTCCACAAGAGCATCTCTCGTAGGTGCACTAAATCGGTTA
CGTCTGTTGTGTCCACGCTGTTTTATGACAAAAGAATGCGAACCACCAACCCAAAGGA
GACCAAAATCGAAATTGACACTACCGGAAGTACTAAGCCTAAGAAGGACGACTTGATC
TTGACGTGCTTCAGAGGATGGGTGAAGCAATTGCAAATTGATTACAAGGGCAATGAAA
TCATGACGGCTGCAGCCTCGCAAGGCTTGACCCGGAAAGGAGTCTACGCTGTCAGATA
TAAAGTCAATGAGAATCCCTTGTACGCACCTAGCTCAGAGCACGTCAATGTCCTGCTTA
CCCGCACAGAGGATAGGATTGTGTGGAAGACCTTGGCTGGAGATCCCTGGATTAAGAC
TCTCACTGCCAGATACGTTGGCGACTTCACCGCCACTCTAGAAGAGTGGCAACAAGAG
CATGATGCCATCATGAAGCACATCTTGGAGAAGCCTGACCCTACAGACGTGTTCAGA
ACAAAGCTAACGTATGCTGGCCAAGGCCTTAGTACCAGTGCTGAAGACCGCCGGTAT
TGATTTGACAACAGACCAATGGAACACTGTAGTTCATTTTAAGGAAGACAAAGCTCAT
TCGGCAGAGATCGTACTAAACCAGCTATGCGTACGATTCTTCGGACTGGATTTGGACTC
AGGTTTGTTCTCCGCACCCACCGTACCACTGTCAATAAAGAGCAACCATTGGGACAAT
TCGCCCTCACCGAACATGTATGGGTTGAACAAGGACGTGGTGCGCCAACTTTCAGTGC
GTTACCCTCAGCTTGCCCGAGCAACAGCGACAGGCAGAGTATTCGATATGAATACCGG
GACGCTGCGAAGCTATGACCCGCGTGTCAATCTAGTGCCCGTCAATCGCAAGCTGCCG
CACGCGCTGGTTACACACCGCTCGGAGCAGCCCCGTAGTGATTTTTCTACGTTTGTTAG
TAAGTTGAGGGGTCGAACAGTCCTGGTTGTGGGAGATAAGATGAATATCACTGGTAAG
ACAGTCGACTGGCTGTCTGAAAATCCTGAGGCCACCTTCAAATCCCGTCTAGACCTTGG
CATCCCAAATGGGCTACCCAAATATGACATCGTGTTTGTGAACGTTCGGACGCCCTAC
AAGTATCATCACTACCAGCAATGTGAAGACCATGCGATAAAGCTCAGCATGCTGACAA
AGAAAGCGTGCTTGCATCTCAACCCCGGAGGAACCTGCGTCAGCATCGGTTATGGCTA
TGCAGACAGGGCCAGTGAAAGCATCATCGGGGCTATCGCACGGCAGTTTAAGTTTTCT
AGAGTGTGTAAGCCGAAAGCTTCATTGGAGGAGACGGAAGTGCTGTTTGTTTTCATAG
GGTATGACCGGAGGGTTCGTACGCACAACCCATACAAACTATCCTCTACCCTGACCAA
TATCTACACCGGATCGCACCTGCATGAGGCCGGATGTGCCCCTTCCTACCACGTGGTAC
GTGGAGACATTGCCAACGCCGAGGAGGGGTTATTGTCAACGCAGCAAATAGCAGAG
GGCAGCCGGGTGGGGGTGTATGTGGGGCTCTCTACAAACGCTTCCCGGAGAGTTTCGA
CATGCAACCCATCGAGGTGGGTAAATCAAGGTTAGTAAAAGGGGCAGCGAAGCACAT
CATTCATGCTGTGGGGCCGAATTTTAACAAGGTATCAGAGCTGGAAGGGGACAAACAA
TTGGCCGAAGCTTATGAATCTGTCGCTAAGATTATTAATGACAATCATTACCGGACAGT
GGCAATACCGCTTTTGTCTACTGGCATTTTTGCCGGGAACAAGGACCGACTTATGCAGT
 
CTCTGAATCATTTGCTCACGGCGTTAGATACTACCGACGCGGACGTGGCTATATATTGT
AGAGACAAGAAGTGGGAGGCCACCCTGAAAGAAGTTATAGCAAGGAGAGAGGCTGTT
GAAGAGATATGTATCTCGGAAGATGCGTCTATTGCGGAACCGGACGCTGAATTGGTCA
GGGTACATCCCAAGAGCTCCCTGGCGGGGCGCAGAGGCTTTAGTACGACCGATGGGAA
GACATTCTCATACCTTGAGGGGACTAAATTTCATCAGGCGGCTAAGGATGTGGCTGAG
ATCAACGCGATGTGGCCTAATGCAACTGAGGCGAATGAACAGATCTGCCTGTACATCT
TGGGAGAGAGCATGAGCAGCATTCGTTCAAAGTGTCCAGTTGAAGAATCAGAAGCTTC
GACACCACCCAGTACATTGCCATGCCTATGCATCCATTCCATGACACCCGAACGAGTTC
ACAGACTTAAGGCCTCACGTCCTGAGCAGATAACGGTGTGCTCGTCTTTCTCGTTGCCC
AAGTACAGGATCACCGGAGTGCAAAAAATTCAATGCTCTCATCCTATACTCTTCCTCCC
GAAAGTGCCTGAGTACATTCATCCGCGCAGGTATCTTCCCCAAACGGAGAACCCTGTG
GATCCACCTGAACTTCCGGAGCCTTCAGCCGTCGCCACACCTACAGCAGACGGTCAGA
CAGAGGAGGCCATGAGCATAATGTCGGAAACGACTGTTGCCTCTCTTGTACCTAATGA
GATTGAGCCATCAACCGCCGCCACACCTGCAGCAGATAGTCAGACAGAGGAGACCAT
GAGCATAATGTCGGAAACGACTGTTGCCTCTTTTGTACCTAATGAGATTGTGGTCGAAG
CGCAAGTACACTCAGACCTTATGCGCTTCTCCTCTAGCTGGTCAATACCGCAAGCATCT
GACTTCGATGTAGACAGCGTGTCAGTCCAGGGCACCGTGGATATATTCAACAGTGCGC
TGCAGGAGGATGCGAGTAGTGTAGCATCGTCGTACTGCTCGGGAATAAAACCTGTCCC
TGCACCGCGCACTGTTTTACTCAATCACCAAAACCTCGCAGACGGCGCCGTCGATCTA
AAGCCAGCACGAGTTCTACGGCCCCTCTGGTGACCACATCCCGTGCTACATCACGATC
AAGCCTTGTCTCAAATCCACCTGGGGAAAGGTGACCACATCCCGTGCCACATCACGA
TCAAGCCTTGTCTCAAATCCACCTGGGGAAAGAGGGTTATTACTAGAGAGGAGTTCG
AGGCCTTTGTTGCGCAACAGCAATGACGGTACGAAGCGGGTGCGTACATTTTTCCTCT
GATACCGGTCAGGGGCATTTACAACAAAAGTCAGTGAGGCAGACGACGCTGTCCGAG
GTAGTACTTGAACGGACAGAGTTAGAGCATTCGTACGCCCCGCGCCTCGACCTAGAGA
AAGAAGAGATACTACGTAAAAAATTACAATTGAACCCGACACCAGCTAATAGGAGCA
GATATCAATCCAGAAGAGTGGAGAACATGAAAGCGGTGACTACCAAAAGACTCCTCG
GTGGTTTGGGGAGCTACCTGAAAACAGAAGGAAAGGTTGAATGCTACCGTACACTGTA
TCCTGTTCCCGAGTATTCTGCAAGCGTCAACCAAGTCTTTTCTAGTCCGCGCGTGGCTA
TTGAAGCATGTAATGCTGTATTGAAAGAGAATTTTCCAACAGTGACTTCATACTGCATA
ACACCCGAGTATGACGCCTACATGGACATGGTTGATGGAGCCTCCTGCTGTTTAGACA
CTGCTAGCTTTTGCCCAGCAAAGTTGCGCAGCTTTCCGAAACGACACGCCTATCTGGAG
CCAACTATTCGTTCTGCAGTACCATCAGCCATACAGAATACCTTGCAGAATGTGCTGGC
GGCGGCCACAAAAAGAAACTGTAACGTTACCCAGATGAGAGAGTTACCTGTTTTGGAT
TCCGCTGCTTTTAACGTGGAGTGTTTTAAAAAATATGCATGTAATAATGAATATTGGGA
AACGTACAGGGAACATCCTATTAGGCTAACCGAGGAGAATGTAACCAATTACATTACT
AAATTGAAGGGCCCGAAAGCAGCCGCTTTGTATGCAAAAACTCATAACCTTAGCATGT
TGCAGGATATTCCAATGGATAGGTTCGTTATGGACTTGAAAAGAGATGTCAAGGTGAC
GCCTGGTACTAAACATACGGAGGAGAGGCCGAAGGTGCAGGTCATCCAGGCGGCGGA
CCCATTGGCTACGGCTTACCTGTGCGGAATCCATCGAGAGCTAGTGCGCAGGCTGAAT
GCGGTCCTATTGCCTAACGTCCATACCTTGTTTGACATGTCTGCTGAAGACTTCGACGC
 
CATTATTGCCGAACATTTTCAGCCCGGAGACAGTGTGTTAGAGACTGACATTGCGTCGT
TTGATAAGAGCGAGGATGATGCAATGGCGTTGACTGCTCTGATGATATTGGAGGATTT
GGGCGTAGATCCAGAGCTGCTCACACTAATAGAGGCTGCATTTGGGGAGATATCATCC
ATCCATCTCCCCACGAAAACCAAGTTTAAATTTGGAGCAATGATGAAGTCTGGTATGTT
TCTTACTTTGTTTATTAACACGGTTATCAACATTGTCATAGCCAGCAGAGTTCTACGTG
AACGTCTGACCAACTCACCCTGTGCTGCATTTATTGGTGACGACAACATCGTCAAGGGT
ATCAAGTCAGATAAGCTGATGGCCGATAGATGTGCTACCTGGCTGAACATGGAAGTCA
AGATCATCGATGCCGTAGTAGGAACAAAAGCCCCTTATTTTTGTGGAGGCTTCATCTTG
TGTGACACAGTAACAGGTACAGCATGTCGGGTGGCTGACCCTCTGAAGAGATTGTTCA
AACTAGGGAAGCCGCTCGCAGCAGACGACGAACATGACGATGATCGTCGTCGGGCTTT
GCATGAGGAGTCAGCTCGCTGGAACCGTGTGGGCATACACTCCGAGCTGTGTAAGGCC
GTAGAGTCACGCTATGAAACTACAGGTACATCTGTAATCATAACAGCCATGACTACCC
TGGCACAGAGTGTCTCCGCATTCAAACATCTGAGAGGAAACCCTCTGACCCTCTACGG
CTAA
SEQ ID NO: 29-TONV subgenomic promoter
   CCTGAATGGACTGTGACGTAGTACAGTCCCCAAAATAGC
SEQ ID NO: 30- sequence of TONV 3' UTR 
ACCGCAGTAGCAATTGGCAAGCTATATAAAAGAATTATAAAGCGATAATTGGCAAATC
GCATGTAAATCCCAGCAGCAATTGGCACGCTGCATATATAATTTACTTGGCGGCAATT
GGCAAGCCGCCCATAACAAATTTTTATTTTCTTTTCCAATAATTGGATTTTGTTTTTAAT
ATTTC
SEQ ID NO: 31 - sequence of BEBV 5' UTR 
ATGGCGGCTGTGTGACACACGAGCCGTCGATTTCAACCTTCTTGCTCCCTCCAATTCTG
AGAGGAATCATCAAGCCAAG
SEQ ID NO: 32 - sequence encoding BEBV nonstructural protein nsP1-4 
ATGAACAAGGTGCACGTCGACATCGATGAGGAGAGTCCTTTCCTCAAATCGTTGCAGC
GTGCCTTCCCTTCACTTGAGATTGAAGCGAAGCAGGTCACTGCCAATGACCATGCTTGT
GCCAGAGCATTTTCGCATCTGGCTACCAAGTTAATTGAGCAAGAGGTTGACAAGGACA
CGCTCATCCTGGACATCGGCAGCGCGCCAGCCAGGAGAATGATGTCCGATCATAAATA
CCACTGCGTATGCCCAGTGCGCAGTGCTGAAGATCCAGAACGATTGGTGGCTTATGCT
AAGAAGCTGGCTGATGCCGCTGCTGTGGTGTTGGACCGTAAAATCTCTCAGAAAATCC
ATGATCTAAACACGGTGATGGCCACCCCCGACAGTGAGAGCGATACGTTCTGCCTACA
TACAGACGCAACGTGTCGAATGGGGGCAGAAGTGGCGGTCTACCAAGACGTGTACGC
AGTACATGCTCCAACCTCGCTGTATTTACAGGCGGTGAAAGGGGTGCGAACCGCGTAC
TGGATTGGGTTCGATACAACACCGTTTATGTTTCGGCTATGGCCGGAGCATACCCATC
CTATGCCACCAACTGGTCAGACGAGCAGGTCCTTCAAGCACGTAACATAGGTCTGTGT
GCAACAGATCTGACTGAAGGCAGAACGGGCAAGCTGTCGATCCTCAGGAAAAAGATG
CTGCGACCTAGCGACACCTTGATGTTCTCTGTGGGTTCTACACTGTATACAGAAAGCAG
AAAACTGCTGAAGAGTTGGCACCTACCTTCGACGTTTCATCTGAAAGGCAAACAGTCG
TTCACCTGTCGCTGTGACACGATAGTCTCCTGCGAGGGGTACGTCATGAAGAAGATCA
CCATGTGCCCCGGCCTGTATGGCAAACCCGTAGGATACGCTGTCACACACCATGCAGA
 
AGGTTTCCTGGTGTGTAAGGTCACGGACACCGTTAAAGGGGAACGTATGTCGTTCCCA
GTTTGCACATACGTGCCATCTAGCATCTGTGACCAGATGACCGGAATCATGGCTACTG
AGGTGACGCCTGACGATGCGCAGAAGCTGCTGGTCGGACTTAACCAGAGAATAGTTGT
CAACGGGAGAACCCAGAGGAACACAAACACTATGAAGAACTACCTCCTGCCGGTTGTC
GCTATGGCTTTCAGCAAGTGGGCCAAGGAGTACAAGGCCGATCTGGACGACGAGAAA
CCATTAGGAGTTAGGGAGCGCAGTCTTACGTACTGCTGCCTGTGGGCATTTAAAACGA
GAAAAACGCACACGATCTACAAGAAGCCTGACACGCAGTCAATTGTGAAAGTGCCGT
GTGAGTTCAACTCGTTCATTGTCCCTAGCCTATGGTCGTCTAACCTCTCGTTGCCAATTA
GAAGGAGAGTCAAGTTGTTTTAGCTAAGTCAGCTACTCGATCAATAGTTGAACGCAA
GCTTGACTCAGCTGATGCATTGGCGGCAGAGAGTGAAGAACTTGAAAGGATCGAAGC
GGAGAAGACCCGTGAGGCTCTGCCTCCTTTAATCGCGTCGACCACTGAAGACTGTCGT
GAAGTCGACGTCGAAGAACTTGAATATAGGGCTGGAGCAGGGGTTGTGGAGACACCC
CGTTGTGCTCTGAAGATTACAGCGCAACCAGGGGACGTGACTATCGGCTCCTACATTG
TGCTGTCCCCCCAGACAGTGTTGAAAAGCTGCAAACTGCAACCAGTGCATGCATTGGC
CGAGCAGGTTAAGATCATCACCCACTCAGGGAGATCTGGTAGGTACCAGGTGGAAGGT
TACGACGGGCGGGTTCTGCTACCATGCGGCGTTGCCGTCCCAACCCAAGAGTTCCAGG
CACTGAGCGAAAGTGCGACCATGGTCTATAACGAGAGAGAGTATGTGAACCGTAAATT
GTATCACATCGCTCTACATGGTCCTGCACTGAACACGGATGAAGAGAATTATGAAAAA
ATTCGCGCCGAACGAGCAGAGACCGAGTACGTGTTTGACGTCGACAAGAAGCAGTGTG
TGAAACGTGAGGAGGCCACAGGCATAGTACTTACTGGAGAACTTACCAACCCGCCGTT
TCACGAGTTCGCCTACGAGGGACTGAAAAGAAGACCGGCGGCTCCTTATAAGATTACT
ACTGTAGGCGTTTTCGGTGTACCAGGTTCCGGAAAATCCGCTATTATCAAAAATCTGGT
GACAGCGGACGACCTGGTCACTAGTGGAAAGAAGGAAAACTGCACGGAAATTACTAC
AGATGTCAAGCGTACTCGCGGATTGGACATCACCGCGAAAACTGTCGACTCCATCCTG
CTGAATGGGGTCCGTAAGAAAGTCAGAGTCCTGTTCGTTGATGAGGCGTTCGCCTGTC
ATGCCGGAACGTTGCTAGCGCTTATTGCGCTGGTTCGCCCAACTGAGAAAGTGGTACT
GTGCGGAGACCCGAAACAGTGTGGATTCTTTAACCTGATGCAGCTGAAAGTCAATTAT
AATCACAACATCTGTACGACGATCTGCCATAAGAGCATTTCTAGACGCTGTACACCAG
CGGTAACCGCGATCGTCTCAACACTACATTATGGGGGAAAGATGCGTACGACCAACAC
GCGCAATAGCCCTATAATCATAGACACTACTGGACAGACAAAACCCAAACGCGGAGA
TCTTGTGTTAACATGTTTCCGTGGATGGGTAAAGCAGTTGCAACTTGACTATCGTGGAC
ACGAGGTCATGACCGCGGCTGCTTCTCAAGGACTGACTAGAAAAGGGGTTTACGCGGT
TCGCCAGAAGGTTAACGAAAACCCGTTATATGCCCCATCGTCTGAGCACGTTAATGTG
CTGTTGACACGCACGGAAGACAGGCTAGTGTGGAAAACGCTAGCCGGAGATCCGTGG
ATTAAAGTCCTAACCAACATCCCAAAAGGTAACTTCTCAGCATCACTAGAGGAATGGC
AGGCAGAACATGACGACATCATGAGACAACTAGAGAGACCAGGAGACGATGTTGACG
AATTCCAGAACAAAGCAAACGTGTGCTGGGCTAAATGCCTCGTACCCGTGTTGCAAAC
TGCCGGTATCAGATTAACAGCTGAGGAGTGGTCCGCTACCATCAAAGCCTTCAATGAC
GACGCCGCGTACTCTCCTGAGGTCGCCCTCAATGAGATCTGCACCCGTATGTACGGCGT
GGATCTGGACAGCGGACTGTTCTCTTCGCCGACGGTGTCCCTCTACTATTCGGACAATC
ACTGGGACAATCGTCCTGGGGGAAAGATGTACGGGTTCAATAGAGAAGCGGCTGCAC
 
GGATAGAGCAGAGGCACCCCTTCTTGGCAGGCAAGTGGCAGAACGGCAAACAGCTGG
TTGTTGCAGAGAGAAAGGAACAACCTCTTGATGCTGCATGCAACATTATCCCATTAA
TAGGAGACTACCGCACGCTTTAGTCGCCGAGTACAAACCACTGCACGGTGAGCGCGTG
GAGTGGATGCTCCAGAAGATCAAGGGGTACCACGTGCTGTTGGTTAGCGAATACAACC
TGGTTCTTCCCTCGAAACGCGTGACTTGGATAGCTCCTCTGCACGTCCGAGGTGCCGAC
AGGGTATACGACCTTAATCTCGGTCTACCAACTGATGCCGGGCGTTTCGATACAGTGTT
CGTTAACATACACACCGAATACAGGTTACACCATTACCAACAGTGCGTCGACCACGCT
ATGAGATTGCAGATGCTGGGAGGTGATGCTCTGCGGCTCTTGAAGCCCGGAGGCACCC
TGCTAATTCGCGCCTACGGGTACGCAGACAAGGTCAGTGAGTCAGTCGTACTGTGCCT
CAGCCGGAAGTTTCAGAGCTTTAGGGTGCTACGACCTATGTGTGTCACCAGCAATACA
GAGGTTTTTCTTCTGTTCCAAAACTTTGACAACGGAAAGCGCGTGGTGACATTACATCA
GAACAACAGGAAGCTCACGGGGATATACAGCGGCGAGGCACTCCATACCGCAGGATG
TGCACCCTCGTACAGGGTTAAGCGTGCAGACATCGCTACTAGCGAGGAAGAGGCAGTT
GTAAACGCCGCCAACGCAAAAGGTAGACCGGGAGACGGAGTGTGCAGAGCCATCCAC
CGAAAGTGGCCTGAGGCCTTCGTGGGCGCTGCCACGGCGACCGGCACGGCAAAAACC
ATAAAGGTCGGACAGACATACATCATCCATGCCGTCGGACCGAACTTTTCCTCGACCC
AAGAACAGGAGGGTGATAAGTTGCTGGCCGGAGCGTACCGAGCGGTAGCCGAAGAAG
TAATTAAATACGGGTGCCGCAGCGTCGCCATTCCGCTGCTGTCTACGGGCATTTATGGC
GGTGGTAAAGACCGAATGTACCAGTCGTTGAACCATCTTTTTACCGCGTTGGATGCGA
CCGACGCAGATGTGGTGATCTATTGCAGGGACAAGACGTGGGAGACGAAGATCCAGG
AGGCGATAGATCGCAGACTGGCGGTGGAGCTAGTGTCCGATGAAATGGAACTGCAGA
CAGACTTAGTCCGCGTCCATCCCGATAGCAGCTTAGTGGACGGCGGGGTTACAGCAC
TACTGACGGTAAGCTGTATTCATACCTAGAGGGCACCAAGTTCCATCAATGTGCCGTC
GATATGGCAGAGATTCTGGTATTATGGCCGAACACCAGGGAGGCGAATGAGCAAATTG
CATTATATGCCCTAGGAGAAAGTATGGACACTATAAGATCTAGGTGCCCAGTAGATGA
CAATGATTCGTCTTCACCACCGCGGACGGTGCCATGTCTGTGTAGGTATGCTATGACAG
CCGAGAGGGTGACCAGATTACGCATGCACCATACTAAGTCGTTCACAGTCTGTTCTTCA
TTCCCCTTGCCTAAATACAACGTGGAAGGGGTTCAGAGGGTGAAATGCGAGAAGGTTC
TACTGTTTGATCCGACGGTTCCTTCGCTGGTGAGCCCTAGGAAGTACGTGTGTAACACC
ACTATACAGGCCGATGATCTCTCCTCGATCACGGAGTGTTCCTTATCGTCACGTAGACC
ATCTGTCTCAATATCTGTCTCCTCCATTTCTACAACAGATTTCATGCCGCGAAACACTTC
CGTGGACAACATACTACGGGTCATTGCCGAGATTCACCCTGTCCCAACCGAGGTACAG
ACGCTGCCTGTGCCCGAGCAAGGGGATGTCCCCTGCGGCACATTACCGGTGGAGCATC
AAGCACCCGTGCCCCCTCCTAGACCGAAGCGCGCCAGGGCATTGGCGGCTGCCAGGAT
ACCACCAGTTCCGGCGCCGAGACATTCAAAGGCACGTCCCGTTCCGGCCCCGAGGACC
ATTTTCAGAACCAGTAGGCCCGTTGTCCGAGCAGCCGTTGAACTACCATGGAAAATAC
AGGTTGTGCCTGGGCTGACTTTCGGCGATTTGCCGGAGCCCTCGAGCACACCAGCCGTT
GAGCTGCCATGGGAGCCGGAGGAGTCGTCAGGACTATCATTTGGCGACTTCGGGACGT
TCTGACTAGGACGAGCGGGTGCGTACATATTCTCATCGGACACAGGACCAGGCCACCT
GCAGCAGAAATCGGTTAGGCAACACAACCTGCCTACCCACACACTTGACGAAGTGCCG
ATGGAAAAAACGCACCCGCCGACACTGGACCACGCCAAGGAAAAACTCCTGCTAGCA
 
AGGATGCAGATGGCGCCAACGGACGCTAACCGCAGCCGGTACCAATCACGTAAGGTA
GAAAACATGAAGGCAACGGTTGTCCAGAGACTGCAGAGCGGCGCGAAGTTGTATACA
AGCTGCGACGCGGTGAGAGTGCCTACGTACGCTGTCAAGTACCCCAAACCGCTATATT
CCGCTCCGGTGGTTAGGGCACTCCGGCGCCCTGAGACTGCGGTTGCGGCTTGTAACGA
GTTCTTGACAAGGAACTACCCAACAGTGGCATCGTACCAGGTGACTGACGAGTACGAT
GCCTATCTGGATATGGTGGATGGATCGGAAAGCTGCTTGGACAGGGCTAACTTCTGCC
CTGCCAAACTGCGCAGCTACCCGAAACATCACGCATACCATCAGCCAGCCATCAGAAG
CGCCGTACCGTCACCCTTCCAGAACACACTGCAGAACGTACTATCGGCCGCTACTAAG
CGCAATTGCAACGTAACGCAGATGAGAGAACTGCCTACTTTAGACTCTGCAGTGTTTA
ACGTGGAGTGCTTTAAGGCATTTGCGTGTAACAATGAGTACTGGAAGGAGTTCTCTGA
GCATCCGATCCGAGTCACTACAGAAAACATCATGACTTATGTGACGCGCCTGAAGGGA
CCAAAAGCTGCTGCACTGTTTGCCAAGACTCACTCACTGGTCCCACTGCAGGAGGTTCC
TATGGACAAGTTCATAATGGACATGAAACGTGATGTAAAAGTCACGCCTGGGACCAAA
CATACCGAGGAGAGACCAAAAGTGCAAGTCATACAGGCTGCCGAGCCACTGGCCACT
GCTTACTTGTGTGGCATTCACCGGGAGCTGGTACGGAGACTGAATGCCGTCCTGTTGCC
AAATATCCATACACTGTTTGACATGTCAGCCGAGGACTTCGACGCGATTATTGCTGAGC
ATTTCCACCCTGGAGATCGCGTGTTGGAGACCGACATTGCCTCCTTTGATAAGAGTCAG
GACGACTCGTTGGCACTGACAGGACTAATGATACTGGAAGATCTTGGAGTTGACCAAC
CTTTGCTAGAGTTGATTGAGGCGGCCTTCGGAGAGATTACCAGCACGCACCTCCCTACT
GGTACTCGGTTCAAATTCGGTGCCATGATGAAATCCGGAATGTTCCTCACTCTGTTCGT
TAACACCATGCTCAACATCGTGATCGCTAGCAGGGTCCTCGAACGCAGACTGACTGAG
TCCGCCTGCGCAGCTTTCATCGGAGACGATAACATCATACATGGCGTTACTTCTGACCC
GCTCATGGCTGAGCGGTGTGCCTCCTGGATGAATATGGAAGTGAAGATCATCGACGCT
GAAATGTGCGTGCGCCCTCCTTACTTCTGCGGTGGCTTTATACTGTACGACAGCGTGAC
TCAGTCTGCCTGTAGAGTGGCAGACCCGCTAAAGAGACTGTTTAAGCTGGGTAAACCG
CTGCCTGCTGACGATGTGCAGGACGAGGACCGGAGGAGAGCGCTAGCGGACGAGGTC
AAACGGTGGAGCCGCGTCGGATTGGTTGCAGAGCTGGAAACGGCGGTGTGTTCGCGGT
ATGAGGTGCAAGGTTGTGACAACATTGTCTCGGCCATGGCCACCTTCTCGCAGAACAT
ACGCAACTTTAAGACACTGAGAGGTCCGGTAATACACCTATACGGTGGTCCTAAATAG
SEQ ID NO: 33 - BEBV subgenomic promoter
   ATGTGCAGTTACAGGAGTATACACATCGAATAACTATCCCGAGACC
SEQ ID NO: 34 - sequence of BEBV 3' UTR 
ATAACATATAATAAAGCCTAAATCTAATAGAATCATGTTAATCATTCTAAGATAAGCA
CTAGTTAAATATTAGAGGTATCCTAAGTGTAAGCAGAAAACGGAAAATCAAGAAAAA
TTAAGGTAAGAAATAGGATCTAGGAATTTATGTTAATCATTTTAGGTTAGCATTATAGT
AAGAATTAGGATCTAGGAATTTACATTAATCACTTTAGGTTAGTATCATGTTAAATATT
AGAGGTATCCTAAGTGTAAGCAGAAAACAGAAAAATTAAGAAAAATTAAGGTAAGAA
TTAGGATCTAAGAATTTATATTAATCACTTTAGGTTAGTACCATGATTTAGAAATTATA
GTGATCATTTTAAGCTAATTACTAGGTAAGTAACTGGTTAGTCTATCGGTAGCTTATGT
ATAAGTAGAAAAATGATAATAAAAGAAAAAATATAGAGTAGTATGTAGCTGTAAGTT
GAAAATATTGGAAAAACTATTACGAGCATCTACCACCGACGCCTCATCGGCTTATAGG
 
GCGTCATATAATTGAATTGATTATGCAATTGGAAAAACTTTAATCAGAAATATAATTG
GACAACATTGGTTTTTAATATTTCC
SEQ ID NO: 35 - sequence of EVEV 5' UTR
   ATGGGCGGCGCATGAGAGAAGCCCAAACACCTAACTGCCCAAA
SEQ ID NO: 36 - sequence encoding EVEV nonstructural protein nsP1-4 
ATGGAGAAAGTTCACGTTGACATCGAGGAAGATAGTCCTTTCCTCAGAGCTTTGCAAC
GGAGCTTCCCGCAGTTTGAGGTAGAAGCTAAGCAGGTCACTGATAATGACCATGCTAA
TGCCAGAGCGTTTTCGCATCTGGCTTCAAAGTTGATCGAAACGGAGGTGGACCCATCC
GACACGATCCTTGACATTGGAAGCGCGCCCGCCCGCAGAATGTATTCAAAGCATAAAT
ACCATTGCATCTGCCCGATGAGATGCGCAGAGGACCCGGACAGATTGTACAAGTACGC
TACTAAGCTGAAGAAAAACTGCAAGGAAATAACTGATAAAGAACTGGACAAGAAAAT
GAAGGAGCTCGCCGCCGTCATGAGCGACCCGGACCTGGAAACTGAGACCGTGTGCCTC
CACGACGATGAGTCGTGTCGCTATGAAGGGCAGGTCGCTGTATACCAGGATGTATACG
CGGTTGACGGACCGACAAGTCTTTATCATCAAGCCAATAAGGGGGTTAGAGTTGCCTA
CTGGATAGGTTTCGACACTACCCCTTTTATGTTTAAGAACTTGGCAGGAGCATATCCTT
CGTACTCTACCAATTGGGCCGACGAGACCGTGCTCACGGCTCGAAATATAGGCCTGTG
CAGCTCCGACGTTATGGAGCGGTCACGCAGAGGTATGTCCATTCTTAGGAAGAAATAC
TTAAAACCATCCAATAATGTCCTATTCTCTGTTGGCTCAACCATTTACCACGAGAAGAG
AGACTTATTGAAGAGTTGGCACCTACCGTCTGTATTTCACTTACGTGGCAAGCAAAATT
ACACATGTCGGTGCGAGACAATAGTTAGTTGCGACGGGTACGTCGTTAAAAGAATAGC
TATTAGTCCTGGCCTGTATGGGAAACCTTCAGGCTATGCTGCTACGATGCACCGCGAG
GGATTCTTGTGTTGCAAAGTGACAGACACATTGAACGGGGAGAGGGTCTCTTTTCCCG
TATGTACATACGTACCAGCTATATTGTGTGACCAAATGACTGGCATACTGGCAACAGA
TGTCAGTGCAGACGACGCTCAGAAATTGTTGGTTGGGCTCAACCAACGCATAGTCGTC
AACGGTCGCACTCAAAGGAATACCAACACAATGAAGAATTACCTTTTGCCCGTAGTGG
CCCAGGCGTTTGCCAGGTGGGCAAAGGAATATAAAGAAGATCAAGAAGATGAGAGGC
CGCTAGGTCTACGAGATAGACAGTTAGTCTTGGGATGCTGCTGGGCCTTTAAGAGGCA
CAAGATAACGTCTATTTATAAACGTCCCGACACCCAAACTATTATTAAAGTGAATAGT
GATTTCCATTCATTTGTGCTGCCCAGGATTGGCAGTAACACGTTGGAGATCGGGCTGAG
AAATAGAATCAGGAAGATGCTGGAAGAACATAAGGAGCCGTCACCTCTCATCACTGCC
GAGGATGTACACGAGGCTAAGTGCGCGGCCGATGAGGCTAAGGAGGCTCGCGAAGCA
GAGGAGTTGCGGGTAGCACTACCACCTTTGGCGGCCGACGTTGAGGAGCCCACTTTGG
AAGCCGATGTCGACCTGATGCTACAGGAGGCCGGAGCCGGCTCAGTGGAGACACCAC
GTGGCTTAATAAAAGTTACTAGCTACGCCGGAGAGGATAAAATCGGCTCCTATGCAGT
GCTTTCCCCGCAGGCCGTGCTTAAGAGTGAAAAATTATCGTGCATCCACCCCTTGGCTG
AACAAGTCATAGTAATAACACACTCTGGCCGAAAGGGGCGCTACGCGGTGGAACCGT
ATCACGGAAAAGTAGTCGTACCTGAGGGACATGCTATACCTGTCCAGGATTTTCAAGC
TCTGAGTGAAAGCGCCACCATTGTGTACAACGAACGAGAGTTCGTGAACAGGTACCTG
CATCACATTGCCACACATGGGGGGGCGTTGAATACAGATGAAGAATACTACAGAGTTA
TCAAGCCTAGCGAGCATGACGGCGAATATCTGTATGATATCGACAGAAAACAGTGTGT
CAGGAAAGAACTAGTCACTGGGCTAGGGCTAGCCGGCGAGCTGGTGGATCCGCCATTC
 
CATGAATTCGCCTACGAGAGCTTGAGGACACGACCAGCTGCTCCCTACCAAGTACCGA
CCATAGGGGTGTATGGCGTGCCAGGGTCAGGTAAATCTGGCATTATTAAAAGTGCAGT
TACTAAGAAAGACCTGGTGGTAAGCGCCAAGAAGGAAAACTGCACAGAAATAATAAG
GGACGTCAAGAAAATGAAAGGGTTAGACGTCAATGCCAGAACCGTGGACTCGGTGCT
CTTGAATGGATGCAAACACCCCGTTGAGACCTTGTACATCGATGAGGCATTTGCCTGCC
ATGCAGGCACCCTCAGAGCGCTTATAGCCATTATACGACCAAAAAAGGCTGTACTATG
TGGGGACCCGAAACAGTGCGGTTTCTTCAATATGATGTGTCTGAAAGTGCACTTTAATC
ACGAGATTTGCACGCAAGTTTTCATAAAAGCATCTCTCGCCGGTGCACTAAATCTGTG
ACCTCTGTCGTCTCAACTCTGTTTTACGACAGGAAGATGAGGACGACAAACCCGAAAG
AGACCAAGATCGAGATAGACACTACCGGCAGCACCAAACCAAAGCAGGGCGATCTTA
TCCTTACTTGCTTCAGAGGGTGGGTAAAGCAGTTGCAAATAGATTATAAAGGCAACGA
AATAATGACGGCAGCAGCCTCCCAAGGGTTGACACGCAAAGGCGTGTATGCCGTTCGG
TACAAGGTGAATGAAAATCCTTTGTACGCACCCACCTCAGAGCATGTGAATGTCCTGC
TAACCCGCACGGAGGACCGCATCGTGTGGAAAACGCTAGCTGGTGATCCCTGGATAAA
AACATTGACGGCTAAATATTCAGGAAATTTTACTGCCACGATGGAGGAATGGCAAACA
GAGCATGATGCCATTATGAGGCACATCTTGGAGAAACCGGATCCTACCGACGTCTTCC
AGAATAAGGCGAATGTGTGCTGGGCCAAGGCTCTAGTGCCTGTACTGAAGACTGCAGG
CATAGATATGACTACCGAACAATGGAGCACTGTGGACCACTTTGAAACGGATAAAGCC
CACTCAGCAGAAATAGTGTTGAACCAATTGTGCGTGAGATTCTTCGGACTCGACTTGG
ATTCCGGTCTTTTTTCTGCGCCCACAATTCCGTTATCCATCAGGAACAACCACTGGGAT
AACTCCCCGTCGCCTAATATGTACGGGTTGAATAAGGAAGTGGTTCGCCAACTTTCCCG
CAGGTACCCACAACTGCCTCGTGCGGTTGCTACTGGAAGAGTCTATGACATGAACACC
GGCACACTACGCAACTATGACCCGCGCATAAACTTAGTACCTGTGAATAGAAGATTGC
CTCATGCTCTAGTCCTCCACCACAATGAACATCTGCAGAGTGACTTTTCGTCATTCGTT
AGCAAACTGAAGGGCAGGACCGTCCTGGTGGTCGGGGAAAAATTGTCCGTTCCAGGCA
AATCGGTTGACTGGTTGTCAGACCGGCCCGAGGCTACCTTCAGAGCTCGCCTGGACTT
GGGCATCCCTGGTGAAGTACCCAAGTATGACATAGTATTCATTAATGTGAGGACCCCG
TATAAATATCATCATTATCAGCAGTGCGAAGATCATGCCATTAAACTTAGCATGCTGAC
CAAGAAAGCTTGCCTGCACCTGAATCCCGGAGGTACCTGTGTCAGCATAGGTTACGGC
TATGCAGACAGAGCCAGCGAGAGCATCATTGGTGCCATAGCGAGGCAGTTCAAGTTCT
CCCGGGTATGCAAACCGAAATCATCGCTTGAAGAGACAGAAGTGCTATTTGTATTCAT
TGGATACGATCGCAGGGCACGCACGCACAATCCCTACAAGCTCTCATCGACATTGACT
AACATTTACACAGGTTCCGGACTCCATGAAGCCGGGTGCGCACCCTCGTATCACGTGG
TGCGCGGGGACATTGCCACAGCCACCGAAGGAGTGATTGTGAATGCTGCCAACAGCAA
AGGACAACCCGGCGGCGGAGTGTGCGGAGCGCTGTACAAAAAATTTCCGGAGAGCTT
CGATTTACAGCCGATCGAAGTAGGAAAGGCGCGATTGGTTAAAGGCGCAACAAAACA
CATTATCCACGCTGTGGGGCCCAATTTCAACAAAGTCTCTGAGGTTGAAGGTGACAAA
CAGCTGGCGGAAGCTTATGAGTCCATTGCAAAAATTGTCAACGACAACAATTACAAAT
CGATTGCAATTCCGCTGTTGTCTACCGGCATATTTTCCGGGAACAGAGATCGATTGACC
CAGTCACTGAATCATTTGCTGACAGCCTTGGATACCACTGATGCAGATGTAGCCATATA
CTGCCGGGACAAGAAATGGGAAATGACCCTCAAGGAGGCAGTGGCTAGGAGAGAGGC
 
AGTGGAGGAGATATGCATATCTGATGATTCGTCAGTGACAGAACCGGATGCGGAGTTA
GTGAGGGTGCATCCGAAAAGCTCCCTAGCCGGAAGGAAGGGCTACAGCACCAGCGAC
GGAAAGACTTTTTCATACCTGGAAGGGACAAAATTTCATCAGGCGGCCAAAGACATAG
CTGAAATTAATGCCATGTGGCCCGTGGTAACGGAAGCCAATGAGCAGGTGTGCATGTA
CATTCTTGGAGAAAGCATGAGCAGTATCAGGTCAAAATGCCCCGTTGAAGAGTCGGAG
GCATCTACACCACCTAGCACACTGCCTTGCTTGTGTATCCATGCAATGACTCCAGAAAG
GGTGCAGCGCCTGAAAGCCTCGCGCCCTGAACAAATTACGGTGTGCTCGTCTTTCCCGC
TGCCGAAGTATAGAATTACTGGCGTGCAGAAGATCCAGTGCTCCCAGCCTATACTGTTT
TCACCGAAAGTGCCTGCATACATCCATCCGCGGAAGTATCTCGTGGTAGAGAACCAAT
CCGCGGAAAGGGAAATGGAACAGCCAGCAAACAGGACTGTGAGCGAGACAAGTGCTA
GAATGCCTGAGCCAGTCAATATCGAAGAAGAAGAGGAGGGCAGCATAAGTTCGCTGT
CAGATGGCCCTACCCGGCAGGTGCTGCAGGTTGAGGCGGACGTCCACAGGCCACCTTC
CGTGTTCGGCTCATCTTGGTCCATTCCCCATGCATCTGACTTTGACGTGGATAGTTTATC
CATCCTTACTTTGGAGGGGGCTAGCATGATTGAGGCGGCGCCCACGGAGACTGACTCT
TACTCCGCAAGAAGTATGGAGTTTATGGTGCGACCGGTGCCAGCGCCCAGGACAATAT
TTAGGAAACCCCCTCACCCTGCTCCACGCACAAGAATACAGTCGCATGCACTAAGCAG
GTTTAATTCGAGAACCAGCCTAGTCTCTAGCCCGCCAGATGTAAATAGGGTGACCACT
AGAGAGGAACTCGAGGCGTTACCGTCACGAGCACCAAGTAGGCCGGCCTCGAGGACC
AGCCTGGTTTCCAACCCACCAGGCGTGAATAGGGAGATTACTAGGGAAGAGTTCGAGG
CGTTCGTAGCACAACAGCAATGACGGTTCGACGCGGGTGCATACATATTTCCTCCGAT
ACCGGTCAAGGGCATTTACAGCAAAAATCAGTAAGGCAAACGGTGCTATCCGAAGTG
GTATTGGAGAGGACCGAATTGGAGATTTCGTATGCCCCGCGCCTCGACCTAGAGAAGG
AAGAATTACTACGCAAGAAATTACAGTTAAACCCCACACCCGCCAACAGAAGTAGATA
TCAGTCCAGGAAGGTGGAGAACATGAAAGCTATAACAGCTAGACGTATTCTGCAAGGC
CTGGGGCACTATTTGAAGGCAGAAGGAAAGTGGAATGCTACCGCACTCTGCATCCCG
TTCCCTTGTATTCGTCCAGTGTGAATCGTGCCTTTTCTAGCCCTAAGGTCGCAGTGGAG
GCCTGTAACGCCATGTTGAAAGAAAATTTCCGACCGTGGCCTCGTATTGCATAATTCC
GGAGTACGATGCCTATCTGGACATGGTTGATGGTGCTTCCTGTTGCCTGGACACAGCTA
GTTTTTGCCCTGCAAAGTTGCGTAGTTTTCCAAAGAAGCACTCTTACTTGGAACCCACA
ATACGGTCGGCAGTACCCTCGGCGATTCAGAACACGCTCCAGAACGTTCTGGCTGCTG
CCACTAAGAGGAACTGCAATGTCACGCAAATGAGAGAACTGCCAGTTTTGGATTCGGC
CGCTTTCAATGTGGAGTGCTTCAAGAAATACGCGTGCAACAACGAATATTGGAAGACA
TTTAAAGAGAACCCCATAAGGCTTACTGAAGAGAATGTGATAAATTATATTACTAAAT
TAAAAGGACCTAAAGCTGCTGCCCTTTATGCAAAGACACATAACTTGAACATGTTGCA
GGATATACCAATGGACAGGTTTGTTATGGATTTAAAGAGGGATGTGAAAGTGACTCCC
GGAACCAAACATACTGAAGAACGGCCGAAGGTGCAGGTAATTCAGGCGGCAGATCCC
CTAGCAACTGCGTACTTGTGCGGAATCCACCGCGAGCTGGTTAGAAGATTAAACGCGG
TCTTGCTTCCAAACATTCACACACTGTTTGACATGTCAGCTGAAGACTTCGACGCCATT
ATCGCAGAGCACTTCCAGCCTGGGGATTGTGTCTTGGAAACTGACATTGCGTCGTTTGA
TAAAAGCGAAGACGACGCCATGGCTCTGACCGCGATGATGATTTTGGAAGATCTAGGC
GTGGATGCAGAGCTGTTGACGCTGATTGAAGCAGCTTTTGGCGAAATTTCATCTATACA
 
CTTGCCCACCAAAACCAAATTTAAATTTGGAGCCATGATGAAGTCTGGGATGTTCCTTA
CACTGTTTGTGAACACCGTCATTAACATTGTCATTGCCAGTAGGGTGTTGAGAGAACG
GCTAACTGGATCACCCTGCGCAGCATTTATCGGAGACGACAACATCGTAAAAGGAGTC
AAATCGGACAAGTTAATGGCAGACAGGTGCGCCACATGGCTGAATATGGAAGTTAAG
ATTATAGATGCCGTAGTGGGAGAGAAAGCGCCCTATTTTTGTGGAGGGTTTATTTTGTG
TGACTCCGTAACCGGCACAGCGTGCCGTGTGGCAGACCCCCTAAAAAGGTTGTTTAAG
CTTGGAAAACCCCTGGCAGCAGACGATGAACATGATGATGATAGGAGGAGGGCATTG
CATGAAGAGTCAACACGCTGGAACCGAGTGGGAATTCTTCCAGAGTTGTGTAAAGCAG
TAGAATCAAGGTATGAGACCGTAGGAACTTCTGTTATAGTTATGGCTATGGCAACCCT
GGCTAGCAGTGTTAAATCATTCAGCTACCTGAGAGGGGCCTCTATAACTCTCTACGGCT
AA
SEQ ID NO: 37 - EVEV subgenomic promoter
   CCTGAATGGACTACGACTTAGTCTGGTCCGCCAAG
SEQ ID NO: 38 - sequence of EVEV 3' UTR 
ATATAGCAGCAATTGGCGAGCTGCTTAGATAGAACTTGCGGCGATTGGCATGCCGCTT
TAAAATTTTATTTTATTTTCTTTTCTTTTCCAAATTGGATTTTGTTTTTAATATTTC
SEQ ID NO: 39 - sequence of FMV 5' UTR
   ATAGGGTATGGTTTAGAGGCGCCTACCCTACTTAACCGATCCAAAC
SEQ ID NO: 40 - sequence encoding FMV nonstructural protein nsP1-4 
ATGGAGAAAGTGCATGTAGACTTAGACGTTGATAGCCCGTTCGTCAAGTCTTTGCAAA
AGAGCTTTCCACAGTTTGAGATCGAAGCAAAGCAGGTCACTGACAATGACCATGCTAA
TGCCAGAGCGTTCTCGCATCTGGCTACTAAGCTTATAGAAAGCGAAGTCGACCGGGAA
CAAGTGATATTGGACATTGGTAGCGCACCGGTGCGTCATGCGCATTCGAGTCACAATT
ACCACTGTGTTTGCCCGATGATTAGCGCTGAAGATCCAGATCGCTTACAGAGATACGC
AGAGCGGCTGCGCAGGAGCGACATCACTAACAAGCGCATTGCCTCTAAGGCAGCTGAT
TTGCTGCAAGTCTTGTCCGCGCCAGATTCAGAGACTCAATCGCTCTGCATGCACACCGA
TGCAACGTGCAGTTATCAAGGCACCGTGGCAGTTTACCAGGATGTTTACGCTGTGCAT
GCCCCTACCTCAATTTACTATCAAGCTATGAAAGGGGTGCGGACAATTTACTGGATTG
GATTTGATACAACCCCGTTTATGTATAAAAGTATGGCGGGTGCGTACCCATCATACAA
CACAAACTGGGCAGATGAGAGTGTGTTGGAGGCTAGAAATATAGGATTGGGGGATGC
GGATGTGCAGGAATCCAAGTTAAGAAGGGTACCGACATTCTGTAGGAAGAAGCTAAG
ACCTACCGACAAAGTAGTTTTTCAGTCGGTTCAACTATCTATACAGAAGACAGGTCCT
TACTTGAGAGTTGGCATCTACCTAATGTATTTCACCTAAAAGGAAAAAATAACTTTACA
GGGAGGTGTGGCACACTTGTGAGCTGCGAGGGGTATGTAATAAAGAAAATCACCATCA
GTCCGGGGTTGTATGGGCGAGTGGACAATCTTGCTTCAACCATGCACCGCGAAGGTTT
CCTGTGTTGTAAAGTGACAGACACGTTGCGTGGCGAGAGGGTCTCTTTCGCGGTCTGTA
CCTATGTTCCTGCCACTCTGTGCGATCAGATGACGGGTATATTGGCCACCGACGTCAGC
GTGGATGACGCTCAAAAACTGCTGGTTGGGCTCAACCAGCGGATTGTGGTCAATGGTA
GGACGCAGCGCAACGCCAACACTATGCAGAACTACCTGCTTCCAGTAGTTGCCCAGGC
GTTTTCTAGATGGGCGCGTGAATATCGTGCTGATTTGGAGGACGAGAGAATGTTAGGT
GAGCGGGATCGTTCACTTGCCATGGGGTGCTGCTGGACGTTTAGAACTCACAAAATAA
 
CATCTATCTATAAGAAACCAGGCACGCAGACTATCAAGAAGGTGCCGGCGGTCTTTGA
TTCCTTTGTACTACCACGGTTATCCAGTCATGGGTTGGATATTACCCTAAGAAACCGTA
TAAAACTGTATTTGGAACCAGCCATCACCCATGCGCCAGCTATAACACAGGCAGATGT
GCACCAGCTAGAGACCTTACAGGAAGAGGCTGAGGAGGTAGCAGCCGCGGAGGAGCT
GCGTGAAGCTCTGCCGCCGTTGTTACCTGAGCTGGACGGAGAGACGGTAGAGGCAGAG
ATCGACCTGATTATGCAGGAGGCTGGCGCAGGAAGCGTCGAGACGCCGCGCCGACAT
ATCCGAGTCACCAGTTACCCTGGCGAGGAAATGATCGGTTCCTATGCAGTACTCTCACC
GAAGGCAGTACTTAACAGCGATAAGCTATCTTGCATACACCCATTAGCTGAACAGGTT
CTTATAATGACTCATAAAGGGAGAGCAGGTCGCTATAAAGTGGAACCATATACAGGAA
AAGTCGTAGTTCCCGAAGGCGTCGCTGTCCCCATCCAGGACTTCCAGGCACTTAGTGA
AAGTGCCACGATCGTGTACAATGAACGCGAATTCGTGAACAGATACCTTCACCATATC
GCAACAAATGGTGGAGCTTTGAACACCGATGAAGAGTATTACAAGGTTATACGGTCCA
GCGAGGCAGAGTCCGACTATGTCTTCGATATAGATCAACGCAAGTGCGTCAGAAAAAA
TGATGCCGGCCCACTGTGCCTGGTAGGTGAGTTGGTAGATCCTCCATATCACGAATTCG
CGTATGAAAGCCTGAAAACCAGACCCGCCGCACCCCACAAGGTCCCTACTATCGGCAT
CTACGGGGTGCCGGGTTCAGGTAAATCCGGCATAATAAAAAGCGCCGTCACAAAGAA
AGATCTTGTAGTCAGCGCTAAGAAAGAAAACTGTGCTGAGATAACCCGGGACGTTAAG
CGGTTGCGGAAAATGGACATTACAGCACGCACTGTTGACTCTGTTCTGCTCAACGGCA
GCAAGCATGCTGTTCAGAACTTATTCATTGACGAGGCGTTCGCATGCCATGCGGGAAC
GCTATTGGCGTTAATAGCCATCGTCAAGCCGAAGAAGGTAGTTCTCTGCGGCGACCCG
AAACAGTGCGGCTTCTTTAACATGATGTGCCTAAAAGTCCACTTCAACCATGATATTTG
CACAGAAGTGCACCATAAAAGCATCTCTCGCAGATGCACACAGACTGTTACAGCTGTT
GTATCGACACTGTTCTATGAAAAACGCATGAGAACAGTCAACCCTTGCACCCAGCGCA
TTATCATTGACACAACCGGCTCAACCAAACCTGAGAAAGACGATTTGATATTGACATG
CTTCCGCGGGTGGGTTAAGCAGCTGCAAATTGATTACAAACACCATGAGGTTATGACA
GCTGCGGCATCGCAAGGATTAACCCGGAAAGGAGTCTACGCAGTCAGGTACAAAGTTA
ACGAGAATCCGCTATACGCACAAAACTCAGAGCACGTCAATGTATTGCTGACTCGAAC
CGAGAAGCGAATAGTTTGGAAGACTCTGGCAGGAGATCCATGGATAAAAACGCTGAC
GGCGAATTACCCAGGTGACTTTACCGCCACCTTGGATGAATGGCAACAAGAACACGAT
GCCATCATGGCTCGAGTGCTTGATGCCCCGGTATCTACAGACGTCTTCCAGAACAAGG
TGAATGTGTGCTGGGCCAGGGCACTTGAACCTGTTCTGGCCACTGCAAACATCACGCT
GACACGTGCCCAGTGGGATACCATACCGCCTTTTGCACTGGATAGAGCCTATTCGCCTG
AGATGGCCTTGAATTTCTTATGCACTCGGTTCTTTGGCGTTGACCTGGATAGTGGGTTA
TTTTCTGCCCGTACGGTACCACTCACTTATAAAAACCAGCACTGGGACAACAGCCCGG
GTATTAACATGTACGGTCTCAATATGACCGTTGCACGTGAGTTAGCTCGTAGGTACCCT
TGCATCTTGAAAGCAATTGAAACAGGGCGTATGGCGGATATACGGGTGGATGCTGTCA
AAGAGTATTCTCCTACGACCAATGTCGTGCCACTAAATCGCAGACTACCTCATGCATTG
GTGACTGAACACCGTAATTATGGTGTTGCTGATTACTCTGGCTTCCTTGCCAAACTGAA
AGGGAGCACGTTGTTGGTCATTGGCGATCCTATCCACGTACAAGGTAAACGCGTGGAA
TCTTTGGGTCCATCGCCGACTGCAACGTACAGAAGCAGTCTTGATCTGGGAATACCGC
AGGAAATTGGCAAGTACGACATTGTCTTCATTAATGTCAGAACAGAGTACAAGTACCA
 
CCATTACCAGCAATGCGAGGATCATGCTATTCACCATAGTATGTTAACATGTAAGGCTC
TAAACCATCTGAACAAAGGCGGCACATGCGTAGCCGTGGGCTACGGTATGGCTGACAG
AGCCACTGAGAATATCTTGACTGCTGTAGCCCGCTCGTTCAGGTTCACACGCGTCTGTC
AACCTAGAAACACCCAGGAGAACACGGAGGTGTTGTTCGTGTTCTTTCATAAGGATAA
TGGCAACCACTTGCAGGATCAGGATAGGTTGGGAATTATCCTGAATAATATCTACCAG
GGATCATCGCAACACGAAGCTGGTCGTGCGCCTGCATATAGAGTGGTCCGAGGGACA
TCACTAAAAGCAGTGACGAAGCAATCGTGAATGCCGCCAACAGTAAAGGACAACCAG
GGTCTGGAGTCTGCGGGGCTATATATAGAAAATGGCCGTCAGCGTTCGACCACAGACC
GGTAGCAGTGGGTACTGCCAGAATGGTTGATCATAGACCCTGCATTATCCATGCAGTA
GGGCCAAATTCTCCAGAGTATCCGAAACAGAAGGTGACATCAAGCTAGCCGAAGCAT
ATGCAAGTATTGCTCATATAGTCAATACTAAGCGCATTAATTCCATCTCTATTCCATTG
TTATCAACAGGGATATACTCAGGCGGAAAAGACCGCGTTGCTCAGTCCCTTGACCACC
TATTCGCAGCAATGGACACTACGGATGCAGATGTTACTATATACTGCCTGGATAAGAC
CTGGGAATCTAGAATCAAAGATGCCATAGACAAAAGGAACAGTGTTGAAGAAATTGC
CGAGGAAGACAAACCTGTAGATGTGGACCTGGTACGGGTCCACCCGCGGAGTTCACTC
GCAGGGAGACCCGGGTACTCAACATCAGAGGGCCGCATTCACTCCTATCTAGAAGGTA
CCCGTTTTCACCAAACAGCAAAAGACATTGCTGAAATCTATGCTATGTGGCCTGACAA
AGCTGAAGCCAATGAGCAGATCTGCCTTTATATCTTAGGTGAAAGCATGCCGAGTATC
AGGTCCAAATGTCCAGTTGAAGACTCTGAGGCATCGTCGCCTCCATATACCATCCCGTG
TCTGTGTGATTATGCTATGACTGCCGAAAGGGTATTTAGGCTGAGAGCTGCAAAGAAA
GAGCAATTTTCGGTGTGCTCCTCTTTTCACCTGCCGAAGTATAGGATCACAGGCGTGCA
AAAGATTCAGTGTAGTAAACCTGTTATATTTTCAGGTATTGCTCCACCCGCGGTGCACC
CCAGAAAGTACACTGCAATTATTAGTGAAAGAACCACACCAGAACCAACTGAAATTTT
TAGCATCGAAGAACCACCTAATGTAATACCGAGCCCTACACAGTTCCTGGACTACGGT
GCAGAGTCCCTATGCTTCGATAATGCAGTCACAACAACCGGTGATTCCGCACTGTCTCT
ATGCAGTTCTGATGGAGCATCCGAAACACCTGAAGATTTATCAGTAAGACGTACAGTA
TCCACTTGGAGCATTCCCAGTGCTACTGGGTTTGAAATTAAGGAGGAAGCTACCGAGG
ATGAGCACGTGTACATTGCTGAGTACGATCAGCGAGATTACAGCAATGTCACCGAGAT
CCTGCTTGAGTTTAGCAGGGCTCCAGTCCAATTCCTCTCGGATTTAAACCCATACCGG
CTCCAAGGAGCATCAGGCCCAAAATTTGTCCTGTGCCTGCTCCCAGGACAAAGGTTAC
CGGGCCGTCCTTTGGGGCCTCAACGCTTCAGTACAGCAAAGTGTACGAACGGCCGCCT
GGGGTAGCCAGAGCTATATCTGAGGCAGAATTGGATGCTTACATACAACAGCAACTGA
ATTGACGCTACGAAGCGGGAGCATACATATTCTCATCAGAGACTGGTCAGGGTCACTT
GCAACAGAAATCTAACAGGCAAGGCCCTTACGTGTACCCGGTCCTGGAAAAATCAGTA
CATGAGAAATTTTATGCCCCGCGCCTCGATATGGAGAAAGAGAAAATTCTCCAAAAGA
AATTACAACTCTGTGCTACAGAAGGCAATCGTAGCAGGTACCAATCACGAAAGGTTGA
AAACATGAAGGCCATCACAGTGACCAGGCTACTGTCTGGTATGGGGGAGTACTTATCG
GCTGACGCCGAACTCCCGGAATGCTACAAAGTGAATTATCCTGTACCAAGCTACTCCG
CCCAACTAGAGTGTGCGTTTAAGTCACCGATTGTAGCTGTAAAGGTATGCAATCTGATC
TTGCAGGAAAATTATCCGACAGTGGCCAGCTATGGAATCACAGACGAATATGATGCCT
ATTTGGATATGGTAGACGGCGCCTCGTGTTGCTTAGATACGGCTTCATTCTGCCCGGCG
 
AAACTTAGAAGTTATCCAAAGAAACACAGCTACTTATACCCTGAGATTAGGTCAGCAG
TCCCCTCACCCATTCAGAACACGTTGCAGAACGTGTTGGCAGCGGCTACTAAGCGCAA
CTGTAATGTCACACAAATGCGTGAATTGCCAGTCTTAGATTCAGCTGCCTTCAATGTGG
AATGCTTCAAAAAATATGCCTGCAATACTGAATACTGGGACGACTTTAAGGAACATCC
TATACGTCTGACCACCGAGAACGTCACTCAGTATGTTACCAAACTTAAGGGGCCTAAA
GCTGCTGCCCTCTTCGCTAAGACTCACAATCTGGTGCCATTACATGAAATACCTATGGA
TAGATTTGTGATGGACTTAAAGAGGGACGTTAAAGTTACACCGGGGACTAAGCATACT
GAAGAGAGACCCAAAGTGCAGGTTATCCAAGCGGCAGATCCCCTTGCCACAGCGTACC
TCTGTGGCATCCATAGAGAGCTGGTACGGAGGCTGAATGCGGTGCTGCTACCGAATGT
CCACACCTTATTCGACATGTCAGCAGAGGACTTCGATGCCATCATTGCCGAACATTTCC
ACTACGGAGACCCTGTATTGGAAACTGATATTGCCTCGTTCGACAAAAGTGAAGATGA
TGCCATTGCTACCTCTGCACTGATGATCTTAGAAGATCTAGGTGTGGATCAACCTCTGT
TGGATCTTATTGAGGCGGCGTTTGGTAATATTACGTCAGTCCACTTGCCGACGGGCACC
AGATTCAAATTTGGGGCAATGATGAAATCTGGAATGTTTCTGACGCTGTTTGTCAATAC
ATTGGTTAACATCATGATAGCTAGCAGGGTGCTCCGCGAGAGGTTGACTAACTCCGCT
TGTGCCGCTTTCATCGGGGATGATAACATAATTCACGGAGTAGTCTCCGATGAACTGAT
GGCAGAACGGTGCGCCACCTGGCTGAACATGGAAGTGAAAATCATCGACGCCGTCATA
GGCGTAAAGGCACCGTACTTCTGTGGAGGGTTCATACTGGTGGATCAAATTACCGGAA
CTGCCTGCCGAGTGGCTGATCCCTTAAAACGCCTGTTTAAGCTGGGCAAGCCGTTACCA
ACTGATGATGCCCAAGATTGCGACAGGCGCCGTGCTTTGCATGATGAGGCAAAGCGCT
GGAACAGGATTGGGATCACTGACGAAATAGTGAAGGCTGTAGAGTCGAGGTACGAGA
TTAGTCTGGCTAGGTTGATCATTATGGCACTAACTACGTTGGCCGCCAGCGTGGACAAT
TTTAAAAACATAAGAGGACAACCAATAATTTTGTACGGGTAA
SEQ ID NO: 41 - FMV subgenomic promoter
   CCTAAATAGGTGACGCAACATAGTATACTGTGTTACGTTGCCTGCTCTT
SEQ ID NO: 42 - sequence of FMV 3' UTR 
TAGCCTAACCTAGCTTATAAACTATTATACTTATGCTTGCTTGTAGTTTAGATTAGTATT
TACTTGTAGTAATTGTAATTAGTATTATATTATTAACTTAGTTTCAATTTATTTTTCTTA
CATTTAACTTTAAACCTTTTATTCTTTATCCTTATTTTATTTAGTCTACTAGATTAGTTTT
GTTTTTAATATTTC
SEQ ID NO: 43 - sequence of GETV 5' UTR 
ATGGCGGACGTGTGACATCACCGTTCGCTCTTTCTAGGATCCTTTGCTACTCCACATAG
TGAGAGACAAACAACCCAA
SEQ ID NO: 44 - sequence encoding GETV nonstructural protein nsP1-4 
ATGAAGGTAACCGTGGACGTTGAGGCTGATAGCCCATTCCTTAAGGCCCTTCAGAAGG
CGTTTCCCGCCTTTGAGGTTGAATCACAGCAGGTCACACCGAATGACCATGCTAACGCT
AGAGCATTTTCGCATCTGGCTACTAAACTGATTGAGCAAGAGGTTCCAACAGGCGTCA
CCATCCTGGACGTGGGTAGTGCACCCGCAAGGAGGTTGATGTCTGACCACACCTACCA
CTGCATCTGCCCCATGAAAAGTGCGGAAGACCCAGAGAGGCTGGCGAATTACGCTCGA
AAGCTGGCGAAAGCATCGGGGACTGTGCTAGACAAGAATGTGTCCGGAAAGATAACG
GACCTACAAGACGTCATGGCCACTCCAGACTTGGAATCCCCGACTTTTTGCCTGCATAC
 
TGACGAGACGTGCCGCACTAGGGCTGAGGTCGCCGTGTACCAGGACGTATACGCTGTG
CACGCACCGACGTCACTGTACCACCAGGCCATCAAAGGTGTCAGGACGGCGTATTGGA
TTGGATTCGACACCACTCCATTCATGTTCGAGGCACTAGCGGGCGCGTACCCTGCGTAC
TCGACCAACTGGGCAGATGAGCAAGTGCTGCAGGCTCGTAACATCGGCCTGTGCGCGA
CAGGCCTCTCCGAGGGGCGTCGCGGCAAACTCTCCATCATGAGAAAGAAGTGCTTGCG
ACCGAGCGATAGAGTAATGTTTTCGGTCGGGTCCACCTTGTACACCGAGAGCCGAAAG
CTGCTGCGCAGCTGGCATTTACCTTCCGTGTTTCACCTGAAGGGTAAGAACAGTTTTAC
CTGCAGGTGCGACACGGTGGTGTCATGCGAAGGTTACGTGGTAAAGAAGATCACCATA
AGCCCGGGCATATATGGAAAAACAGTCGATTACGCAGTTACCCATCACGCAGAGGGTT
TCCTGATGTGTAAGATCACTGATACAGTCAGAGGAGAAAGAGTCTCTTTCCCGGTCTGT
ACCTATGTGCCTGCAACCATATGCGACCAGATGACGGGTATACTTGCCACTGACGTGA
CACCAGAGGATGCCCAGAAGCTCCTGGTTGGATTGAACCAACGCATAGTGGTGAACGG
TAGGACGCAAAGAAACACAAACACAATGAAAAACTACCTACTGCCAGTGGTAGCGCA
AGCATTCAGTAAATGGGCACGAGAGGCACGCGCAGACATGGAGGACGAAAAACCCCT
AGGCACCAGAGAACGCACGTTGACGTGTTGTTGCCTGTGGGCGTTTAAAAGCCACAAA
ATCCACACCATGTATAAGCGGCCTGAAACACAAACTATCGTCAAAGTGCCTTCCACTTT
TGACTCCTTTGTGATACCGAGCCTGTGGTCATCCAGTCTTTCCATGGGTATCAGACAGA
GGATCAAATTGCTACTCAGCGCAAGAATGGCCCAAGGCCTACCATACTCAGGAGACCG
CACTGAAGCTCGCGCGGCAGAAGAAGAGAAGGAGGTGCAGGAGGCTGAACTTAC
GAGGGCTGCGCTGCCACCGCTAGTGAGCGGCTCTTGTGCTGACGATATCGCCCAGGTG
GATGTAGAGGAACTAACCTTCAGAGCCGGAGCCGGGGTTGTGGAAACACCCAGGAAT
GCCCTGAAGGTTACACCGCAAGCACACGACCATCTCATAGGCTCCTACTTGATCCTTTC
CCCCCAAACGGTGTTGAAAAGCGAGAAGCTGGCACCCATCCACCCTCTTGCTGAGCAA
GTCACGGTCATGACCCACTCTGGAAGATCCGGCAGATACCCAGTCGACAAGTACGACG
GACGGGTATTGATCCCAACAGGAGCAGCCATCCCAGTGAGTGAGTTCCAGGCACTCAG
CGAGAGCGCAACCATGGTGTACAATGAGAGGGAATTTATAAATCGCAAGCTACACCAC
ATAGCGCTATACGGGCCAGCCTTGAATACCGACGAGGAAAGCTACGAAAAAGTGAGA
GCTGAAAGGGCAGAGACAGAGTATGTGTTCGACGTGGACAAGAAGGCATGTATCAAG
AAGGAGGAGGCATCAGGCCTTGTGTTAACAGGGGACCTAATCAATCCACCTTTCCACG
AATTCGCATACGAAGGACTCAAGATCCGCCCAGCAGCCCCGTACCACACGACGATCAT
TGGTGTGTTTGGCGTTCCGGGTTCGGGCAAGTCGGCTATCATTAAGAACATGGTGACG
ACTCGCGATCTGGTGGCCAGTGGAAAGAAGGAGAACTGCCAAGAGATCATGAATGAT
GTAAAGAGGCAACGCGGGTTGGACGTAACCGCTAGGACCGTCGACTCAATCTTACTGA
ATGGGTGCAAGAAAGGCGTAGAAAACCTTTACGTCGATGAGGCGTTCGCGTGTCACTC
GGGTACTTTGCTAGCGCTCATCGCGCTGGTGAGACCGTCAGGTAAGGTAGTACTGTGC
GGCGACCCTAAGCAGTGTGGTTTCTTCAATTTGATGCAACTGAAGGTGCACTATAACC
ACAACATTTGTACAAGGGTGCTCCATAAGAGCATCTCCAGAAGATGCACTCTACCTGT
TACGGCGATCGTGTCCACCTTGCACTACCAAGGGAAGATGAGAACGACGAACCGATGC
AACACCCCCATTCAGATTGACACCACCGGTTCCTCCAAACCAGCCTCAGGAGATATCG
TGTTAACGTGCTTCCGCGGCTGGGTGAAGCAACTGCAAATCGACTATCGTGGACACGA
GGTGATGACCGCAGCTGCTTCCCAGGGTCTGACAAGGAAAGGCGTGTACGCCGTGAGA
 
CAGAAAGTGAACGAAAACCCACTGTACTCACCTCTGTCGGAGCACGTCAACGTGCTGT
TGACCCGAACTGAAAACCGACTGGTGTGGAAGACACTGTCGGGTGACCCGTGGATAAA
GGTGTTAACCAATGTTCCACGTGGGGATTTCAGTGCAACTCTGGAGGAATGGCATGAA
GAACATGACGGTATCATGAGAGTGTTGAACGAGCGACCGGCGGAGGTTGATCCATTCC
AAAACAAGGCTAAGGTGTGCTGGGCAAAATGTCTGGTGCAAGTTCTTGAGACGGCCGG
AATACGTATGACGGCAGATGAATGGAACACCATCTTGGCTTTCAGAGAGGACAGAGCG
TACTCACCAGAAGTCGCTCTCAACGAGATTTGCACTCGTTACTACGGCGTTGACCTAGA
CAGCGGCCTATTCTCAGCGCAGTCGGTTTCCCTCTTTTATGAGAACAACCACTGGGACA
ACAGGCCTGGAGGACGCATGTACGGGTTCAACCATGAAGTAGCCAGGAAATACGCAG
CCAGGTTTCCATTTCTACGTGGCAACATGAACTCGGGGCTACAACTAAACGTCCCTGA
GAGGAAGCTCCAGCCCTTTAGCGCTGAATGCAACATAGTCCCATCCAATCGTCGGTTA
CCGCATGCTCTGGTCACAAGTTATCAGCAGTGTCGTGGGAGAGGGTAGAGTGGTTGC
TGAAAAAGATTCCAGGTCACCAAATGTTACTTGTAAGTGAGTACAACCTGGTGATACC
TCACAAAAGAGTCTTCTGGATTGCACCTCCGCGGGTGTCAGGCGCGGACCGCACGTAC
GACTTGGACCTAGGGTTACCTATGGATGCAGGCCGTTACGACCTGGTATTCGTCAACAT
CCATACTGAGTACCGGCAACACCACTACCAACAATGCGTCGACCATTCAATGCGCCTG
CAGATGCTGGGAGGGGATTCACTACACCTGCTTAGACCAGGAGGCTCGCTGCTGATGA
GAGCATATGGTTACGCAGACAGAGTCAGCGAGATGGTGGTGACAGCCCTGGCTAGGA
AATTCTCGGCGTTCCGTGTCCTGAGACCGGCGTGTGTGACGAGCAACACAGAAGTGTT
CCTGCTGTTTTCTAACTTTGATAACGGCAGAAGGGCGGTAACCTTGCACCAAGCTAACC
AGAAACTTAGCTCAATGTATGCCTGCAACGGATTGCACACTGCTGGTTGTGCACCGTC
ATACAGGGTCCGCCGCGCAGATATATCAGGACACAGTGAGGAAGCGGTCGTAAATGCT
GCCAATGCCAAAGGTACCGTGAGCGACGGAGTGTGCAGGGCGGTCGCTAAGAAGTGG
CCATCATCTTTCAAAGGGGCTGCAACTCCAGTCGGCACAGCCAAAATGATCCGCGCAG
ATGGCATGACCGTAATCCACGCAGTGGGACCAAACTTCTCCACCGTAACAGAAGCCGA
AGGGGACAGAGAGCTAGCGGCCGCGTATCGAGCTGTGGCTAGCATAATTAGTACCAAC
AACATAAAGAGCGTCGCAGTACCGCTGCTGTCCACAGGCACCTTTTCCGGCGGTAAGG
ACAGAGTGATGCAGTCCTTGAACCACTTATTCACGGCACTGGACGCAACCGATGCAGA
CGTGGTTATCTACTGCAGAGATAAAAACTGGGAAAAGAAGATTCAGGAAGCCATCGA
CAGGCGGACGGCAATCGAGCTCGTATCTGAAGACGTGACCTTGGAAACCGATCTGGTT
AGGGTACACCCGGACAGTTGCTTAGTCGGCAGAAATGGTTACAGTGCAACTGACGGTA
AACTGTACTCCTACCTTGAGGGCACGAGGTTCCACCAGACGGCGGTCGACATGGCTGA
AATATCAACTTTATGGCCAAGACTCCAAGATGCTAACGAGCAGATCTGCCTGTACGCC
CTAGGGGAGACGATGGACAGCATACGCACTAAATGCCCAGTAGAGGACGCCGATTCG
TCTACGCCGCCGAAAACGGTACCGTGTCTATGTCGGTACGCGATGACCGCGGAGCGGG
TTGCCAGACTTAGGATGAATAACACCAAAAACATCATCGTGTGCTCCTCCTTTCCATTA
CCGAAGTACAGGATAGAAGGCGTGCAGAAGGTGAAGTGTGACCGAGTGCTAATTTTTG
ACCAGACCGTCCCGTCACTAGTAAGTCCCAGAAAGTACATACAGCAGCCGCCGGAACA
GCTGGATAATGTGAGCCTGACTTCTACGACGTCGACGGGATCCGCATGGTCATTTCCGT
CGGAAACGACCTACGAAACCATGGAAGTCGTAGCCGAGGTACACACCGAACCTCCAA
TCCCTCCGCCTCGCCGACGTAGAGCAGCCGTCGCCCAACTTAGACAGGATCTGGAAGT
 
CACCGAGGAGATCGAGCCGTACGTGACACAGCAAGCAGAGATCATGGTCATGGAGAG
GGTCGCGACGACAGACATACGCGCTATCCCAGTCCCGGCACGGCGGGCCATTACAATG
CCAGTCCCAGCCCCCAGGGTTCGTAAGGTCGCTACTGAACCTCCATTAGAACCGGAAG
CTCCTATCCCGGCACCAAGAAAGAGAAGAACCACTAGCACCTCACCTCCGCATAACCC
CGAGGATTTCGTTCCCAGGGTACCTGTTGAGTTACCGTGGGAGCCGGAAGACCTAGAC
ATCCAATTCGGTGACTTGGAGCCACGCCGCCGGAACACCAGGGACCGAGATGTCAGCA
CAGGAATACAGTTCGGTGACATCGACTTTAACCAGTCCTGACTAGGCAGGGCTGGCGC
GTATATCTTTTCGTCTGACACTGGCCCGGGTCACCTACAACAGAAGTCCGTAAGGCAA
CATGAATTGCCATGCGAGACTCTGTACGCCCATGAAGACGAACGCATATACCCGCCGG
CATTTGACGGAGAGAAAGAAAAGGTACTCCAGGCAAAGATGCAGATGGCCCCGACAG
AAGCGAATAAGAGCAGGTACCAGTCGAGGAAAGTAGAGAACATGAAGGCATTAATTG
TAGAAAGACTACGCGAAGGAGCAAAGTTGTACCTCCATGAGCAAACCGACAAAGTAC
CCACGTACACCAGCAAGTACCCTAGACCTGTGTACTCACCATCGGTGGATGACAGCCT
GAGCGATCCGGAAGTGGCTGTGGCCGCCTGTAACTCTTTCTTAGAGGAGAATTATCCG
ACCGTGGCGAACTACCAGATAACCGATGAGTATGACGCTTATCTGGACTTGGTCGACG
GCTCTGAAAGCTGCCTCGACAGAGCTACGTTCTGCCCGGCCAAACTAAGATGTTACCC
TAAGCACCACGCATACCACCAACCACAAATCAGGAGCGCAGTACCTTCCCCTTTTCAA
AACACGTTACAAAACGTGCTAGCCGCGGCCACTAAAAGAAATTGCAACGTCACCCAAA
TGAGAGAATTACCAACCATGGACTCTGCGGTGTTCAACGTAGAAAGCTTCAAAAAATA
CGCATGTACCGGCGAATATTGGCAAGAATTTAAAGACAATCCTATACGGATCACCACC
GAAAACATAACGACGTACGTGGCTAAACTCAAGGGTCCAAAGGCTGCTGCCCTTTTTG
CCAAGACGCATAACCTGGTGCCGCTTCAGGAGGTGCCAATGGACCGCTTCGTGATGGA
TATGAAGAGAGATGTGAAAGTTACACCAGGCACCAAGCATACCGAAGAAAGGCCAAA
AGTGCAAGTAATTCAAGCGGCGGAACCACTGGCCACGGCATATTTATGCGGAATCCAC
AGAGAGTTAGTCAGGCGGCTAAAAGCCGTTCTGACCCCGAACATTCATACTCTGTTTG
ACATGTCGGCGGAGGACTTTGATGCCATCATAGCGGCACATTTCCAACCGGGAGATGC
TGTACTGGAGACAGATATCGCATCCTTCGACAAGAGCCAAGACGACTCCTTAGCGCTA
ACGGCGCTGATGCTTCTGGAAGACCTCGGGGTCGACCAAGAACTGCTGGACCTTATCG
AAGCTGCGTTTGGTGAGATCACGAGTGTGCATCTACCTACCGGTACAAGATTTAAATTC
GGTGCTATGATGAAGTCAGGAATGTTTCTTACACTCTTCATCAACACGCTGCTGAACAT
TGTCATAGCGTGCCGCGTCTTACGCGACAAATTATCGTCCTCGGCGTGCGCCGCCTTCA
TAGGTGATGACAACATAGTGCACGGCGTGAGGTCAGACCCGCTAATGGCAGAAAGGT
GCGCGAGTTGGGTCAACATGGAAGTAAAGATCATCGATGCCACAATGTGTGAGAAACC
ACCGTACTTTTGTGGAGGATTCATCCTGTACGACAGTGTCGCCGGTACAGCGTGTAGG
GTTGCAGACCCGTTAAAGAGGCTGTTCAAACTCGGGAAACCGCTCCCGGCGGACGACA
ACCAGGATGAAGACAGAAGAAGGGCACTAAAAGATGAAACAGTTAAGTGGTCCCGCA
TAGGATTGAGAGAAGAATTAGACGTGGCATTGAGCTCAAGATACCAAGTCAGCGGCGT
CGGAAACATCACTAGAGCGATGTCCACGCTGTCTAAGAGTTTGAAGTCTTTTAGGAAA
ATAAGAGGTCCCATCATACATCTGTACGGCGGTCCTAAATAG
SEQ ID NO: 45 - GETV subgenomic promoter
   ATGCAGGATTACACTACATCTAAAGACCACGTATTACAGACACC
SEQ ID NO: 46 - sequence of GETV 3' UTR 
CCGGGAGGCTTGACATAATGTATATATATAAGCATCATAGTTTTAATAAAGCCCCTGA
ATAGTAACAAAACATAAAAACCAAAAACAGTAGTTCAAAGGGCTATACAACCCCTGA
ATAGTAACAAAATACAGAAAAACCATAAAAATTATAAAATTAACTAATCAGATCATCT
AAATTTGACTAATTGGAAATAGCCGAACTCTACGGAGATGTAGGCGTCCGAACTCCAC
GGAGACGTAGGACAAAATTCTGCCGAACCCCAGACCATCGGGGACGTAGGCGTCTAAT
TTGTTTTTTAATATTTTAC
SEQ ID NO: 47 - sequence of NDUV 5' UTR
   ATGGTGCGGAGTTGAGAGACGAAGCACCAAACAACTACGCGGCTCACC
SEQ ID NO: 48 - sequence encoding NDUV nonstructural protein nsP1-4 
ATGGCTAAACCAACTGTACACGTGGACATTGAGGCGGATAGCCCGTTTGTCAAAACGT
TGCAGAAAAGTTTCCCCCAGTTTGAAGTCGTTGCAGAGCAGGTCACACCGAATGACCA
TGCTAATGCAAGAGCGTTTTCGCATCTTGCAAGTAAGCTCATAGAGTTGGAGGTGGAT
AAGAAAGCCACCATCCTTGACATAGGCAGCGCACCGGCGCGCCGTATGTACTCTGAGC
ATACTTACCACTGCGTGTGCCCGATGAAGACTGCGGAAGATCCCGACAGGATCATGGG
TTACGCGCGCAAACTGAAGGAGAAGTGTCTTGAAATAACAGACAGGAATCTAGCCGCT
AAATTGAAAGATCTGAAAGACGTGATGGCACGCCCGGATGAAGAGTCGCCTTCGTTCT
GCCTGCATACTGACAGTACATGTAGGACTTGCGGGGATGTAGCTGTATACCAGGATGT
ATATGCAGTGCACGCACCCACCTCGATCTACCACCAAGCGGTGAAGGGTGTGAGGACA
GTGTATTGGATAGGATTCGATACGACCCCCTTTATGTTTCCTACATTGGCAGGATCGTA
CCCATCGTACGCCACTAACTGGGCTGATGAGACTGTCTTGCAAGCGCGCAATATCGGA
CTGTGTGCAAACGGTCTGACAGAGGGCGGTAGAAAGGACTCTCAATCCTAAGAAAG
AAGGCGTTACGTCCTAGCGGTAAAATACTGTTCTCTGTGGGTTCGACACTTTACACAGA
GAACCGAGAAATGCTGCGTAGTTGGCATTTACCATCAGTCTTCCACCTCAAGGGCAAG
AAGAGTTTCACAGCGCGCTGTGATACAATTGTATCGTGCGAGGGATACGTGGTGAAGA
AGATCAGCCTGTGTCCAGGCCTGTACGGAAGACCGTCAGGCTATGCCGTTACATATCA
TAGTGAAGGATTCTTGATCAGTAAAGTTACGGACACCATCAGAGGAGAGAGGGTATCA
TTCCCGGTGTGTACGTACGTTCCAGCAACTTTATGTGATCAGATGACAGGAATCTTAGC
TACTGAGGTCTCCCCCGATGACGCACAGAAGCTGCTGGTGGGGCTAAACCAGAGGATT
GTGGTTAACGGCCGCACACAGCGTAACACCAACACCATGAAGAATTACCTGCTGCCAG
TCGTCGCCCTGGCCTTTAGCAAGTGGGCGAAAGAATATCGGGCGGATCTTGAAGATGA
GAAATTGCTAGGAGTCAGAGAGAGAGAGCTTACTTGTTGCTGTATGTGGACATTTAAG
ATCAGAAAAAGCCACACCATGTACAAGAAGCCTGAGACCCAGACTATAGTGAAAGTT
GTGTCAGAGTACTCCAGTTTTCTCTTTAAGAGCCCGTGGACAGAGGGCCTTTCAATTGG
ATTGTGCACGAAAATCAAGCTGATGTTAGACAATGTGAAGCCTCGGAAAACCGTGACA
ATCACGGAGAGTGACGTTAAAGAGGCTCAGGACAGGCAACAGGAAGCCCATGAGGAG
AGGGAAGCGGAACTGGATAGAGAGGCATTACCGGCTCTGCAGCCAACAGCGCCACCT
GTGGAGCAAATTCAGGTGGACTTCGAGGACCTAGAGAGAGCAGGGGCTGGGGTAGTG
GAGACACCCAGACGAGCAGTCAGGGTCACAGCCCAGGCCGGGGACACAATGATAGGT
GAATACCTTCTTGTCTCCCCCCAAACGGTCTTACGAAGTGCAAAGCTGGAGTGCGTGC
ATGAGTTAGCTGAACAGGTAAAGATTATGACTCATTCTGGCCGATCCGGTAGGTACCC
 
CGTTGAAGGGTACGATGGTCGAGTATTACTCCCCGCCGGAACAGCCCTTGAAATACCG
GATTTTCAAGCCTTGAGCGAAAGCGCCACAATGGTTTACAACGAACGTGAATTTGTGA
ACCGCAAGCTGAAACACATAGCGCTGCACGGGGCATCATTAAACACCGATGAGGAGA
ACTATGATCATGTGGATGCAGAAGCAGTGGACCACGAGTACGTATTCGACGTGGACAA
AGGGATGTGCGTAAAAAGGGACCAGACCTCCGGTCTAGTGCTGGTGGGAGATCTTACC
AACCCGCCCTACCACGAGTTTGCTTACGAGGGGCTAAGGATTCGCCCCTCCGCAGTGC
ATAAGGTACCCATTATAGGAGTTTTTGGTGTGCCTGGCTCCGGCAAGTCTGCTATCATC
AAGTCTCTCGTGAACACGAGGGATCTGGTAACCAGCGGAAAGAAGGAGAATTGCACC
GAGATAGTCAATGATGTGAAGAAGCAAAGAAACATGACTATCATTGCAAAGACCGTG
GATTCCATCTTACTGAATGGATGCAGGAGCAGACCGGAGACTTTGTACGTCGACGAGG
CGTTCGCATGCCACGCAGGTACCCTGCTGGCGCTAATCTCTATTGTCAAACCATCAAAG
ΛΛΛGTTGTGCTGTGTGGGGΛCCCTΛΛGCΛGTGCGGΛTTCTTCΛΛCΛTGΛTGCΛGTTGΛ
AGGTCAATTACAATCATGATATCTGCACGCAGGTGTTCCATAAAAGCATATCACGTCG
GTGTACGCAGCCGATTACGGCAATCGTGTCCACATTGCATTATGGTGGAAAAATGAGG
ACTACAAACCCCTGCACGAGACCGGTGGAGATTGATATAACAGGAAAGACCAAACCA
CAGAAAGGTGACCTGATTTTGACGTGCTTCCGTGGGTGGGTTAAACAGCTGGAGATAG
ATTACCGCGGGCATGAGGTTATGACAGCAGCTGCCTCCCAAGGTTTGACACGCAAGGG
CGTATACGCAGTCAGGCAAAAAGTAAACATGAACCCGCTGTATGCTGAAAAATCAGA
GCATGTCAACGTGTTGCTGACGAGGACAGAGGATCGTCTGGTGTGGAAAACGTCATCA
GGAGACCCTTGGATATCTGTGCTGACTAATGTCCCGCCAGGCAATTTCAGCGCCACGA
TCGAGGAGTGGGAAGCCGAGCACAACGCAATAGTGTCTGTTTTGGAAGGGGCGCATGC
GCCGATCGATGTCTTTGCCTGCAAAAGTAGAGTATGCTGGGCGAAGGCCCTGCAACCT
GTCTTGGACACTGCAGGCATACAAATGACGGCAGACCAGTGGAGCGAGCTGCTTCCGC
CGTTCAAGGAAGATCAGGCATATTCACCCGAAGTGGCGTTGAATGCGATCAGCACAAG
ATTCTATGGTTACGATCTGGATAGCGGGTTATTCTCTGCTGACACCGTGTCGCTGAGAT
ACACAGAAAACCACTGGGACAACGAGCCAGGCGGCAAGAAGTACGGCTTTGATCACG
CGGTGGCCCGCCGACTGGAACAACGACACCCGTACCTCAAAAACAAGTGGAAATTAA
ACCAACAGCTGTTGGTTGCGGAGGGAGTCGCACAGCCAGTGTCAACAACGTGCAACGT
GGTGCCCGTTAACAGGAGACTACCACATCCGCTGGTATTGCAGCACGAGGTGCTGCAA
GGCAAACCTGTAGAGGATTTTCTGATGCAGTTTGTCGCGCAGGATGTGGCAGTGGTGG
CCCCCCGCAGAGTGAGTATGCCCTTGCGTAAGGTCACGTGGGTATCTGATATCAAGTA
TGACGGCGACATCAGGTGCCATCTGGACGTGGGTCTGCCTGCAGTTATGGGAATGTAC
GACCTAGTGGCCGTGCTTGAAGATACTACATACAGAGGCCACCACTACCTGCAGTGCG
AGGACCACGCTTTGAAAATGCACATGTTAGCGGGGGACGCCGTGAAGCACCTGAGGCC
GGGCGGGACTCTTGTGGTTAAGTGCTATGGCTACGCAGATAGGTTCAGTGAGATGGTT
GTCTGTGCACTCGGTCGCAAGTTCCGCAGGGTGAGGGCGTGTCGCCCTCCTTGCGTGA
ACAGTAACACCGAGATGTACTTGGTATTTACTCACTTTGACAACCGCAACAGACCGTTC
ACACTAAAGACACTGAACGAGACGTACGCTCAGCATTTGCCAAGAGCGGGGGCAGCA
CCGGCATATCGCGTGCGCAGAGCTGATATCGCAAACTGTACAGAAGAAGTGGTGGTGA
ATGCCGCTAACAGCAGAGGCGTGATAGGGGAAGGCGTCTGCGGGGCTATCGGCAGGA
AGTGGCCCGCGGCCTTCAAGGGTTCAGCCACGCCCGTAGGGACAGCCAAGCTCACCAA
 
GAGTCCTAGGCCGGTGATCCATGCAGTAGGACCTAATTTCCATCAGGTGACTGAGTTG
GAAGGGGAACAACAACTCAGAGCTGCATACCAGGCGGTTGCCGATTTGGTGAATAAG
GAAAACTACACCAGCGTGGCGATACCGCTGCTGTCTACTGGTATATATGCGGCCGGTA
AAGATAGGCTTATGCAGTCCTTGAACCACCTTTTTACCGCTATGGATAACACAGATGCA
GATGTTACTATTTATTGCAGGGATAAGAAATGGGAAAGAACAATTGCAGATGCGATCA
GGCAACGTGAAACACCTGAGGCGTTGTCACTGACTGTGGATGAGCCATTAGATGTCGT
GAGGGTTCACCCGCTCAGTAGCCTGGTGGGAAGACCAGGTTACAGTAAAGAGACCGG
GAAATTACATTCGTACCTTGAAGGGACGAAGTTCCATCAGACCTCCATGGATATGGCT
GAGATCTACACGATGTTTCCAAAAGTGGAAGATGCAAACGAGCAAATATGCATGTACG
TTATGGGGGAGACGATGGACCAGATTAGGCAGAAATGCCCCGTGGAAGATCAGGATT
CATCGTCCCCGGTCGCAACGGTGCCATGCTTGTGTCGACTTGCGATGACAGCAGAAAG
AGTTCAGAGACTTCGCGCCGTGTCGACCAAACAGTTTGCAGTGTGCTCGTCTTTTCCGT
TGCCAAAGTATCGGATCCAGGGAGTACAGAAGGTACAATGCGGGCAAGTGTTACTATT
TTCTAGTGGTACTGTCAACCATGTGAGCCCGCGTAAATACACTGTAGTTGCCCCCAGAT
CAGACTCCGTTTCTGTGTGTTCTGTGCGTACACCATCTGACGCATGTTCTCAGGCATCT
GTAACTTTTGATATTATCTGTGACACTCCACCGGCAGCACGCAGGTCGATGGCCCGGC
AAACGTTAGATGAGGATATTGAATCTATCACTGATACCCCGATTATTCACGCTCCTATT
GTAGAGAGCGTGACAGAAGTGGAGGTACATGCACCACCTCCAGAGCATGTAATCCCTG
TGTCTGCACCACGGAGACAGACACCGGTGCCTCGCCCAAGAACAATATTTAGAGGTAG
AGCACCGGAATTCTGCCCGTCAAGCGCACTGTTATTTGGAGATTTCGCGGAGGGTGAA
GTAGAGCACATCCTCCAAAGACCAATTGCGAAACCCAGAGTTAGGATTACATTTGGGG
ATTTTACAGCTGAAGAGAGTGAGGCCATCCGTCTGCGAAGCTTTGCCCTGCCGCAATG
ACTAGGCCGAGCCGGGGCCTACATCTTTTCAACAGACGTAGGACCCGGCCACTTACAA
CAGAAGTCGGTCAGACAGAATGATAGTACTGAAACCATCCTCAATGAAGTGAAGCCTG
ATAAATTCTACCCACCGCGGTATGATGAAGCTGCTGAGGAGAAATTAAGAGTGAGACA
TCAGCTGGTCCCTTCCGCTGCTAACAGGAGCAGGTACCAATCCCGCAAAGTGGAGAAC
ATGAAAGCAGTGACCATTAATCGACTTTTAAGCGGATTACATAAGTATCTGGACTCAG
AAGAACATGAACCAACGTACAGGGTCACCTACCCGCGGCCACAATATTCAGCAACGGT
GGTTGAAGAACTATCAAGCGCTTGCACAGCAGTGGCCGCGTGCAATGCCGTACTTACG
GAGAATTACCCTACAGTGACCAGTTACCAGATTACGGATGAGTACGACGCATATTTAG
ACATGGTGGATGGGTCAGAGAGTTGCCTGGACAGGGCGAACTTCAACCCTTCCAAGCT
ACGCAGCTTCCCAAAGAAGCATGCGTACCATGATCCTCAGATTCGTAGCGCCGTCCCG
TCCGCTTTCCAGAATAACTTACAGAATGTGCTAGCAGCTGCTACTAAACGTAACTGTAA
TGTTACACAAATGCGGGAATTGCCTGTCTTGGACTCAGCAGTGTTTAATGTGGAATGTT
TCAAAAAATTTGCATGTAATAATGAATACTGGGAAGAGTTTAAACAGAAGCCGATCAG
GATAACAACTGAAAACGTGACCCAGTATGTGACTAAACTGAAGGGGCCGAAAGCAGC
TGCGTTATTTGCAAAAACACACAACCTGATACCACTCCAAGAAGTACCCATGGATAGG
TTTACCATGGACATGAAGAGGGATGTGAAGGTTACGCCAGGCACGAAGCACACGGAA
GAACGTCCGAAGGTGCAGGTTATACAGGCTGCTGAACCGCTCGCAACCGCATACATGT
GCGGTATCCACAGAGAGTTGGTCAGACGGCTAAATGCAGTGCTGTTGCCTAATATCCA
TACCTTATTCGATATGTCGGCTGAGGATTTTGATGCAATAGTAGCCGAGCATTTTCATA
 
ATGGGGATAAGGTGCTGGAGACGGACATTGCTTCGTTTGACAAAAGCCAGGATGATTC
TCTGGCGTTGACAGCCCTCATGATTTTGGAGGATCTCGGGGTGGATGACAGATTGCTTG
ACCTGATAGAGATTGCCTTTGGGGAAATAACCAGCATCCATCTGCCGACTGGGACCAA
ATTTAAGTTTGGGGCCATGATGAAGTCCGGAATGTTTCTGACGTTATTTGTTAACACGC
TGCTTAACGTCGTTATAGCTAGTCGCGTCCTAGAATCCAAATTGACGGGGTCGCGATGT
GCCGCCTTCATTGGGGACGATAACATCGTGCATGGCGTGGTCTCAGATAAGTTGATGG
CAGAAAGGTGTGCCACCTGGATGAACATGGAGGTGAAAATTATCGACGCAGTCATCGG
AGAAAAACACCCGTATTTCTGCGGCGGGTTCATCCTACAGGATGCTGTGACCGGCACG
GCGTGCCGAGTATCCGACCCATTGAAGAGACTGTTTAAGTTGGGTAAACCACTGCCTG
CGGACGATGAGCAGGATGAGGACCGCAGACGAGCACTCCGTGACGAGGTGATGAGAT
GGTTTAGGGTAGGTCTGCGGTCTGAGGTGTGTGCTGCAGTTTATTCCAGGTACGGCGTG
CAGGGGCTGGATGTTGCTTTGATGGCTATGGCAACCCTGTCGAAGACTAGGAAGCACT
TCGACATGATTAGGGGACCCGTAAGGGTTCTCTACGGTGGTCCTAAATTGTAG
SEQ ID NO: 49 - NDUV subgenomic promoter
   CAGCCACAGAGTGACGCTACTACACTGTGCCTGCTACGCC
SEQ ID NO: 50 - sequence of NDUV 3' UTR 
CTTTGGTGTGGTCCCAGCATGCTGAGGTATTATAGATAACTTAGTTAGGTATTATAGGT
AACTTAGGTGTAAGCAGAAAAATGGAAAACCGAATAAAAAGTTAGAGTAAGTAGTGT
AAATTAGAAAATAAGTTTTGATAGTGGTAGTTAGGTGTAAGCAGAAAACAGAAAAAC
GAATAAAAAGCTAGAGTAAGTAGTTAGCTGCATATAGAGGTAGTATAGGTGTAAGCA
GAAAATGGAAAACCAGTAAAAAGTTAGAGTAAGTAGTAGAGAATAAGTTTTGCTATG
CATTAGATAGGTTGCTTTGATTTTATAGAAAATAGTAGATGCTTATAGAGGTAGTATAG
GTTTAAGCAGAAAAATACAGAAAAATCTAAAGATATGCGACGAGCAGACCGTCGTGA
GCGCCAATTGGATCGGCGCAACAGGGTTATTGGACTGCCCTGCATGAACCCTTATTGG
ACGATGGGTTCTTCGTCTGCAAAATTCATATTTAAAATTTTGTTTTTATTTTTTGATTCG
ATCAATTGGTTTTTAATATTTCCT
SEQ ID NO: 51 - sequence of RNV 5' UTR
   ATGGGCGGCGCATTAGAGAGTAGCCCAAAACTAAACTACCCAAC
SEQ ID NO: 52 - sequence encoding RNV nonstructural protein nsP1-4 
ATGGAGAAAGTTCACGTTGACATCGAGGAAGACAGTCCGTTCCTCAGAGCTTTACAAC
GGAGTTTTCCACAGTTTGAGGTAGAAGCCAAGCAGGTCACAGACAACGACCATGCTAA
TGCTAGAGCGTTTTCGCATCTAGCTTCCAAATTAATCGAAACGGAGGTTGACCCATCCG
ATACGATTTTAGACATAGGCAGTGCGCCTGCCCGCAGAATGTACTCGAAACACAAGTA
CCACTGTATCTGTCCGATGAAATGCGCCGAAGATCCGGACAGATTATTTAAGTATGCA
TCCAAATTAAAGAAGAACTGCAAAGACATAACTGACAAGAATTTGGATAAGAAAATG
GAAGACCTGGCAGCAGTGATGACTGAGCCCGACATCGAGCGAGAAACGCTATGCCTG
CATGATGATGAATCTTGCAGGTATACTGGGCAAGTCGCCGTCTATCAAGACGTTTATGC
TGTCGACGGTCCTACGAGCCTTTACCATCAGGCCAACAAAGGGGTAAGAGTTGCATAT
TGGATAGGATTTGACACCACGCCGTTCATGTTCAAGAACCTCGCGGGGGCCTACCCGT
CTTATTCCACAAACTGGTCCGACGAGGTTGTGCTGACGGCGCGGAACATCGGACTGTG
CAGTTCTGACGTCATGGAAAGATCACGACGCGGACTCTCCATAATGCGTAAAAAATTC
 
CTCAAACCCTCCAGTAACATCATATTTTCTGTAGGATCAACTATTTATCATGAGAAAAG
AGACTTGTTGAAAAGCTGGCATTTACCGTCCGTATTTCACCTACGTGGAAAAAACAAC
TACACGTGTCGGTGTGAGACTATAGTTAGTTGCGACGGGTACGTCGTCAAAAGGATAG
CTATTAGTCCGGGCCTGTATGGGAAACCGTCAGGCTACGCTGCCACTATGCACCGCGA
GGGATTCTTGTGCTGCAAGGTGACGGACACGCTGAACGGGGAGAGGGTATCTTTTCCC
GTTTGCACATATGTGCCGGCAACTTTGTGCGACCAAATGACAGGCATCTTAGCTACTGA
CGTAAGTGCTGATGATGCTCAAAAATTGTTGGTTGGGCTCAACCAGCGTATTGTCGTGA
ATGGACGCACTCAAAGAAACACCAATACCATGAAGAACTACTTACTACCTGTAGTGGC
GCAGGCGTTCGCGCGCTGGGCCAAGGAATACAAAGAAGATCAAGAGGATGAACGACC
TTTAGGGCTCAGAGACCGTCAACTGGTAATGGGGTGCTGCTGGGCATTTAGAAAACAC
AAGGTTACCTCCGTCTACAAAAAACCAGACACCCAAACCATTGTCAAAGTGCCAAGTG
ATTTCCACTCATTTGTGCTTCCTCGATCTGGGAGTACGGCGCTCGATATCAAGCTGCGA
GATCGCATCAGGAAATTGATGGAGCGCAAGAAACAGCCGCCGCCCCTGACAACACCG
GAGGACGTACAAGAGGCCACGGCGGCAGCAGTAGAGGCGCGCGAAGTTCGCGAAGCC
GAGGAATTGCGTGCAGCACTACCACCCTTAGCACCAGATGTCGAGGAGCCGGCCCTCG
AAGCAGATGTCGACTTAATGCTACAGGAAGCCGGGGCAGGTACGGTGGAAACGCCTA
GAGGACTCATCAAAGTTACTAGCTATCCAGGGGAGGAAAAGATCGGCTCCTATGCTAT
TCTTTCTCCTCAAGCTGTGCTGAAGAGTGAAAAACTCAGCTGCATCCATCCACTGGCAG
AGCAAGTTGTGGTGATTACCCATTCAGGGCGGAAAGGTAGATACGCAGTAGAACCTTA
TCATGGCAAAGTAGTCGTTCCTGAAGGTCATGCTATTCCTGTCACCGATTTCCAAGCGT
TAAGTGAAAGCGCTACCATCGTGTACAACGAACGAGAATTCGTGAACAGGTACCTACA
CCACATTGCAGTGAACGGTGGAGCATTAAACACTGATGAGGAGTACTACAAAACTGTA
AAACCATCTGAATATGAAGGGGAATACTTGTACGACATCGATAAGAAACAATGTGTAA
AGAAAGAACTCATTACCGGGTTAGGACTGTCCGGCGAGCTAATTGAACCACCTTTTCA
TGAGTTCGCCTATGAAAGCTTGAAGACAAGACCGGCAGCACCTTACCAAGTACCTACG
ATTGGTGTGTACGGAGTACCCGGTTCAGGCAAGTCTGGTATTATAAAAAGTGCAGTAA
CCAAAAGAGACTTGGTAGTGAGTGCAAAGAAAGAAAACTGCGCGGAAATAATCCGCG
ATGTGAAACGAATGAGAGGTCTAGATGTGATAGCACGCACTGTGGACTCTGTATTGCT
CAATGGATGCAAGTACCCAGTAGACACCTTGTATGTGGATGAAGCTTTTGCTTGCCAC
ACTGGTACCTTGCGCGCATTGATTGCCATCGTGAAGCCGAAGAAGACGGTACTGTGTG
GAGACCCGAAGCAGTGCGGATTCTTTAACATGATGTGCTTGAAAGTCCATTTCAACCA
TGAACTGTGCACGCAGGTATTCCATAAGAGCATATCCAGAAGATGCACACCTGCAGTC
ACCTCTATCGTTTCTACCCTATTTTATGACAAACGCATGAGAACCACGAATACTCGCCC
TACGAAAATTGAAATTGATACAGTAGGAGGCACAAAACCACGTAAAGATGATTTGATC
CTTACATGTTTTAGAGGGTGGGTGAAGCAGTTGCAAATTGATTACAAGGGCAATGAGA
TAATGACGGCGGCGGCGTCCCAGGGCCTTACACGTAAGGGAGTGTATGCCGTCAGGTA
TAAGGTCAACGAGAACCCCCTGTATGCCGCGAATTCCGAGCACGTTAACGTGCTACTG
ACTAGGACTGAAGACAGAATAGTCTGGAAAACGCTGGCAGGAGATCCGTGGATCAAG
ACACTCACGGCAAAATACCCAGGGAACTTCTCCGCAACCTTGGAAGAGTGGCAACAGG
AACATGATGCCATCATGAGGCATATATTGGAAAAACCCCAACCAACCGACGTGTTCCA
GAATAAAGCAAATGTATGCTGGGCCAAAGCACTGGTGCCCATACTCCGCACCGCGGGC
 
ATCTCTATGACTCAAGAGCAGTGGAATACAATTCCGTATTTCAAAGAAGATAAAGCAC
ATTCAGCTGAGATAGTACTCAACCAGTTATGCGTGCGTTTCTTCGGCTTGGATTTGGAC
TCCGGACTGTTCTCTGCCCCTACAGTGCCACTGGCCATAAAAAATAATCACTGGGACA
ACTCACCGTCACCAAATATGTATGGTATTAATAAGGAAGTGGTTCGGCAACTGGCCCG
TCGGTACCCTCAATTGCCGAGGGCGACAGCCACTGGGAGGATGCTCGACGTAAATACC
GGTACGTTGAGAGAATATGATCCCAAAGTTAACCCAGTGCCCGTTAACCGTAGACTGC
CACATGCCCTGGTGACATCACACACTAATCACCCCGCCTGCGATTACTCCGCTTTTGCA
AGCAAACTAAAGGGGCGGACTGTACTTGTGATCGGAGAGAAAATGAATATTCCCGGA
AAGACTGTCGACTGGCTTTCAGACCATCCGGATTCGACCTTCCGGTCTCGACTAGATCT
TGGAATTCCGGCAGATGTACCCAAGTATGACTTAATATTCATTAATGTCCGCACCCAGT
ACATACATCACCACTACCAGCAGTGTGAAGATCACGCCATTAAACTAAGTATGCTGAC
TAAGAAAGCGTGCCTCCACTTGCAACCAGGAGGCACCTGTGTTAGTATTGGCTACGGA
TATGCGGACAGAGCTAGCGAGAACATTATCGGGGCCGTCGCTAGACAATTTAAATTCG
CCCGAGTGTGCAAACCACGTATTTCCATGGAGGAGACTGAGATCTTGTTTGTATTCATA
GGGTATGACCGAAAGAACCGTACTCATAATCCCTACAAGTTGTCTACGACTTTGACAA
ACATCTACACAGGCTCAGGGCTCCACGAGGCTGGCACAGCTCCATCCTACCACGTAGT
TAGAGGGGATATAGCCACGGCCACCGAGAGTGTCATAGTGAACGCAGCCAATAGCAA
AGGACAACCAGGAGGTGGAGTATGCGGGGCACTGTACAAGAAATTTCCTGATAGCTTC
GACATGCAGGCTATAGAAGTTGGAAAGGCCCGGTTAGTGAAAGGTGGCAAGCACATT
ATTCACGCAGTCGGGCCGAATTTCAACAAAGTGGCGGAGATGGAAGGTGATAAGCAG
CTAGCCGAGGCTTATGAGTCTATCGCTAAGCTAGTAAATGACAACAATTACCAGTCCG
TAGCCATCCCCTTGTTGTCCACAGGAATTTTTGCAGGGAGCAAGGACAGACTTATGCA
GTCTCTTAACCATCTATTGACTGCTATGGATACAACTGATGCAGATATTGCAATATATT
GTAGGGACAAGAAGTGGGAAACGACACTGAAGGAGGTCATAGCACGAAGAGAAGCG
GTGGAGGAAATATGTATCTCAGAAGATTCTAACGAAGAACCTGATGCGGAACTAGTTA
GAGTACACCCCAAGAGTTCACTGGCCGGTAGAAAGGGGTACAGTGTGACGGATGGAA
AAACTTTTTCTTACCTAGAAGGTACTAAGTTCCACCAGGCTGCCAAGGACATAGCGGA
GATTAACGCCATGTGGCCCAAAGTAACAGAGGCCAACGAACAAATTTGCTTGTACATC
CTAGGGGAGAGCATGAGCAGTATACGTTCGAAATGTCCTGTGGAAGAATCTGAGGCCT
CCACGCCTCCAACTACCTTGCCGTGCCTGTGCATCCATGCTATGACGCCTGAAAGAGTG
CAAAGGCTTAAGGCTTCGCGCCCTGAGCAGATTACTGTTTGTTCCTCTTTTGCTTTGCCC
AAGTATCGTATTACGGGTGTGCAAAAAATTCAATGTTCCACCCCTATACTGTTTTCACC
GAAGGTTCCCGATTATATACCACCACGGCGATACATTCCTTCGAGTGGGCAATCTTCAG
AAGTCGTGCTACCTGAGCCACCTGCGGCAGCTGCAGCACCTGCGCAAGAACCTGAGCC
CGTCGCTCCGGAACCTGAAGCAGTAACTCCCACTGTGCCACCTAATGCCGTCATTGTTC
AGGCGGATGTGCACGCTGAACAAGCTTCCTCTAGGTGGTCTATACCTTCAGCTTCTGAT
TTTGAACTGGACAGCATTTCCATACTAGACGGATTCAGCATGCACCAGAGCTCATCTGC
AACTAGTACTGGTACGTTTTCCATAAGGTCAGCACCATCTTTAAATTCATTAGCTAGTA
GTGCCAATCAATCGCAGGCCAGTTCGAGAGCTAGCGCAAGTACATCTAGATCGAATCG
CAGTGCTCGGACTAGTCGTAGCTCTCGGGCAAGTTCCAGGACCAGTGCCGGCACCAGA
AGCTCACGGACTAGTAACGCCAGCGTTAGAACATCTACCTCTAATCAACCTGGCAATG
 
TTTACCAGCAAATCCCGGCTGCAGCGCCAACCAGAGCTGCGAGAAGTACAGGCTCAAG
GACAAGCTTAGTATCTAATCCGCCGGGAGTGAACAGAGTGATTACTAGGGAAGAATTC
GAGGCTTTCGTGGCGCAACAACAATGACGATATGAAGCGGGTGCATACATATTCTCCT
CGGACACCGGCCAAGGACATTTACAGCAAAAATCGGTGAGGCAGACGGTGCTGTCCG
AGGTCGTGCTAGAACGCACGGAATTGGAGATATCGTATGCCCCGCGCCTCGATTTAAA
TAAAGAAGAAATTTTAAGGAAGAAACTGCAGGCCAACCCCACCCCGGCCAACCGTAG
TCGTTACCAGTCACGCAAAGTTGAAAATATGAAAGCTGTTACTACCCAGCGTGTCCTC
AAAGGATTAGGACACTTTTTGAAGGCTGAAGGCAAGGTTGAATGCTATCGAACACTGT
ATCCGATCCCCATTTACTCCTCAAGCGTTGACAGGGCATTTTCTAATCCGCGGGTAGCC
GTGGAGGCGTGCAATGTTATGCTGAAGGAAAATTTTCCAACCGTCACCTCGTACGGGA
TCATTCCTGAGTACGATGCTTATTTGGATATGGTGGATGGTGCAACCTGCTGCTTGGAC
ACAGCGAACTTCTGTCCAGCCAAGTTGCGTAGCTTTCCAAAAAGACATGCCTACTTAG
AACCGACAGTGAGATCGGCTGTCCCTTCCGCTATACAGAACACACTGCAGAATGTATT
GGCAGCGGCCACGAAAAGAAATTGCAATGTGACACAAATGAGAGAATTACCCGTATT
AGATTCAGCTGCATTTAATGTGGAATGCTTTAAGAAATTTGCCTGCAATCAGGAGTATT
GGAAGACCTTCAAAGAAAATCCCATTCGACTGACCACAGAAAATGTAACGAATTATAT
TACCAAGTTAAAAGGCCCAAAAGCCGCGGCCCTATATGCTAAGACCCACAATCTCGCA
ATGCTGCAAGATATTCCCATGGACCGGTTTGTAATGGACCTGAAACGCGATGTTAAAG
TAACACCAGGTACCAAGCACACTGAAGAACGTCCTAAAGTGCAGGTAATACAGGCAG
CAGACCCACTCGCAACCGCGTATCTGTGTGGAATACATAGGGAACTTGTGAGGAGGCT
AAATGCAGTACTCCTGCCTAATGTGCATACTTTGTTTGATATGTCAGCAGAAGACTTTG
ATGCCATTATTGCAGAACATTTTTCACAAGGTGACATAGTGCTTGAGACTGACATTGCA
TCCTTTGACAAGAGTGAGGACGATGCGATGGCGCTAACAGCGCTGATGATTTTGGAAG
ATCTAGGCGTCGACCAGGAACTGTTAACATTGATAGAAGCAGCTTTTGGGGAAATAAC
ATCAATTCATTTGCCAACCAAGACAAAATTCAGGTTCGGAGCCATGATGAAATCTGGC
ATGTTCTTAACCTTGTTCGTGAATACTATCATCAATATAGTCATAGCTAGTCGAGTGCT
CCGTGAGCGGCTAACCAATTCACCGTGTGCCGCATTTATAGGTGATGACAACATTATC
AAGGGGGTTAAGTCCGACAAGCTGATGGCGGATCGATGCGCGACCTGGCTGAACATG
GAGGTTAAGATCATTGATGCAGTAGTCGGTGAGAAAGCACCATACTTTTGTGGAGGGT
TTATACTGTGTGACTCAGTTACAGGTACAGCATGTAGGGTGGCTGATCCACTGAAACG
ACTATTTAAACTTGGAAAGCCACTGGCTGCCGACGACGAACATGATGACGATCGGCGC
CGAGCACTCTATGAGGAAGCCGAACGGTGGTGTCGAGTAGGTATCTTCGCTGAGTTGT
GTAAGGCAGTGGAGTCACGTTATGAAGTGATAGGAACCTCCATCATTATAATGGCTAT
GGCAGCCTTGGCAAAGGATAATGCCGCCTTTAGACACCTAAGAGGGAACCCCATAGTT
CTCTACGGCTAA
SEQ ID NO: 53 - RNV subgenomic promoter
   CCTGAATGGATTGCACGTTAGTTCGATCCGCTACA
SEQ ID NO: 54 - sequence of RNV 3' UTR 
GAAGAAGCAGCAATTGGCAAGCTGCATGCAAAATATATGTAGAGCAAATTACGACAG
CAATTGGCAGGCTGTATAGAGTTTATTAGATTTTGTGTATAATTGTTTAGCAGCAATT
 
GGCAAGCTGCTTTTAAAATTTTATTAGATTTTTTTTTTTTTTATATACCAATTGGATTT
TGTTTTTAATATTTC
SEQ ID NO: 55 - sequence of MUCV 5' UTR
   ATGGGCGGCGTATGAGAGTAGCCCAAATTAAAACTACCCATT
SEQ ID NO: 56 - sequence encoding MUCV nonstructural protein nsP1-4 
ATGGAGAAAGTTCACGTTGATATCGAGGAGGATAGCCCCTTCCTCAGAGCTTTGCAAC
GGAGCTTCCCCCAGTTTGAGGTAGAAGCCAAGCAGGTCACAGACAATGACCATGCTAA
TGCTAGAGCGTTTTCGCATCTAGCTTCCAAACTGATCGAAACGGAGGTAGAACCATCC
GACACGATCCTAGACATTGGAAGTGCGCCCGCCCGCAGAATGTATTCAAAACACAAGT
ACCATTGTGTCTGTCCGATGAGATGTGCTGAAGACCCGGACAGATTATTTAAGTATGC
AGCCAAGCTGAAGAAGAATTGTAAAGACATAACAGACAAGGACCTAGACAAAAAGAT
GGCAGAGCTCGCAGCTGTCATGGAGGACCCCGATTTGGAGCAGGATACCATATGTTTG
CATGATGATGAAACGTGCCGCTATGAAGGACACGTTGCTGTTTACCAGGACGTCTATG
CGGTAGACGGTCCCACCAGCATTTACCATCAAGCAAATAAAGGTGTAAGAGTGGCCTA
TTGGATCGGCTTTGATACCACGCCGTTTATGTTTAAAAATTTGGCCGGAGCCTATCCTT
CCTACTCTACAAATTGGGCTGACGAAACAGTGTTGACAGCCCGTAACATCGGCTTATG
CAGCTCAGATGTCATGGAAAGGTCCCGTAGAGGTTTGTCTATTTTAAGGAAGAAATAT
TTAAAACCATCGAATAACATCATATTTCAGTGGGTTCCACCATCTACCATGAAAAA
GAGACTTGTTAAAAAGTTGGCACCTACCTTCTGTGTTTCACCTACGTGGCAAGCAGAAT
TACACATGTCGGTGTGAGACGATAGTGAGTTGCGACGGGTACGTCGTAAAACGGATCG
CTATCAGTCCAGGCCTGTATGGGAAACCGTCGGGCTATGCTGCCACTATGCATCGCGA
GGGATTCTTGTGCTGCAAGGTAACAGATACGCTCAACGGGGAGAGGGTCTCTTTTCCC
GTCTGCACGTATGTGCCAGCGACTCTATGCGACCAAATGACCGGCATATTGGCTACAG
ATGTCAGTGCCGAAGACGCACAAAAGCTGTTGGTTGGGCTCAACCAACGCATTGTCGT
AAATGGCCGCACACAGAGAAATACTAATACTATGAAAAACTATTTATTACCAGTAGTT
GCACAGGCTTTTGCCAGGTGGGCTAAAGAATACAAAGATGACCAAGATGATGAGAGA
CCGCTAGGGTTACGGGACCGACAGCTCGTGATGGGCTGTTGTTGGGCATTTAAGAAAC
ACAAGATTACATCTGTGTATAAACGTCCCGACACTCAAACGGTTATCAAGGTACCTAG
TGACTTCCATTCCTTTGTGCTACCCAGACCTGGTAGCAATACATTGGAAATTGGGCTTA
GAACCCGGATAAAGAAGTTACTGGAAGATAAAAAGATACCACCACCTATAATTACTCC
AGAAGACGTCTCAACAGCAAAAGAAGCCGCAGCGGAAGCCAAGGAGATTAGGGAGGC
TGAGGAGTTGCGAATCGCACTACCCCCTATAGTGCCGGACGTGGAAGAACCAACTCTA
GAAGCCGACGTGGACTTGATGCTGCAAGAGGCAGGCGCTGGTTCCGTCGAGACTCCCA
GAGGATTAATCAAAGTTACTAGCTACTCTGGTGAGGATAAGATTGGTTCTTATGCCGTG
TTGTCGCCTCAAGCCGTCCTGAGAAGTGAAAAACTGTCCTGCATACACCCCCTTGCGG
AGGAAGTCATCGTAATAACGCACGCAGGAAGGAAAGGACGCTATGCTGTTGAGCCGT
ATCATGGCAAAGTGGTGGTCCCTGAAGGACATGCGATCCCTGTAGCAGACTTTCAGGC
TCTCAGTGAGAGCGCTACTATTGTGTACAACGAACGAGAGTTTGTGAACAGGTACTTG
CATCACATCGCCATTAATGGAGGAGCCTTGAATACCGATGAAGAGTACTATAAAGTGG
TCAAACCTACCGAGTATGATGGAGAGTACTTGTATGATATTGACAGGAAACAGTGTGT
CAAGAAAGAACAGGTGTCTGGCCTCGGCCTGACAGGTGAGCTGGTAGAGCCTCCATTT
 
CACGAATTCGCGTATGAAAGCCTGAAGACCAGACCGGCAGCTCCATATCAAGTGCCTA
CCATAGGAGTGTACGGAGTACCAGGCTCGGGTAAGTCAGGAATTATAAAAAGTGCCGT
TACCAAGAGAGATCTGGTGGTCAGCGCAAAGAAGGAAAATTGCGCCGAGGTTATCCG
CGACGTGAAGAAAATGCGAGGCTTAGACATAGTGGCACGCACTGTAGATTCAGTGCTG
CTGAATGGCTGCAAGCACCCAGTTGATACACTGTACATTGATGAGGCTTTTGCTTGTCA
TGCCGGGACCCTCCGCGCTTTGATTGCAATTATTAAACCAAAAAAGGCAGTTCTCTGCG
GAGACCCGAAGCAGTGCGGCTTCTTTAATATGATGTGCCTCAGAGTGCATTTCAATCAC
GAAATTTGCACGCAGGTGTTCCATAAAAGCATATCCCGTAGGTGCACTAAATCGGTCA
CATCCGTGGTGTCTACGCTGTTTATGACAAGAAGATGCGCACTACTAATCCCAGGGA
GACAAAAATTGAAATTGACACCACAGGGAGTACAAAGCCTAGAAAAGATGATCTCAT
ATTGACGTGCTTCAGGGGATGGGTAAAGCAGCTGCAAATTGACTACAAGGGCAATGAA
ATCATGACGGCGGCAGCCTCACAGGGATTAACCCGAAAAGGGGTTATGCCGTCAGGT
ATAAAGTCAATGAGAATCCCCTATACGCCGCAAATTCAGAGCACGTCAACGTCCTACT
CACTCGTACAGAGGATAGGATTGTGTGGAAAACCCTGGCAGGAGATCCTTGGATTAAA
ACTCTTACGGCCAAGTACACCGGTGACTTTACTGCTACCTTGGAAGAATGGCAACAAG
AGCATGACGCCATTATGAAGCACATTCTGGAAAGACCTGATCCTGCGGACGTGTTCCA
GAATAAAGCCAATGTGTGCTGGGCTAAAGCCTTAGTACCCGTACTGAAGACTGCAGGT
ATTGACCTAACGGCAGAGCAGTGGAACACCATTGTCCAATTCAGAGATGACAAGGCTC
ACTCGGCAGAAATAGCACTCAACCAATTGTGTGTACGGTTCTTTGGACTAGATTTGGAT
TCAGGGTTGTTTTCTGCCCCCACTGTGCCGTTGTCTATAAAAAATAACCACTGGGATAA
TTCACCTTCACCCAATATGTACGGGTTGAATAAGGAAGTAGCGCGACAACTTTCTCGA
CGCTACCCGCAGCTTCCTCGTGCAGTGGCGACTGGCAGAGTTTTGGACATGAATACTG
GGACCCTGCGTGGTTACGACCCATGCATTAATCTTGTGCCCGTTAATCGGAGGCTACCG
CATGCATTGGTAACGCACCATTCAGAGCAACCACGCAGCGATTTTTCGGCATTTGCCA
GCAAGTTGAGGGGTAGGACTGTCCTGGTTGTAGGAGAGAAACTAAACATAACCGGGA
AAAATGTTGACTGGCTATCCGAAAGTCCCGAAGCCACTTTCAAATCGCGCCTGGATCT
CGGTATCCCGCACGAACTGCCCAAATATGACATTGTGTTTGTGAATGTCCGGACACCTT
ACAAGTACCATCACTACCAGCAGTGTGAAGACCATGCGATCAAGCTCAGTATGTTGAC
AAAGAAAGCGTGCTTACATCTTAATCCTGGTGGAACCTGTGTCAGCATTGGCTATGGCT
ACGCAGATCGAGCCAGTGAAAGCATTGTCGGAGCTATTGCAAGGCAGTTTAAGTTCTC
CCGAGTGTGTAAGCCAAAAGTATCAACGGAGGAGACAGAGATACTATTTGTCTTCATA
GGGTATGACCGGAAGGTTCGTACACACAATCCCTATAAATTGTCTTCAACATTGACCA
ACATTTACACAGGCTCACACTTACATGAGGCAGGCTGCGCTCCATCCTACCATGTAGTG
CGTGGTGATATTGCCAATGCTGAGGAGGGAGTAATTGTCAACGCCGCTAATAGCAGAG
GGCAACCTGGGGGTGGGGTATGTGGAGCTCTTTACAAACGCTTCCCGGAGAACTTCGA
CCTGCAGCCCATAGAGGTCGGCAAGTCAAGGCTGGTTAAAGGGGCGGCTAAACATATC
ATTCATGCAGTAGGACCCAATTTTAATAAGGTGTCGGAACTTGATGGGGACAAGCAAT
TGGCAGAAGCTTATGAATCAGTAGCTAAAATCATTAATGATAATCATTACCGCACCGT
GGCAATTCCGCTGCTGTCGACAGGTATTTTTGCCGGAAACAAAGACAGACTCATGCAG
TCATTAAATCACCTGCTTACGGCATTAGACACTACCGATGCAGATGTAGCTATATACTG
CAGAGATAAGAAGTGGGAAACTACTCTGAAGGAAGTTATAGCACGGAGAGAGGCTGT
 
CGAAGAGATATGTATCTCTGAAGACGCATCCGTAGCAGAACCTGACGCAGAGCTGGTA
AGAGTACATCCCAAAAGTTCGCTGGCAGGACGCAAAGGCTTTAGCACGGTCGACGGG
AAAACATTTTCATATTTGGAAGGGACTAAATTTCATCAGGCAGCGAAGGATGTGGCCG
AGATTAATGCGATGTGGCCCACAGTTACCGAGGCTAATGAGCAAATATGCCTGTACAT
TTTGGGCGAAAGTATGAGCAGCATCCGATCGAAGTGCCCCGTTGAAGAATCTGAGGCG
TCAACTCCACCCAGCACGTTGCCGTGTCTCTGTATCCATTCTATGACACCGGAACGAGT
ACACAGGCTTAAGGCCTCACGTCCAGAGCAAATAACGGTGTGTTCGTCGTTTCCCCTAC
CCAAGTACAGAATCACCGGGGTGCAGAAGATTCAATGCGCTCAGCCTATACTCTTCCT
CCCGAAAGTGCCGGAACATATCCATCCACGCAGGTATCTTCCCCCGCAGGAGAACAGA
GAGGAAGAGGAGAATTCTGTGGAAACGCTGCCTGAATCGCCTGAAATTCCTACAGTTG
CTGCCGCCTCTGAAATTGACACCCAAACGGAAGAGACCATGAGCATAACGTCGGAAAC
GACTGTTGCCTCGTTCGTGCCTAACGAAATTGTGGTCGAGGCGGAAATACACTCGGAT
CTCATGCGATTCTCCTCTGACTGGTCCATTCCTCAAGCCTCTGACTTTGACGTTGACAG
CGTGTCGGTTCAAGGTACTGTGGACATATTTAACAGTGCGCTGCAGGAGGATGCGCGT
AGCGCCGTATCATCTTACTGCTCGGGAATAAAACCCATTCCCGCGCCACGTACCGTTTT
TATCCAGCCACCGAAACCTCGCAGAAGACGTCGTAAGTCGAAGGCCAGTACCAGTTCT
ACGGCTCCCTTGGTAACTGTGTCCAGCGCCACTTCGAGATCTAGTTTGGTGTCTAACCC
ACCCGGAAGAAAAGGGGTTATTACCAGAGAGGAGTTCGAAGCCTTCGTCGCGCAACA
ACAATGACGGTACGAAGCGGGTGCGTACATCTTCTCCTCCGACACCGGTCAAGGGCAT
CTACAACAAAAATCTGTGAGGCAAACAGCGCTGTCCGAAGTGGTGCTTGAACGGACGG
AGTTGGAGAATTCGTACGCCCCGCGCCTCGACTTAATAAAAGAGGAAAGTTTACGTAA
GAAATTGCAATTGAATCCGACACAGGCTAATAGGAGCAGGTATCAATCCAGAAGAGT
GGAGAATATGAAAGCAGTGACCACCAAAAGACTACTCGGCGGACTGGGACATTACTT
GAAGTCTGAAGGAAAAGTTGAATGTTACCGTACATTGTACCCCGTCCCCTTGTATTCAG
CAAGCGTTAACCGCGTTTTCTCGAATCCTCGAGTGGCAGTCGAAGCGTGTAATGCTATG
CTAAAGGAGAACTTTCCAACAGTGGCTTCGTACTGCATAACACCAGAGTACGATGCGT
ACATGGACATGGTGGACGGCGCCTCTTGTTGTTTAGACACTGCGAGTTTTTGCCCTGCC
AAATTGCGTAGTTTTCCTAAGAGACATGCTTATTTGGAGCCGACCATTCGCTCAGCAGT
GCCGTCTGCTATACAAAATACCTTGCAGAATGTACTAGCAGCGGCCACCAAACGGAAC
TGCAATGTTACACAAATGAGAGAGCTACCTGTGTTGGATTCCGCCGCCTTCAATGTAG
AGTGCTTTAAGAAGTATGCATGCAATAATGAATATTGGGAAACATATAAGGAATGTCC
TATTAGATTAACTGAAGAGAATGTGACTAATTATATTACTAAATTGAAAGGTCCGAAG
GCGGCTGCTTTGTATGCTAAAACCCACAACCTTAGCATGCTTCAGGACATTCCCATGGA
TAGATTTGTTATGGATTTAAAAAGAGATGTGAAGGTGACACCTGGTACCAAACATACG
GAGGAGAGACCCAAGGTACAGGTAATCCAGGCAGCAGATCCATTGGCCACAGCCTAC
TTATGCGGGATTCATCGTGAACTGGTGCGGAGGTTAAACGCAGTTTTACTGCCGAACG
TCCATACCTTGTTCGACATGTCCGCTGAGGATTTCGATGCCATTATTGCTGAACATTTC
CAACCTGGGGACAGTGTGTTGGAGACCGACATCGCTTCGTTCGATAAGAGCGAGGACG
ATGCGATGGCACTGACAGCATTGATGATCCTGGAAGATTTAGGCGTAGACCAAGAGTT
GCTTACGTTGATTGAAGCCGCATTCGGAGAAATATCATCCATCCACCTTCCCACGAAA
ACTAAATTTAAATTTGGTGCTATGATGAAATCTGGTATGTTTCTTACCCTGTTTGTGAAT
 
ACAGTTATTAATATTGTTATAGCCAGCAGAGTGCTCCGAGAACGCCTGACACAGTCGC
CTTGCGCTGCATTTATCGGCGATGACAACATTGTTAAAGGCGTTAAATCGGATAAGTTG
ATGGCCGACAGGTGTGCCACGTGGTTGAACATGGAGGTAAAAATTATTGATGCCGTAG
TAGGAATAAAGGCCCCGTACTTTTGCGGCGGGTTTATCCTGTGTGACACGGTAACCGG
CACGGCATGTCGTGTGGCCGATCCTTTGAAGAGACTGTTTAAACTCGGGAAACCTCTC
GCGGCTGACGATGAACACGATGACGATCGTCGCCGCGCTCTGCAGGAGGAATCAGCCC
GCTGGAACCGCGTAGGCATACACTCTGAATTATGCAAGGCGGTGGAGTCACGTTACGA
GACCACAGGCACGTCCGTGATTATAACAGCTATGACCACCTTGGCACAGAGTGTTCCT
GCGTTCAAGCACCTGAGAGGAAACCCAGTAACTCTCTACGGCTGA
SEQ ID NO: 57 - MUCV subgenomic promoter
   CCTGAATGGACTGTAACGTAGTTCAGTCCGCAACC
SEQ ID NO: 58 - sequence of MUCV 3' UTR 
ACCACAGCAGCGATTGGAAAGCTGCCTATTAGAAACATGTAGCGGCAATTGGCAAGCC
GCCTATAAATGTTTAGCAGCAATTGGCAAGCTGCATATATAAATTACCTAGCGGCAAT
TGGCACGCCGCTTATAAAATTTTTATTTTCTTTTACCAATAATTGGATTTTGTTTTTAAT
ATTTC
SEQ ID NO: 59 - sequence of HJV 5' UTR
   ATAGGGCATGGTATAGAGGCACCTACCCTACAAACCGATCCAAAC
SEQ ID NO: 60 - sequence encoding HJV nonstructural protein nsP1-4 
ATGGAAAAAGTTCACGTTGACTTAGACGCCGACAGCCCGTTCGTCAAGTCACTGCAAC
GGAGCTTTCCGCAGTTTGAGATCGAAGCCAAGCAGGTCACAGACAATGACCATGCTAA
TGCCAGAGCGTTTTCGCATGTGGCTACAAAGCTCATAGAGAGCGAAGTCGACCGGGAG
CAGATTATACTGGACATAGGAAGTGCACCTGTAAGGCATGCACATTCCAATCACAAGT
ACCATTGTATCTGCCCAATGATTAGTGCAGAGGACCCGGATCGACTACACAGATACGC
AGAGAAATTGAAGAAGAGTGACATTACTGACAAGAACATAGCCTCTAAAGCTGCAGA
TCTACTCGAAGTCCTTTCCACTCCCGATGTGGAGACACCATCACTATGTATGCACACGG
ACACAACATGCCGGTATTTCGGCAGCGTGGCAGTGTACCAAGATGTATATGCGGTTCA
CGCCCCGACGTCCATCTACCACCAGGCGCTAAAAGGCGTAAGAACTATATACTGGATC
GGCTTCGACACTACCCCGTTCATGTATAAAAATATGGCAGGTTCGTACCCCACCTACAA
CACTAATTGGGCTGATGAGAGCGTTTTAGATGCTCGAAATATAGGTCTCGGCAACTCT
GACCTTCAGGAGAGTCAGTTTGGTAAGCTCTCCATTCTTAGAAAGAAACGCTTACGGC
CGACAAACAAGATCATATTTCGGTGGGGTCGACGATCTATACTGAAGATCGTTCGTT
GTTACGCAGCTGGCACCTACCGAACGTGTTCCATCTTAAAGGGAAATCAAGCTTTACT
GGTAGGTGCGGAACCATAGTCAGCTGTGAGGGGTACGTCATCAAGAAGATCACATTAA
GTCCTGGATTGTATGGCAAAGTAGAAAATTTAGCGTCCACTATGCATCGCGAGGGATT
CTTGAGTTGCAAAGTAACGGATACACTACGCGGTGAGAGGGTATCTTTTGCCGTCTGT
ACCTACGTACCAGCCACACTGTGCGATCAAATGACAGGGATCCTGGCAACAGACGTCA
GTGTGGACGACGCACAAAAACTGCTGGTTGGGCTCAACCAGCGAATTGTCGTCAATGG
TAGGACGCAACGTAACACTAATACCATGCAGAACTACCTCCTACCAGTTGTTGCCCAA
GCTTTCTCCAGGTGGGCGCGTGAATATCGCGCCGACCTCGAGGATGAGAAGGAATTAG
GGGTACGGGAACGTACCCTTACAATGGGCTGCTGCTGGGCTTTTAAGACGCACAAAAT
 
CACGTCTATTTACAAGAAACCAGGCACACAGACCATTAAGAAAGTCCCTGCTGTCTTT
GACTCATTCGTACTACCACGCCTTACGAGCCACGGCCTGGACATTGGGCTTAGACGCA
GGTTGAAGATGCTGCTAGAGCCCGCCGTTACGGCGGCCCCAAATATTACATCTGCTGA
TGTTGAACACTTACGCGGTCTTCAGCAAGAAGCTGAGGAAGTGGCAGCCGCGGAAGA
ACTGAGAGAGGCATTACCGCCTCTGCTCCCCGAAATAGAAAAGGAGACTTTAGAGGCT
GAGATAGACCTCATCATGCAGGAGGCGGGTGCCGGAGCGTGGAGACACCTCGCAGA
CACATCAAGGTAACTAGTTACCCAGGAGAAGAGACTATTGGGTCGTATGCTATCCTCA
CTCCTCAAGCGGTCCTCAACAGTGAGAAACTAGCTTGCATACATCCCCTGGCTGAACA
GGTGCTTGTCATGACACACAAGGGGAGGGCAGGCAGATACAAAGTGGAACCATATCA
TGGGAAAGTAGTGGTTCCCGAAGGGGTAGCAATACCGATCCCTGACTTCCAGGCCCTC
AGTGAGAGTGCAACCATCGTGTTTAACGAACGTGAGTTTGTTAACAGATATTTGCACC
ACATAGCCATCAACGGAGGAGCACTGAATACTGATGAAGAGTACTATAAGGTCGTCAA
ATCGCATGAGGCAGAATCAGAATACGTGTTCGATATTGACGCGCGTAAGTGCGTCAAG
AAAGCGGACGCGGGCCCACTTTGTCTGATCGGAGAACTTGTCGACCCTCCATTCCACG
AATTCGCGTATGAAAGTCTGAAAACCCGTCCGGCTGCACCCTTGAAAGTACCTACCAT
CGGAGTGTATGGTGTGCCCGGCTCCGGTAAGTCGGGCATAATCAAAAGTGCTGTCACA
AAGAAAGACCTCGTCGTCAGCGCCAAGAAAGAGAACTGCGCTGAAATTATCAAGGAT
GTGAAAAGAATGAGGCAAATGGACATAGCTGCACGCACAGTCGATTCTGTGCTTCTAA
ATGGTGTGAAACACCCTGTCAATATCTTGTATATTGACGAAGCATTTGCGTGCCACGCC
GGCACTTTGCTGGCTGTGATTGCTATTGTCAAACCTAAGAAAGCAGTGCTATGTGGTGA
TCCCAAGCAGTGCGGATTCTTTAACATGATGTGTCTGCGAGTGCATTTTAACCATGACA
TATGCACTGAAGTACACCACAAGAGTATATCAAGGAGGTGTACGCAGACAGTGACAG
CTATAGTATCAACTCTCTTTTATGATAAACGCATGAAAACCGTTAATCCATGTGCCGAT
AAAATCATAATTGATACTACAGGCACTACGAAACCGAACAAAGATGACTTGATACTGA
CTTGCTTTCGTGGATGGGTGAAGCAATTACAAATAGATTACAAGAACCATGAGATCAT
GACTGCCGCAGCATCCCAAGGACTCACGCGTAAAGGCGTGTACGCAGTGAGATATAAA
GTCAATGAAAACCCTCTCTATGCTCAGACTTCGGAGCACGTTAACGTGCTACTAACCCG
CACTGAAAAACGAATTGTTTGGAAGACGCTAGCAGGGGACCCATGGATAAAGACTCTC
ACCGCACAATACCCAGGTGATTTTACAGCCACGCTAGATGACTGGCAACGTGAGCACG
ACGCCATCATGGCTAGGGTACTTGATGCCCCGTATGCCACCGATGTTTACCAGAATAA
GGTCAATGTCTGTTGGGCAAAGGCTTTAGAGCCGGTCCTAGCCACGGCCAATATCCAG
TTAACTCGTGCACAGTGGGAAACCCTAGCCCCTTTCAGAAATGACAAAGCGTACTCGC
CTGAGATGGCTTTAAATTTCTTCTGCACCCGATTCTTTGGTGTGGACCTGGACAGCGGA
TTATTTTCAGCACCCGCTGTTGCACTCACCTATAAGGATCATCACTGGGATAATTCGCC
AGGAAGAAATATGTATGGCCTCAATCGCGAAGTAGCTAAAGAGTTGGCCCAGCGTTAC
CCGTGCCTGATTAAAGCCATAGATACCGGCAGGGTGGCAGATATTCGTAATAATACCA
TAAAAGACTACCTGCCCACGATTAACATAGTCCCACTCAATCGCAGGCTACCTCACTC
ACTGGTTGTTCATCACAAGAACTTGGGCCAAACAGACTACAGCGACTTCCTATCTAAG
CTGAAGGGCAGGACAGTGTTGGTCATAGGAGACCCAGTGAAGATTCCAGGAAAAAGA
GTGGAATCAATAGGGCCATCCCCGGATAACACCATCCGTTGTAATCTCGACCTAGGCA
TACCAACCACAGTTGGCAAGTATGATATGGTATTCGTGAACGTCAGGACCCCGTATCA
 
ATATCATCACTACCAGCAGTGTGAAGACCACGCCATACACCACAGTATGTTAACCTGC
AAAGCAGTGCAGCACCTTAACAACGGCGGCACCTGCGTCGCCGTGGGTTATGGCATGG
CAGATCGCGCCACAGAGAATATTATAACGGCAGTGGCTCGTTCTTTCAGGTTCACTCGC
GTTTGCCAGCCCAAAAGCACAGCCGAGAATACAGAAGTGCTGTTTGTGTTCTTTGGTA
AGGACAATGGCAATCATTTGCAGGATCAGGACCGGTTGAGCGTTGTGCTGGACAGCAT
CTACCAAGGCTCCACTAGATATGAGGCGGGTCGCGCTCCGGCGTATCGCGTCATTCGG
GGAGATATCTGTAAGAGTGAGGACCCAGTCGTGGTCAACGCTGCGAACAGCAAAGGA
CACCCAGGGTCAGGGGTTTGTGGGGCAATCTACAGAAAGTGGCCTGGCGCCTTTGACA
ACCAACCGATCGCTGTAGGAGCAGCACGATTGATTAAGCACACACCGAACGTTATCCA
TGCTGTGGGCCCCAATTTTTCCAAGGTATCGGAGGCAGAAGGGGACACAAAATTGGCG
GAAGTATATATGAACATAGCCGCCATTATTAATGCCGAACGGTATACCAAAGTGTCGA
TCCCGCTGCTGTCAACTGGAATTTATTCGGGCGGAAAGGACAGAGTTATGCAATCCTT
AAACCACCTGTTCACGGCTCTGGATACCACTGACGCAGACGTAACTATATACTGTTTGG
ACAAACAGTGGGAAGCTCGCATAAAAGATGCAATTGCACGTAAAGAGAGCGTCGAGG
TGCTTGACACCGAAGACAGACCCGTAGACATCGACCTGGTGCGTGTTCACCCAAACAG
TTCGCTAGCCAACAGACCAGGCTATTCAGTTACCGAAGGCAAGGTATACTCGTATTTG
GAAGGAACACGCTTCCACCAGACTGCTAAAGATATCGCTGAAATACATGCGATGTGGC
CAAACAAGGCAGAAGCTAACGAACAAATCTGCTTGTATATCTTGGGAGAGAGTATGGC
TAGCATCAGATCCAAGTGTCCAGTTGAAGAGTCTGAAGCATCGGCACCACCACATACA
CTACCGTGTCTGTGTAACTATGCCATGACAGCAGAACGGGTGTTTAGATTACGCGCAG
CAAAGAAAGAACAGTTTGCTGTGTGTTCCTCTTTTCCGCTACCGAAGTACCGTATTACC
GGTGTGCAGAAGCTGCAGTGTAGTAAACCAGTTCTCTTCTCAGGCATAGTACCACCTG
CCGTCCATCCCAGAAGATATGCTACGACCACCATTGAGGTCGCCACATCCCCTATTGA
GCAGCGACCACTCCGAGAAGCGCCTCCAGTACCAGCCCGAATTCCCAGCCCGATCAGC
AACCGTACAATAAGCGTGGAGTCGCTGCTCTCAGTGGGAACACAATCAGCGTCTATCA
GCTGGGATTTGCACGAACAGGTGATCACAGCTGATGTCCACCGCGACGCTGAGAGTTC
CGCATGGAGCATTCCTAGCGCCTCCGGCTTCGAGGTGCTTCCCAGCCCAACACCGAGT
CACAGCATTTCTAATATATCCTGGGAAGAAGTGTCAGCGGACTGTACAAGAGAACGGT
GCGTGGCGGACATTATGCAAGATTTCCGCAGTGCACCGTTCCAATTCCTATCTGACTAT
AAACCGATACCTGCGCCTAGGAGCAGACCGACGCCCGCTCCGAGATCAACGGTATCTG
CACCGCCGATACCTAAACCGCGCAAGATAGTGTACAGGCAACCCCCAGGAGTAGCTAG
ATCTATATCGGAGGCTGAACTGGATGAATACATTCGCCAGCACACGAATTGACGGTAC
GAAGCGGGTGCGTACATCTTCTCCTCTGAGACGGGCCAAGGACACCTCCAGCAGAAAT
CAGTACGGCAATGCAAACTTCAAGACGCAGTACTAGAGAGGGTCACTCATGAGAAGT
ACTACGCCCCGCGCCTCGACCTAGAAAAAGAGAAGTTGTTGCAGAAGAAACTGCAACT
GTGTGCATCGGAAGCTAACAGAAGCAGATACCAATCACGTAAAGTAGAAAATATGAA
GGCGATAACAGCCGATCGCCTTATTTCAGGACTTGGAACGTACGTATCCACAGAGGCC
CAAGCAGTTGAATGCTACAGAGTAACGTATCCAGTACCACAATACTCTGCAGTCATCC
CTAACAAGTTCTCATCTGCTACAGTGGCAATTAAGGTATGCAACCTGGTGATACAAGA
AAACTATCCCACCGTCGCTAGTTACTGCATCACGGACGAGTACGATGCTTACCTAGAC
ATGGTGGATGGAGCATCGTGTTGCCTTGATACGGCCACGTTCTGTCCGGCTAAACTACG
 
AAGCTATCCGAAAAAACATAGCTATTTGCAGCCAGAGATCAGGTCAGCTGTCCCATCG
CCTATACAAAACACTTTACAGAATGTGCTGGCAGCGGCGACTAAACGCAATTGTAATG
TGACACAGATGCGTGAGTTGCCGGTCCTGGACTCCGCTGCGTTCAACGTGGAGTGCTTT
AAACGCTTTGCCTGCAATGATGAATATTGGGACATATTTAAAAATAGCCCTATCCGACT
GACGACGGAAAATGTTACCCAATACGTCACTAAGCTGAAGGGTCCTAAGGCTGCAGCC
CTCTTTGCTAAAACGCACAACCTCAAACCGCTGCATGAGATACCGATGGATCAGTTTGT
GATGGATTTAAAGCGCGACGTCAAGGTGACACCAGGCACGAAGCACACCGAAGAACG
CCCTAAGGTGCAAGTTATTCAAGCAGCCGACCCGCTAGCAACAGCTTACTTGTGCGGA
ATACACAGAGAGCTTGTGAGACGTCTGAATTCAGTGCTATTACCAAATATTCACACCCT
CTTTGATATGTCGGCAGAAGATTTTGATGCCATCATAGCCGAACACTTTCATTATGGAG
ATCCAGTGCTGGAAACTGACATCGCCTCATTCGACAAGAGCGAAGACGACGCTATAGC
ACTATCCGCTCTAATGATTCTGGAGGATTTAGGCGTAGATCAGCCACTGCTGGATCTCA
TTGAGGCTGCATTTGGTAACATAACTTCTGTGCACTTACCAACCGGCACCCGGTTTAAG
TTTGGAGCTATGATGAAATCAGGGATGTTTTTGACCTTGTTTGTTAATACTTTAGTTAA
CATCATGATAGCCAGTCGGGTACTTAGAGAAAGGTTGACCACTTCAGCGTGTGCAGCC
TTTATCGGAGACGACAATATAGTACACGGTGTAGTATCCGATAATTTGATGGCTGATA
GGTGTGCCACGTGGCTAAATATGGAAGTAAAAATCATTGATGCCGTGATTGGCGTAAA
AGCACCATACTTTTGTGGAGGGTTCATCCTGGTCGATCAAATTACAGGTACAGCGTGTA
GGGTAGCAGACCCCCTCAAGCGTTTGTTTAAACTGGGTAAACCACTCCCTTTAGACGAT
GACCAGGATGTAGACCGCCGTCGAGCGTTGTACGATGAGTCTCTAAGGTGGAATAGGA
TAGGTATAACCTCTGAGCTTGTTAAGGCGGTTGAATCCCGATATGAGGTTGTTCTGTCT
AGCTTAATTATTATTGCGCTGTCAACACTTGCAGATAGCGTGAAGAACTTTAAGCGGAT
AAGAGGAAACCCCATTACTCTTTACGGCTGA
SEQ ID NO: 61 - HJV subgenomic promoter
   CCTTAATAGGTGACGTAGTAGATACGCACCTAACCGCGAAA
SEQ ID NO: 62 - sequence of HJV 3' UTR 
CCCGGCGTTCCCCTGACCACAGCGGCGAGCACTCGATGTACTTCCGAGGTAACGTGGT
GCATAATGCCACGTGCCGCTAGACACCAAAACTCGATGTACTTCCGAGGAAGCACAGT
GCATAATGCTGTGCAGTGTCGCATTTAACCAATTATTATTATCCATATATTATGTTATC
ACTACACATTATAACTCTAAACATGTAACACTACAGGAATTTTACTAGTATACGTTACC
GTATCTAGTGGGTTCCACTATAATCAACACTACATGAATTTTATTCAAACACACTACAC
ACTTATAACAACTTTTAAAATTTTGATTAGATTACTATATTTTTCTTTTCTTTATTTTTCT
TTTATTTTGTTTTTAAAATTTC
SEQ ID NO: 63 - sequence of VEEV 5' UTR
   ATGGGCGGCGCATGAGAGAAGCCCAGACCAATTACCTACCCAAA
SEQ ID NO: 64 - sequence encoding VEEV nonstructural protein nsP1-4 
ATGGAGAAAGTTCACGTTGACATCGAGGAAGACAGCCCATTCCTCAGAGCTTTGCAGC
GGAGCTTCCCGCAGTTTGAGGTAGAAGCCAAGCAGGTCACTGATAATGACCATGCTAA
TGCCAGAGCGTTTTCGCATCTGGCTTCAAAACTGATCGAAACGGAGGTGGACCCATCC
GACACGATCCTTGACATTGGAAGTGCGCCCGCCCGCAGAATGTATTCTAAGCACAAGT
ATCATTGTATCTGTCCGATGAGATGTGCGGAAGATCCGGACAGATTGTATAAGTATGC
 
AACTAAGCTGAAGAAAAACTGTAAGGAAATAACTGATAAGGAATTGGACAAGAAAAT
GAAGGAGCTCGCCGCCGTCATGAGCGACCCTGACCTGGAAACTGAGACTATGTGCCTC
CACGACGACGAGTCGTGTCGCTACGAAGGGCAAGTCGCTGTTTACCAGGATGTATACG
CGGTTGACGGACCGACAAGTCTCTATCACCAAGCCAATAAGGGAGTTAGAGTCGCCTA
CTGGATAGGCTTTGACACCACCCCTTTTATGTTTAAGAACTTGGCTGGAGCATATCCAT
CATACTCTACCAACTGGGCCGACGAAACCGTGTTAACGGCTCGTAACATAGGCCTATG
CAGCTCTGACGTTATGGAGCGGTCACGTAGAGGGATGTCCATTCTTAGAAAGAAGTAT
TTGAAACCATCCAACAATGTTCTATTCTCTGTTGGCTCGACCATCTACCACGAGAAGAG
GGACTTACTGAGGAGCTGGCACCTGCCGTCTGTATTTCACTTACGTGGCAAGCAAAATT
ACACATGTCGGTGTGAGACTATAGTTAGTTGCGACGGGTACGTCGTTAAAAGAATAGC
TATCAGTCCAGGCCTGTATGGGAAGCCTTCAGGCTATGCTGCTACGATGCACCGCGAG
GGATTCTTGTGCTGCAAAGTGACAGACACATTGAACGGGGAGAGGGTCTCTTTTCCCG
TGTGCACGTATGTGCCAGCTACATTGTGTGACCAAATGACTGGCATACTGGCAACAGA
TGTCAGTGCGGACGACGCGCAAAAACTGCTGGTTGGGCTCAACCAGCGTATAGTCGTC
AACGGTCGCACCCAGAGAAACACCAATACCATGAAAAATTACCTTTTGCCCGTAGTGG
CCCAGGCATTTGCTAGGTGGGCAAAGGAATATAAGGAAGATCAAGAAGATGAAAGGC
CACTAGGACTACGAGATAGACAGTTAGTCATGGGGTGTTGTTGGGCTTTTAGAAGGCA
CAAGATAACATCTATTTATAAGCGCCCGGATACCCAAACCATCATCAAAGTGAACAGC
GATTTCCACTCATTCGTGCTGCCCAGGATAGGCAGTAACACATTGGAGATCGGGCTGA
GAACAAGAATCAGGAAAATGTTAGAGGAGCACAAGGAGCCGTCACCTCTCATTACCG
CCGAGGACGTACAAGAAGCTAAGTGCGCAGCCGATGAGGCTAAGGAGGTGCGTGAAG
CCGAGGAGTTGCGCGCAGCTCTACCACCTTTGGCAGCTGATGTTGAGGAGCCCACTCT
GGAAGCCGATGTCGACTTGATGTTACAAGAGGCTGGGGCCGGCTCAGTGGAGACACCT
CGTGGCTTGATAAAGGTTACCAGCTACGATGGCGAGGACAAGATCGGCTCTTACGCTG
TGCTTTCTCCGCAGGCTGTACTCAAGAGTGAAAAATTATCTTGCATCCACCCTCTCGCT
GAACAAGTCATAGTGATAACACACTCTGGCCGAAAAGGGCGTTATGCCGTGGAACCAT
ACCATGGTAAAGTAGTGGTGCCAGAGGGACATGCAATACCCGTCCAGGACTTTCAAGC
TCTGAGTGAAAGTGCCACCATTGTGTACAACGAACGTGAGTTCGTAAACAGGTACCTG
CACCATATTGCCACACATGGAGGAGCGCTGAACACTGATGAAGAATATTACAAAACTG
TCAAGCCCAGCGAGCACGACGGCGAATACCTGTACGACATCGACAGGAAACAGTGCG
TCAAGAAAGAACTAGTCACTGGGCTAGGGCTCACAGGCGAGCTGGTGGATCCTCCCTT
CCATGAATTCGCCTACGAGAGTCTGAGAACACGACCAGCCGCTCCTTACCAAGTACCA
ACCATAGGGGTGTATGGCGTGCCAGGATCAGGCAAGTCTGGCATCATTAAAAGCGCAG
TCACCAAAAAAGATCTAGTGGTGAGCGCCAAGAAAGAAAACTGTGCAGAAATTATAA
GGGACGTCAAGAAAATGAAAGGGCTGGACGTCAATGCCAGAACTGTGGACTCAGTGC
TCTTGAATGGATGCAAACACCCCGTAGAGACCCTGTATATTGACGAAGCTTTTGCTTGT
CATGCAGGTACTCTCAGAGCGCTCATAGCCATTATAAGACCTAAAAAGGCAGTGCTCT
GCGGGGATCCCAAACAGTGCGGTTTTTTAACATGATGTGCCTGAAAGTGCATTTTAAC
CACGAGATTTGCACACAAGTCTTCCACAAAAGCATCTCTCGCCGTTGCACTAAATCTGT
GACTTCGGTCGTCTCAACCTTGTTTTACGACAAAAAAATGAGAACGACGAATCCGAAA
GAGACTAAGATTGTGATTGACACTACCGGCAGTACCAAACCTAAGCAGGACGATCTCA
 
TTCTCACTTGTTTCAGAGGGTGGGTGAAGCAGTTGCAAATAGATTACAAAGGCAACGA
AATAATGACGGCAGCTGCCTCTCAAGGGCTGACCCGTAAAGGTGTGTATGCCGTTCGG
TACAAGGTGAATGAAAATCCTCTGTACGCACCCACCTCAGAACATGTGAACGTCCTAC
TGACCCGCACGGAGGACCGCATCGTGTGGAAAACACTAGCCGGCGACCCATGGATAA
AAACACTGACTGCCAAGTACCCTGGGAATTTCACTGCCACGATAGAGGAGTGGCAAGC
AGAGCATGATGCCATCATGAGGCACATCTTGGAGAGACCGGACCCTACCGACGTCTTC
CAGAATAAGGCAAACGTGTGTTGGGCCAAGGCTTTAGTGCCGGTGCTGAAGACCGCTG
GCATAGACATGACCACTGAACAATGGAACACTGTGGATTATTTTGAAACGGACAAAGC
TCACTCAGCAGAGATAGTATTGAACCAACTATGCGTGAGGTTCTTTGGACTCGATCTGG
ACTCCGGTCTATTTTCTGCACCCACTGTTCCGTTATCCATTAGGAATAATCACTGGGAT
AACTCCCCGTCGCCTAACATGTACGGGCTGAATAAAGAAGTGGTCCGTCAGCTCTCTC
GCAGGTACCCACAACTGCCTCGGGCAGTTGCCACTGGAAGAGTCTATGACATGAACAC
TGGTACACTGCGCAATTATGATCCGCGCATAAACCTAGTACCTGTAAACAGAAGACTG
CCTCATGCTTTAGTCCTCCACCATAATGAACACCCACAGAGTGACTTTTCTTCATTCGT
CAGCAAATTGAAGGGCAGAACTGTCCTGGTGGTCGGGGAAAAGTTGTCCGTCCCAGGC
AAAATGGTTGACTGGTTGTCAGACCGGCCTGAGGCTACCTTCAGAGCTCGGCTGGATT
TAGGCATCCCAGGTGATGTGCCCAAATATGACATAATATTTGTTAATGTGAGGACCCC
ATATAAATACCATCACTATCAGCAGTGTGAAGACCATGCCATTAAGCTTAGCATGTTG
ACCAAGAAAGCTTGTCTGCATCTGAATCCCGGCGGAACCTGTGTCAGCATAGGTTATG
GTTACGCTGACAGGGCCAGCGAAAGCATCATTGGTGCTATAGCGCGGCAGTTCAAGTT
TTCCCGGGTATGCAAACCGAAATCCTCACTTGAAGAGACGGAAGTTCTGTTTGTATTCA
TTGGGTACGATCGCAAGGCCCGTACGCACAATCCTTACAAGCTTTCATCAACCTTGACC
AACATTTATACAGGTTCCAGACTCCACGAAGCCGGATGTGCACCCTCATATCATGTGGT
GCGAGGGGATATTGCCACGGCCACCGAAGGAGTGATTATAAATGCTGCTAACAGCAA
AGGACAACCTGGCGGAGGGGTGTGCGGAGCGCTGTATAAGAAATTCCCGGAAAGCTT
CGATTTACAGCCGATCGAAGTAGGAAAAGCGCGACTGGTCAAAGGTGCAGCTAAACA
TATCATTCATGCCGTAGGACCAAACTTCAACAAAGTTTCGGAGGTTGAAGGTGACAAA
CAGTTGGCAGAGGCTTATGAGTCCATCGCTAAGATTGTCAACGATAACAATTACAAGT
CAGTAGCGATTCCACTGTTGTCCACCGGCATCTTTTCCGGGAACAAAGATCGACTAACC
CAATCATTGAACCATTTGCTGACAGCTTTAGACACCACTGATGCAGATGTAGCCATATA
CTGCAGGGACAAGAAATGGGAAATGACTCTCAAGGAAGCAGTGGCTAGGAGAGAAGC
AGTGGAGGAGATATGCATATCCGACGACTCTTCAGTGACAGAACCTGATGCAGAGCTG
GTGAGGGTGCATCCGAAGAGTTCTTTGGCTGGAAGGAAGGGCTACAGCACAAGCGAT
GGCAAAACTTTCTCATATTTGGAAGGGACCAAGTTTCACCAGGCGGCCAAGGATATAG
CAGAAATTAATGCCATGTGGCCCGTTGCAACGGAGGCCAATGAGCAGGTATGCATGTA
TATCCTCGGAGAAAGCATGAGCAGTATTAGGTCGAAATGCCCCGTCGAAGAGTCGGAA
GCCTCCACACCACCTAGCACGCTGCCTTGCTTGTGCATCCATGCCATGACTCCAGAAAG
AGTACAGCGCCTAAAAGCCTCACGTCCAGAACAAATTACTGTGTGCTCATCCTTTCCAT
TGCCGAAGTATAGAATCACTGGTGTGCAGAAGATCCAATGCTCCCAGCCTATATTGTTC
TCACCGAAAGTGCCTGCGTATATTCATCCAAGGAAGTATCTCGTGGAAACACCACCGG
TAGACGAGACTCCGGAGCCATCGGCAGAGAACCAATCCACAGAGGGGACACCTGAAC
 
AACCACCACTTATAACCGAGGATGAGACCAGGACTAGAACGCCTGAGCCGATCATCAT
CGAAGAGGAAGAAGAGGATAGCATAAGTTTGCTGTCAGATGGCCCGACCCACCAGGT
GCTGCAAGTCGAGGCAGACATTCACGGCCGCCCTCTGTATCTAGCTCATCCTGGTCCA
TTCCTCATGCATCCGACTTTGATGTGGACAGTTTATCCATACTTGACACCCTGGAGGGA
GCTAGCGTGACCAGCGGGGCAACGTCAGCCGAGACTAACTCTTACTTCGCAAAGAGTA
TGGAGTTTCTGGCGCGACCGGTGCCTGCGCCTCGAACAGTATTCAGGAACCCTCCACA
TCCCGCTCCGCGCACAAGAACACCGTCACTTGCACCCAGCAGGGCCTGCTCGAGAACC
AGCCTAGTTTCCACCCCGCCAGGCGTGAATAGGGTGATCACTAGAGAGGAGCTCGAGG
CGCTTACCCCGTCACGCACTCCTAGCAGGTCGGTCTCGAGAACCAGCCTGGTCTCCAAC
CCGCCAGGCGTAAATAGGGTGATTACAAGAGAGGAGTTTGAGGCGTTCGTAGCACAAC
AACAATGACGGTTTGATGCGGGTGCATACATCTTTTCCTCCGACACCGGTCAAGGGCA
TTTACAACAAAAATCAGTAAGGCAAACGGTGCTATCCGAAGTGGTGTTGGAGAGGACC
GAATTGGAGATTTCGTATGCCCCGCGCCTCGACCAAGAAAAAGAAGAATTACTACGCA
AGAAATTACAGTTAAATCCCACACCTGCTAACAGAAGCAGATACCAGTCCAGGAAGGT
GGAGAACATGAAAGCCATAACAGCTAGACGTATTCTGCAAGGCCTAGGGCATTATTTG
AAGGCAGAAGGAAAAGTGGAGTGCTACCGAACCCTGCATCCTGTTCCTTTGTATTCAT
CTAGTGTGAACCGTGCCTTTTCAAGCCCCAAGGTCGCAGTGGAAGCCTGTAACGCCAT
GTTGAAAGAGAACTTTCCGACTGTGGCTTCTTACTGTATTATTCCAGAGTACGATGCCT
ATTTGGACATGGTTGACGGAGCTTCATGCTGCTTAGACACTGCCAGTTTTTGCCCTGCA
AAGCTGCGCAGCTTTCCAAAGAAACACTCCTATTTGGAACCCACAATACGATCGGCAG
TGCCTTCAGCGATCCAGAACACGCTCCAGAACGTCCTGGCAGCTGCCACAAAAAGAAA
TTGCAATGTCACGCAAATGAGAGAATTGCCCGTATTGGATTCGGCGGCCTTTAATGTG
GAATGCTTCAAGAAATATGCGTGTAATAATGAATATTGGGAAACGTTTAAAGAAAACC
CCATCAGGCTTACTGAAGAAAACGTGGTAAATTACATTACCAAATTAAAAGGACCAAA
AGCTGCTGCTCTTTTTGCGAAGACACATAATTTGAATATGTTGCAGGACATACCAATGG
ACAGGTTTGTAATGGACTTAAAGAGAGACGTGAAAGTGACTCCAGGAACAAAACATA
CTGAAGAACGGCCCAAGGTACAGGTGATCCAGGCTGCCGATCCGCTAGCAACAGCGTA
TCTGTGCGGAATCCACCGAGAGCTGGTTAGGAGATTAAATGCGGTCCTGCTTCCGAAC
ATTCATACACTGTTTGATATGTCGGCTGAAGACTTTGACGCTATTATAGCCGAGCACTT
CCAGCCTGGGGATTGTGTTCTGGAAACTGACATCGCGTCGTTTGATAAAAGTGAGGAC
GACGCCATGGCTCTGACCGCGTTAATGATTCTGGAAGACTTAGGTGTGGACGCAGAGC
TGTTGACGCTGATTGAGGCGGCTTTCGGCGAAATTTCATCAATACATTTGCCCACTAAA
ACTAAATTTAAATTCGGAGCCATGATGAAATCTGGAATGTTCCTCACACTGTTTGTGAA
CACAGTCATTAACATTGTAATCGCAAGCAGAGTGTTGAGAGAACGGCTAACCGGATCA
CCATGTGCAGCATTCATTGGAGATGACAATATCGTGAAAGGAGTCAAATCGGACAAAT
TAATGGCAGACAGGTGCGCCACCTGGTTGAATATGGAAGTCAAGATTATAGATGCTGT
GGTGGGCGAGAAAGCGCCTTATTTCTGTGGAGGGTTTATTTTGTGTGACTCCGTGACCG
GCACAGCGTGCCGTGTGGCAGACCCCCTAAAAAGGCTGTTTAAGCTTGGCAAACCTCT
GGCAGCAGACGATGAACATGATGATGACAGGAGAAGGGCATTGCATGAAGAGTCAAC
ACGCTGGAACCGAGTGGGTATTCTTTCAGAGCTGTGCAAGGCAGTAGAATCAAGGTAT
 
GAAACCGTAGGAACTTCCATCATAGTTATGGCCATGACTACTCTAGCTAGCAGTGTTA
AATCATTCAGCTACCTGAGAGGGGCCCCTATAACTCTCTACGGCTAA
SEQ ID NO: 65 - VEEV subgenomic promoter
   CCTGAATGGACTACGACATAGTCTAGTCCGCCAAG
SEQ ID NO: 66 - sequence of VEEV 3' UTR 
TAAGCGGCCGCTATGTTACGTGCAAAGGTGATTGTCACCCCCCGAAAGACCATATTGT
GACACACCCTCAGTATCACGCCCAAACATTTACAGCCGCGGTGTCAAAAACCGCGTGG
ACGTGGTTAACATCCCTGCTGGGAGGATCAGCCGTAATTATTATAATTGGCTTGGTGCT
GGCTACTATTGTGGCCATGTACGTGCTGACCAACCAGAAACATAATTGAATACAGCAG
CAATTGGCAAGCTGCTTACATAGAACTCGCGGCGATTGGCATGCCGCCTTAAAATTTTT
ATTTTATTTTTCTTTTCTTTTCCGAATCGGATTTTGTTTTTAATATTTC
SEQ ID NO: 67 - sequence encoding SARS-COV2 RBD (delta)

ATGGATGCAATGAAGAGAGGGCTCTGCTGTGTGCTGCTGCTGTGTGGAGCAGTCTTCG
TTTCGCCCAGCCAGGAAATCCATGCCCGATTCAGAAGACGCGTCCAGCCAACCGAGAG
CATCGTCAGATTTCCCAACATTACAAATCTGTGTCCCTTCGGCGAGGTGTTCAACGCCA
CACGCTTCGCTTCAGTGTACGCATGGAACCGCAAGCGCATATCTAACTGCGTCGCGGA
TTATTCTGTCCTCTACAACTCCGCCTCTTTCTCCACCTTCAAGTGCTACGGAGTGTCACC
GACTAAGCTGAACGATCTCTGCTTTACCAACGTCTACGCGGACTCCTTCGTGATAAGAG
GTGATGAAGTGAGACAAATAGCCCCAGGTCAGACTGGTAAGATCGCAGATTACAACTA
CAAATTGCCTGATGATTTCACTGGTTGCGTTATCGCGTGGAACTCTAATAACCTCGATT
CTAAGGTCGGTGGTAACTACAATTACaGGTACCGCTTGTTTAGGAAGTCAAACCTGAAG
CCTTTCGAGAGGGATATTTCAACCGAAATCTATCAAGCGGGTTCAAagCCGTGTAACGG
TGTGgAAGGATTTAACTGCTACTTCCCCCTGCAGTCTTACGGATTCCAGCCAACCAATG
GCGTGGGTTACCAACCTTATCGCGTGGTGGTTCTGAGTTTCGAACTGTTGCACGCTCCC
GCCACGGTATGCGGTCCCAAGAAAAGTACTAACTTGGTGAAGAATAAGTGCGTGAATT
TCGGCGGAGGAGGCAGCGGCGGAGGAGGCAGCGGAAGCGGCTACATCCCAGAAGCCC
CTAGAGACGGACAGGCTTACGTGCGAAAAGACGGCGAGTGGGTGCTGCTGAGCACAT
TCCTGGGAAGGAGCTGA

### Citations

[1] Paessler S., Weaver S.C. (2009) Vaccines for Venezuelan equine encephalitis. Vaccine. 27(Suppl 4): D80-D85.
[2] Samsa MM, Dupuy LC, Beard CW, Six CM, Schmaljohn CS, Mason PW, Geall AJ, Ulmer JB, Yu D. (2019) Self-Amplifying RNA Vaccines for Venezuelan Equine Encephalitis Virus Induce Robust Protective Immunogenicity in Mice. Mol Ther. 27(4): 850-865.
[3] Erasmus JH, Khandhar AP, O'Connor MA, Walls AC, Hemann EA, Murapa P, Archer J, Leventhal S, Fuller JT, Lewis TB, Draves KE, Randall S, Guerriero KA, Duthie MS, Carter D, Reed SG, Hawman DW, Feldmann H, Gale M Jr, Veesler D, Berglund P, Fuller DH. (2020) An Alphavirus-derived replicon RNA vaccine induces SARS-CoV-2 neutralizing antibody and T cell responses in mice and nonhuman primates. Sci Transl Med. 12(555): eabc9396.
[4] Maruggi G, Mallett CP, Westerbeck JW, Chen T, Lofano G, Friedrich K, Qu L, Sun JT, McAuliffe J, Kanitkar A, Arrildt KT, Wang KF, McBee I, McCoy D, Terry R, Rowles A, Abrahim MA, Ringenberg MA, Gains MJ, Spickler C, Xie X, Zou J, Shi PY, Dutt T, Henao-Tamayo M, Ragan I, Bowen RA, Johnson R, Nuti S, Luisi K, Ulmer JB, Steff AM, Jalah R, Bertholet S, Stokes AH, Yu D. (2022) A self-amplifying mRNA SARS-CoV-2 vaccine candidate induces safe and robust protective immunity in preclinical models. Mol Ther. 30(5): 1897-1912.
[5] Li Y, Teague B, Zhang Y, Su Z, Porter E, Dobosh B, Wagner T, Irvine DJ, Weiss R. (2019) In vitro evolution of enhanced RNA replicons for immunotherapy. Sci Rep. 9(1):6932
[6] Blakney AK, McKay PF, Bouton CR, Hu K, Samnuan K, Shattock RJ. (2021) Innate Inhibiting Proteins Enhance Expression and Immunogenicity of Self-Amplifying RNA. Mol Ther. 29(3):1174-1185
[7] Minnaert AK, Vanluchene H, Verbeke R, Lentacker I, De Smedt SC, Raemdonck K, Sanders NN, Remaut K. (2021) Strategies for controlling the innate immune activity of conventional and self-amplifying mRNA therapeutics: Getting the message across. Adv Drug Deliv Rev. 176:113900

## Claims

1. A replicable RNA molecule, comprising, from the 5' end to the 3' end, a 5' cap, a 5' UTR, an open reading frame encoding an RNA replicase, a promoter, a sequence of interest, a 3' UTR and a poly(A) tail, wherein the RNA replicase is capable of amplifying the replicable RNA molecule and is capable of amplifying an RNA molecule containing the sequence of interest and the 3' UTR, wherein the RNA replicase is a nonstructural protein or a functional variant thereof derived from Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV).

2. The replicable RNA molecule of claim 1, **characterized in that** the open reading frame encoding the RNA replicase comprises a nucleotide sequence that has at least 85% sequence identity to SEQ ID NO: 12, 36, 52, 56, 60, 16, 20, 24, 28, 32, 40, 44, or 48.

3. The replicable RNA molecule of claim 1, **characterized in that** the 5' UTR, the promoter, and/or the 3' UTR are derived from the same virus as the RNA replicase, wherein the promoter is a subgenomic promoter of the virus.

4. The replicable RNA molecule of claim 3, **characterized in that** the 5' UTR, the open reading frame encoding the RNA replicase, the promoter, and the 3' UTR respectively comprise nucleotide sequences set forth in (1) SEQ ID NOs: 11, 12, 13 and 14; (2) SEQ ID NOs: 35, 36, 37 and 38; (3) SEQ ID NOs: 51, 52, 53 and 54; (4) SEQ ID NOs: 55, 56, 57 and 58; (5) SEQ ID NOs: 59, 60, 61 and 62; (6) SEQ ID NOs: 15, 16, 17 and 18; (7) SEQ ID NOs: 19, 20, 21 and 22; (8) SEQ ID NOs: 23, 24, 25 and 26; (9) SEQ ID NOs: 27, 28, 29 and 30; (10) SEQ ID NOs: 31, 32, 33 and 34; (11) SEQ ID NOs: 39, 40, 41 and 42; (12) SEQ ID NOs: 43, 44, 45 and 46; or (13) SEQ ID NO: 47, 48, 49 and 50.

5. The replicable RNA molecule of claim 1, **characterized in that** the sequence of interest is an open reading frame encoding a peptide or protein of interest.

6. A replicable RNA molecule, comprising, from the 5' end to the 3' end, a 5' cap, a 5' UTR, an open reading frame encoding an RNA replicase, a promoter, a second 5' UTR, a sequence of interest, a 3' UTR and a poly(A) tail, wherein the RNA replicase is capable of amplifying the replicable RNA molecule and is capable of amplifying an RNA molecule containing the second 5' UTR, the sequence of interest, and the 3' UTR, wherein the RNA replicase is a nonstructural protein or a functional variant thereof of a self-replicating virus.

7. The replicable RNA molecule of claim 6, **characterized in that** the 5' UTR, the open reading frame encoding the RNA replicase, the promoter, and/or the 3' UTR are derived from the same self-replicating virus, wherein the promoter is a subgenomic promoter of the virus.

8. The replicable RNA molecule of claim 7, **characterized in that** the self-replicating virus is an alphavirus, a flavivirus, a measles virus or a rhabdovirus.

9. The replicable RNA molecule of claim 8, **characterized in that** the alphavirus is Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV).

10. The replicable RNA molecule of claim 6, **characterized in that** the second 5' UTR comprises a nucleotide sequence set forth in SEQ ID NO: 5, 6, 7 or 8.

11. A replicable RNA molecule, comprising, from the 5' end to the 3' end, a 5' cap, a 5' UTR, an open reading frame encoding an RNA replicase, a promoter, a first sequence of interest, an internal ribosome entry site (IRES), a second sequence of interest, a 3' UTR and a poly(A) tail, wherein the RNA replicase is capable of amplifying the replicable RNA molecule, and is capable of amplifying an RNA molecule containing the first sequence of interest, the internal ribosome entry site, the second sequence of interest, and the 3' UTR, wherein the RNA replicase is a nonstructural protein or a functional variant thereof of a self-replicating virus, wherein one of the first and second sequences of interest is an open reading frame encoding an immunosuppressive protein.

12. The replicable RNA molecule of claim 11, **characterized in that** the immunosuppressive protein is poxvirus E3L protein, poxvirus K3 protein, poxvirus B18/B18R protein, nonstructural protein 1 of influenza virus, PIV5 protein of parainfluenza virus or MERS ORF4a protein.

13. The replicable RNA molecule of claim 11, **characterized in that** the first sequence of interest is an open reading frame encoding a peptide or protein of interest, and the second sequence of interest is an open reading frame encoding an immunosuppressive protein; or
the first sequence of interest is an open reading frame encoding an immunosuppressive protein, and the second sequence of interest is an open reading frame encoding a peptide or protein of interest.

14. The replicable RNA molecule of claim 11, **characterized in that** the 5' UTR, the open reading frame encoding the RNA replicase, the promoter, and/or the 3' UTR are derived from the same self-replicating virus, wherein the promoter is a subgenomic promoter of the virus.

15. The replicable RNA molecule of claim 14, **characterized in that** the self-replicating virus is an alphavirus, a flavivirus, a measles virus or a rhabdovirus.

16. The replicable RNA molecule of claim 15, **characterized in that** the alphavirus is Mosso das Pedras virus (MDPV), Everglades virus (EVEV), Rio Negro virus (RNV), Mucambo virus (MUCV), Highlands J virus (HJV), Pixuna virus (PIXV), Trocara virus (TROV), Cabassou virus (CABV), Tonate virus (TONV), Bebaru virus (BEBV), Fort Morgan virus (FMV), Getah virus (GETV) or Ndumu virus (NDUV).

17. The replicable RNA molecule of claim 11, **characterized in that** a second 5' UTR is further comprised between the promoter and the first sequence of interest, wherein the RNA replicase is capable of amplifying an RNA molecule containing the second 5' UTR, the first sequence of interest, the internal ribosome entry site, the second sequence of interest, and the 3' UTR.

18. The replicable RNA molecule of claim 17, **characterized in that** the second 5' UTR comprises a nucleotide sequence set forth in SEQ ID NO: 5, 6, 7 or 8.

19. A DNA molecule encoding the replicable RNA molecule of any one of claims 1-18.

20. A vector comprising the DNA molecule of claim 19.

21. A cell comprising the DNA molecule of claim 19 or the vector of claim 20.

22. A method for preparing a peptide or protein of interest, comprising:
i) providing the replicable RNA molecule of any one of claims 1-18, wherein the sequence of interest in the replicable RNA molecule is an open reading frame encoding a peptide or protein of interest, and
ii) introducing the replicable RNA molecule into a cell.

23. A method for treating or preventing a disease in a subject in need thereof, **characterized in that** the replicable RNA molecule of any one of claims 1-18 is administered to the subject, wherein the sequence of interest in the replicable RNA molecule is an open reading frame encoding a disease-associated antigen or a peptide therapeutic agent.
